# EUROPEAN PATENT SPECIFICATION

(11) **EP 2 004 653 B1**
(45) Date of publication and mention of the grant of the patent: **30.12.2009**
(21) Application number: 07723895.4
(22) Date of filing: 02.04.2007
(51) Int. Cl.: C07D 487/04, A61K 31/519, A61P 35/00

(54) **3-SUBSTITUTED N-(ARYL- OR HETEROARYL)-PYRAZO[1,5-A]PYRIMIDINES AS KINASE INHIBITORS**
3-SUBSTITUIERTE N-(ARYL- ODER HETEROARYL)PYRAZO[1,5-A]PYRIMIDINE ALS KINASEINHIBITOREN
N-(ARYL- OU HÉTÉROARYL)-PYRAZOLO[1,5-A]PYRIMIDINES SUBSTITUÉES EN POSITION 3 UTILISÉS EN TANT QU'INHIBITEURS DE KINASES

(30) Priority: 04.04.2006 GB 0606805
(43) Date of publication of application: 24.12.2008
(73) Proprietor: Novartis AG, 4056 Basel (CH)
(72) Inventor: MASUYA, Keiichi, CH-4103 Bottmingen (CH); VAUPEL, Andrea, CH-4125 Riehen (CH); IMBACH, Patricia, CH-4303 Kaiseraugst (CH); FURET, Pascal, F-68800 Thann (FR)
(74) Representative: Dyer, James
(86) International application number: PCT/EP2007/002954
(87) International publication number: WO 2007/113000

(56) References cited:
- WO-A-2005/070431
- WO-A-2007/009773

## Description

The invention relates to 3-substituted N-(aryl- or heteroaryl)-pyrazolo[1,5-a]pyrimidine compounds, their use as kinase inhibitors, new pharmaceutical formulations comprising said compounds, said compounds for use in the diagnostic or therapeutic treatment of warm-blooded animals, especially humans, their use for the manufacture of pharmaceutical formulations useful in the treatment of diseases that respond to modulation of kinase, especially tie-2 kinase, activity, methods of treatment comprising administration of said compounds to a warm-blooded animal, especially a human, and processes for the manufacture of said compounds.

The term kinases comprises both receptor-type kinases and nonreceptor-type kinases, as well as tyrosine and serine/threonine kinases. Among the receptor type tyrosine kinases. Tie-2 (which is also called TEK) is expressed in endothelial cells that line the lumen of blood vessels. It has been shown to be involved in endothelial cell migration, sprouting, survival and periendothelic cell recruitment during angiogenesis.

In contrast to VEGFRs (vascular endothelial growth factor receptors), which control the onset of angiogenesis, the angiopoietins (ligands of Tie-2) and Tie-2 are involved in vessel stabilization and vascular remodeling. It could be shown that Tie-2 is activated by one of its ligands, angiopoieitin-1, which is antagonized by a second ligand, angiopoietin-2 (ang2). Where angiogenesis takes place, the antagonist ang2 is up-regulated. Therefore there was hitherto no direct clue allowing to reasonably assume whether inhibition of Tie-2 promotes or inhibits angiogenesis, but this concept has been confirmed in the meantime.

On the other hand, in view of the many possible mechanisms involved in the pathogenesis of tumor and other proliferative diseases, a need exists to find novel and useful modulators of the activity of kinases which often are involved in their genesis. Therefore novel compounds that modulate the activity of other kinases than those already established compounds as useful in the treatment of proliferative diseases and that can affect tumor growth, especially in cases where no effect is found with VEGFR inhibitiors, are highly desirable.

It is therefore a problem to be solved by the present invention to provide novel chemical compounds with advantageous pharmaceutical properties that are useful in the treatment of proliferative diseases, such as tumor diseases.

Surprisingly, it is possible to establish that a novel class of 3-substituted N-(aryl- or heteroaryl)- pyrazolo[1,5-a]pyrimidine compounds is capable to inhibit the growth of tumors in tumor models that depend on angiogenesis. Especially, it has been found that these compounds can inhibit Tie-2 kinase quite specifically and could be sufficient to inhibit VEGF-induced angiogenesis *in vivo* when tested, for example, in a subcutaneous growth factor chamber implant model and can show, for example, qualitative differences to VEGFR2 inhibitors.

The invention therefore relates to novel compounds of the formula I, 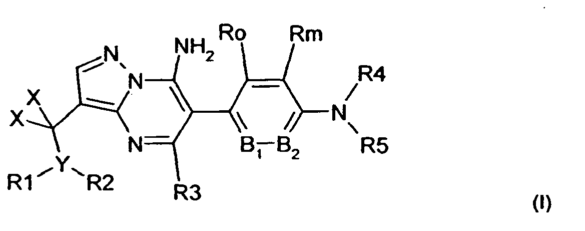 wherein
either
each of R1 and R2 is, independently of the other, unsubstituted or substituted lower alkyl, unsubstituted or substituted C₃-C₁₀-cycloalkyl, unsubstituted or substituted C₆-C₁₄-aryl or unsubstituted or substituted heterocyclyl with 3 to 14 ring atoms and Y is N, or R1, Y and R2 together form an unsubstituted or substituted heterocyclyl with 3 to 14 ring atoms and at least one nitrogen heteroatom which is bound via a ring nitrogen;
each of the two X stands for hydrogen or both together form oxo (=O) or thioxo (=S);
R3 is hydrogen or lower alkyl;
R4 is hydrogen or unsubstituted or substituted lower alkyl;
R5 is acyl;
B, is N or CRo,
B₂ is N or CRm,
and each of Ro and Rm, independently of the other, is hydrogen, lower alkyl, halo or lower alkoxy;
or a salt thereof.

Listed below are definitions of various terms used to describe the compounds of the present invention as well as their use and synthesis, starting materials and intermediates and the like. These definitions, either by replacing one, more than one or all general expressions or symbols used in the present disclosure and thus yielding preferred embodiments of the invention, preferably apply to the terms as they are used throughout the specification unless they are otherwise limited in specific instances either individually or as part of a larger group. In other terms: Independently of each other, one or more of the more general expressions may be replaced by the more specific definitions, thus leading to preferred embodiments of the invention.

The term "lower" or "C₁-C₇-" defines a moiety with up to and including maximally 7, especially up to and including maximally 4, carbon atoms, said moiety being branched (one or more times) or straight-chained and bound via a terminal or a non-terminal carbon. Lower or C₁-C₇-alkyl, for example, is n-pentyl, n-hexyl or n-heptyl or preferably C₁-C₄-alkyl, especially as methyl, ethyl, n-propyl, sec-propyl, n-butyl, isobutyl, sec-butyl, tert-butyl.

Halo or halogen is preferably fluoro, chloro, bromo or iodo, most preferably fluoro, chloro or bromo.

Unsubstituted or substituted lower alkyl is preferably (linear or branched) lower alkyl that is unsubstituted or substituted by one or more, e.g. one to three, substituents, e.g. at a terminal carbon atom, independently selected from the group consisting of unsubstituted or substituted C₆-C₁₄-aryl as described below, especially phenyl or naphthyl, (each of) which is unsubstituted or substituted as described below for unsubstituted or substituted C₆-C₁₄-aryl, (as one preferred embodiment in the case of substituted lower alkyl R1 and/or R2) unsubstituted or substituted heterocyclyl with 3 to 14 ring atoms as described below which is unsubstituted or substituted as described below for unsubstituted or substituted heterocyclyl, especially piperidino, N-lower alkylpiperidinyl, morpholino, thiomorpholino, S,S-dioxothiomorpholino, N-C₁-C₇-alkyl-piperazino, pyridyl, e.g. pyridine-2-yl, -3-yl or 4-yl, or N-mono- or N,N-di-C₁-C₇-alkyl-substituted or unsubstituted pyrrolidino, unsubstituted or substituted cycloalkyl as described below, especially cyclopropyl, cyclobutyl, cyclopentyl or cyclohexyl each of which is unsubstituted or substituted as described below for unsubstituted or substituted cycloalkyl, halo, e.g. in trifluoromethyl, hydroxy, (as one preferred embodiment in the case of substituted lower alkyl R1 and/or R2) C₁-C₇-alkoxy, halo-C₁-C₇-alkoxy, such as trifluoromethoxy, hydroxy-C₁-C₇-alkoxy, C₁-C₇-alkoxy-C₁-C₇-alkoxy, phenyl- or naphthyloxy, phenyl- or naphthyl-C₁-C₇-alkyloxy, C₁-C₇-alkanoyloxy, benzoyl- or naphthoyloxy, C₁-C₇-alkylthio, halo-C₁-C₇-alkthio, such as trifluoromethylthio, C₁-C₇-alkoxy-C₁-C₇-alkylthio, phenyl- or naphthylthio, phenyl- or naphthyl-C₁-C₇-alkylthio, C₁-C₇-alkanoylthio, benzoyl- or naphthoylthio, nitro, amino, (as one preferred embodiment in the case of substituted lower alkyl R1 and/or R2) mono- or di-(C₁-C₇-alkyl, C₁-C₇-alkoxy-C₁-C₇alkyl and/or (mono- or di-(C₁-C₇-alky)-amino)-C₁-C₇-alkyl)-amino, mono- or di-(naphthyl- or phenyl-C₁-C₇-alkyl)-amino, C₁-C₇-alkanoylamino, benzoyl- or naphthoylamino, C₁-C₇-alkylsulfonylamino, phenyl- or naphthylsulfonylamino wherein phenyl or naphthyl is unsubstituted or substituted by one or more, especially one to three, C₁-C₇-alkyl moieties, phenyl- or naphthyl-C₁-C₇-alkylsulfonylamino, carboxyl, C₁-C₇-alkyl-carbonyl. C₁-C₇-alkoxy-carbonyl, phenyl- or naphthyloxycarbonyl, phenyl- or naphthyl-C₁-C₇-alkoxycarbonyl, carbamoyl, N- mono- or N,N-di-(C₁-C₇-alkyl)-aminocarbonyl, N-mono- or N,N-di-(naphthyl- or phenyl-C₁-C₇-alkyl)-aminocarbonyl, cyano, C₁-C₇-alkenylene or-alkynylene, C₁-C₇-alkylenedioxy, C₁-C₇-alkylsulfonyl, phenyl- or naphthylsulfonyl wherein phenyl or naphthyl is unsubstituted or substituted by one or more, especially one to three, C₁-C₇-alkyl moieties, phenyl- or naphthyl-C₁-C₇-alkylsulfonyl, sulfamoyl and N-mono or N,N-di-(C₁-C₇-alkyl, phenyl, naphthyl, phenyl-C₁-C₇-alkyl or naphthyl-C₁-C₇-alkyl)aminosulfonyl.

Unsubstituted or substituted C₃-C₁₀-cycloalkyl is preferably cyclopropyl, cycobutyl, cyclopentyl or cyclohexyl and is substituted or (preferably) unsubstituted by one or more substitutents as mentioned for substituted lower alkyl (other than unsubstituted or substituted cycloalkyl).

Unsubstituted or substituted C₆-C₁₄-aryl preferably is a mono- or polycyclic, especially monocyclic, bicyclic or tricyclic aryl moiety with 6 to 14 ring carbon atoms, especially phenyl (very preferred), naphthyl (preferred), indenyl, fluorenyl, acenapthylenyl, phenylenyl or phenanthryl, and is unsubstituted or substituted by one or more, especially one to three, moieties, preferably independently selected from the group consisting of C₁-C₇-alkyl, such as methyl(R5), ethyl, n-propyl, isopropyl, n-butyl, isobutyl, sec-butyl or tert-butyl(R5), C₂-C₇-alkenyl, C₂-C₇-alkinyl, phenyl- or naphthyl-C₁-C₇-alkyl, such as benzyl or naphthylmethyl, halo-C₁-C₇-alkyl, such as trifluoromethyl(R5), hydroxy-C₁-C₇-alkyl, C₁-C₇-alkoxy-C₁-C₇-alkyl, such as 3-methoxypropyl or 2-methoxyethyl, C₁-C₇-alkoxy-C₁-C₇-alkoxy-C₁-C₇-alkyl, phenyloxy- or naphthyloxy-C₁-C₇-alkyl, phenyl-C₁-C₇-alkoxy- or naphthyl-C₁-C₇-alkoxy-C₁-C₇-alkyl, amino-C₁-C₇-alkyl, such as aminomethyl, N-mono- or N,N-di-(C₁-C₇-alkyl and/or mono-C₁-C₇-alkoxy-C₁-C₇alkyl and/or (mono- or di-(C₁-C₇-alkyl)-amino-C₁-C₇-alkyl)-amino-C₁-C₇-alkyl, C,-C₇-alkoxy-C,-C₇-alkylamino-C,-C₇-alkyl, mono- or di-(naphthyl- or phenyl-C₁-C₇-alkyl)-amino-C₁-C₇-alkyl, C₁-C₇-alkanoylamino-C₁-C₇-alkyl, carboxy-C₁-C₇-alkyl, benzoyl- or naphthoylamino-C₁-C₇-alkyl, C₁-C₇-alkylsulfonylamino-C₁-C₇-alkyl, phenyl- or naphthylsulfonylamino-C₁-C₇-alkyl wherein phenyl or naphthyl is unsubstituted or substituted by one or more, especially one to three, C₁-C₇-alkyl moieties, phenyl- or naphthyl-C₁-C₇-alkylsulfonylamino-C₁-C₇-alkyl, pyrrolidino-C₁-C₇-alkyl, piperidino-C₁-C₇-alkyl, morpholino-C₁-C₇-alkyl, thiomorpholino-C₁-C₇-alkyl, N-C₁-C₇-alkyl-piperazina-C₁-C₇-alkyl, N-mono- or N,N-di-(C₁-C₇-alkyl)-amino-substituted or unsubstituted pyrrolidino-C₁-C₇-alkyl, halo (which is especially preferred as substituent in aryl moieties forming part of R5), especially fluoro, chloro (which is especially preferred) or bromo(R5), hydroxy, C₁-C₇-alkoxy(R5), phenyl-C₁-C₇-alkoxy wherein phenyl is unsubstituted or substituted by C₁-C₇-alkoxy and/or halo, halo-C₁-C₇-alkoxy, such as trifluoromethoxy, hydroxy-C₁-C₇-alkoxy, C₁-C₇-alkoxy-C₁-C₇-alkoxy, amino-C₁-C₇-alkoxy, N-C₁-C₇-alkanoylamino-C₁-C₇-alkoxy, N-unsubstituted-, N-mono- or N,N-di-(C₁-C₇-alkyl)carbamoyl-C₁-C₇-alkoxy, phenyl- or naphthyloxy, phenyl- or naphthyl-C₁-C₇-alkyloxy, C₁-C₇-alkanoyloxy, benzoyl- or naphthoyloxy, C₁-C₇-alkylthio, hala-C₁-C₇-alkthio, such as trifluoromethylthio, C₁-C₇-alkoxy-C₁-C₇-alkylthio, phenyl- or naphthylthio, phenyl- or naphthyl-C₁-C₇-alkylthio, C₁-C₇-alkanoylthio, benzoyl- or naphthoylthio, nitro, amino, mono- or di-(C₁-C₇-alkyl)-amino, mono- or di-(naphthyl- or phenyl-C₁-C₇-alkyl)-amino, C₁-C₇-alkanoylamino, benzoyl- or naphthoylamino, C₁-C₇-alkylsulfonylamino, phenyl- or naphthylsulfonylamino wherein phenyl or naphthyl is unsubstituted or substituted by one or more, especially one to three, C₁-C₇-alkyl moieties, phenyl- or naphthyl-C₁-C₇-alkylsulfonylamino, C₁-C₇-alkanoyl, C₁-C₇-alkoxy-C₁-C₇-alkanoyl, carboxyl, C₁-C₇-alkyl-carbonyl, C₁-C₇-alkoxy-carbonyl, phenyl- or naphthyloxycarbonyl, phenyl- or naphthyl-C₁-C₇-alkoxycarbonyl, carbamoyl, N-mono- or N,N-di-(C₁-C₇-alkyl and/or mono-C₁-C₇-alkoxy-C₁-C₇alkyl and/or (mono- or di-(C₁-C₇-alkyl)-amino-C₁-C₇-alkyl)-amino-carbonyl, such as N- mono- or N,N-di-(C₁-C₇-alkyl)-aminocarbonyl, N-C₁-C₇-alkoxy-C₁-C₇-alkylcarbamoyl, N-mono- or N,N-di-(naphthyl- or phenyl-C₁-C₇-alkyl)-aminocarbonyl, pyrrolidinocarbonyl, piperidinocarbonyl, morpholinocarbonyl, thiomorpholinocarbonyl, N-C₁-C₇-alkyl-piperazinocarbonyl, N-mono- or N,N-di-(C₁-C₇-alkyl)-amino-substituted or unsubstituted pyrrolidino-C₁-C₇-alkyl, cyano(R5), C₁-C₇-alkenylene or - alkinylene, C₁-C₇-alkylsulfonyl, phenyl- or naphthylsulfonyl wherein phenyl or naphthyl is unsubstituted or substituted by one or more, especially one to three, C₁-C₇-alkyl moieties, phenyl- or naphthyl-C₁-C₇-alkylsulfonyl, sulfamoyl and N-mono or N,N-di-(C₁-C₇-alkyl, phenyl-, naphthyl-, phenyl-C₁-C₇-alkyl- or naphthyl-C₁-C₇-alkyl)-aminosulfonyl, piperidino, morpholino, thiomorpholino, N-C₁-C₇-alkyl-piperazino, or N-mono- or N,N-di-(C₁-C₇-alkyl)-amino-substituted or unsubstituted pyrrolidino. Especially preferably aryl is phenyl or naphthyl, each of which is unsubstituted or substituted by one or more, e.g. up to three, substituents independently selected from the group consisting of C₁-C₇,-alkyl, hydroxy-C₁-C₇-alkyl, C₁-C₇-alkoxy-C₁-C₇-alkyl, halo-C₁-C₇-alkyl, pyrrolidino-C₁-C₇-alkyl, piperidino-C₁-C₇-alkyl, morpholino-C₁-C₇-alkyl, thiomorpholino-C₁-C₇-alkyl, N-C₁-C₇-alkyl-piperazino-C₁-C₇-alkyl. N-mono- or N,N-di-(C₁-C₇-alkyl)-amino-substituted or unsubstituted pyrrolidino-C₁-C₇-alkyl, halo, especially fluoro, chloro or bromo, hydroxy, C₁-C₇-alkoxy, C₁-C₇-alkoxy-C₁-C₇-alkoxy, amino-C₁-C₇-alkoxy, N-C₁-C₇-alkanoylamino-C₁-C₇-alkoxy, carbamoyl-C₁-C₇-alkoxy, N-mono- or N,N-di-(C₁-C₇-alkyl)-carbamoyl-C₁-C₇-alkoxy, amino, C₁-C₇-alkanoylamino, C₁-C₇-alkanoyl, C₁-C₇-alkoxy-C₁-C₇-alkanoyl, carboxy, C₁-C₇-alkoxycarbonyl, carbamoyl, N-mono- or N,N-di-C₁-C₇-alkyl and/or C₁-C₇-alkoxy-C₁-C₇-alkyl)-carbamoyl, pyrrolidinocarbonyl, piperidinocarbonyl, morpholinocarbonyl, thiomorpholinocarbonyl, N-C₁-C₇-alkyl-piperazihocarbonyl, N-mono- or N,N-di-(C₁-C₇-alkyl)-amino-substituted or unsubstituted pyrrolidina-C₁-C₇-alkyl, nitro, cyano, pyrrolidino, piperidino, morpholino, thiomorpholino, N-C₁-C₇-alkyl-piperazino, and N-mono- or N,N-di-(C₁-C₇-alkyl)-amino-substituted or unsubstituted pyrrolidino.

Unsubstituted or substituted heterocyclyl with 3 to 14 ring atoms is preferably a heterocyclic radical that is unsaturated, saturated or partially saturated in the bonding ring and is preferably a monocyclic or in a broader aspect of the invention poly-, e.g. bi- or tri-cyclic ring; has 3 to 14 ring atoms; wherein at least in the ring bonding to the remaining part of the molecule of formula I one or more, preferably one to four, especially one or two carbon ring atoms are replaced by a heteroatom selected from the group consisting of nitrogen (which is always present as Y in case of heterocyclyl formed by R1, Y and R2 together), oxygen and sulfur, the bonding ring preferably having 4 to 12, especially 5 to 7 ring atoms; heterocyclyl being unsubstituted or substituted by one or more, especially 1 to 3, substituents independently selected from the group consisting of the substituents defined above under "substituted alkyl" or "substituted aryl"; especially being a hetercyclyl radical selected from the group consisting of oxiranyl, azirinyl, 1,2-oxathiolanyl, imidazolyl, thienyl, furyl, tetrahydrofuryl, pyranyl, thiopyranyl, thianthrenyl, isobenzofuranyl, benzofuranyl, chromenyl, 2H-pyrrolyl, pyrrolyl, pyrrolinyl, pyrrolidinyl, e.g. pyrrolidino, oxo-pyrrolidino, such as 2-oxopyrrolidino, imidazolyl, imidazolidinyl, benzimidazolyl, pyrazolyl, pyrazinyl, pyrazolidinyl, pyranyol, thiazolyl, isothiazolyl, dithiazolyl, oxazolyl, isoxazolyl, e.g. isoxazol-3-yl, (R₅), pyridyl, e.g. pyridine-2-. -3- or -4-yl, pyrazinyl, pyrimidinyl, piperidyl, e.g. piperidino or piperidin-4-yl, piperazinyl, e.g. piperazino, pyridazinyl, morpholinyl, thiomorpholinyl, S,S-dioxothiomorpholino, indolizinyl, isoindolyl, 3H-indolyl, indolyl, benzimidazolyl, cumaryl, indazolyl, triazolyl, tetrazolyl, purinyl, 4H-quinolizinyl, isoquinolyl, quinolyl, tetrahydroquinolyl, tetrahydroisoquinolyl, decahydroquinolyl, octahydroisoquinolyl, benzofuranyl, dibenzofuranyl, benzothiophenyl, dibenzothiophenyl, phthalazinyl, naphthyridinyl, quinoxalyl, quinazolinyl, quinazolinyl, cinnolinyl, pteridinyl, carbazolyl, beta-carbotinyl, phenanthridinyl, acridinyl, perimidinyl, phenanthrolinyl, furazanyl, phenazinyl, phenothiazinyl, phenoxazinyl, chromenyl, isochromanyl and chromanyl, each of these radicals being unsubstituted or substituted by one or more, e.g. one to three radicals independently selected from the group consisting of C₁-C₇-alkyl, such as methyl, ethyl, n-propyl, isopropyl, n-butyl, isobutyl, sec-butyl or tert-butyl, phenyl or naphthyl each of which is unsubstituted or substituted by one or more substituents selected from the group consisting of oxo, halo, lower alkoxy, pyrrolidinyl-lower alkyl (especially-methyl), piperidinyl-lower alkyl (especially-methyl), piperazino-lower alkyl (especially-methyl), N-lower alkylpiperazino-lower alkyl (especially-methyl), morpholino-lower alkyl (especially-methyl) and thiomorpholino-lower alkyl (especially-methyl), C₂-C₇-alkenyl, C₂-C₇-alkinyl, phenyl- or naphthyl-C₁-C₇-alkyl, such as benzyl or naphthylmethyl, halo-C₁-C₇-alkyl, such as trifluoromethyl, hydroxy-C₁-C₇-alkyl, C₁-C₇-alkoxy-C₁-C₇-alkyl, such as 3-methoxypropyl or 2-methoxyethyl, C₁-C₇-alkoxy-C₁-C₇-alkoxy-C₁-C₇-alkyl, phenyloxy- or naphthyloxy-C₁-C₇-alkyl, phenyl-C₁-C₇-alkoxy- or naphthyl-C₁-C₇-alkoxy-C₁-C₇-alkyl, amino-C₁-C₇-alkyl, such as aminomethyl, N-mono- or N,N-di-(C₁-C₇-alkyl, mono-C₁-C₇-alkoxy-C₁-C₇alkyl and/or (mono- or di-(C₁-C₇-alkyl)-amino-C₁-C₇-alkyl)-amino-C₁-C₇-alkyl, C₁-C₇-alkoxy-C₁-C₇-alkylamino-C₁-C₇-alkyl, mono- or di-(naphthyl- or phenyl-C₁-C₇-alkyl)-amino-C₁-C₇-alkyl, C₁-C₇-alkanoylamino-C₁-C₇-alkyl, carboxy-C₁-C₇-alkyl, benzoyl- or naphthoylamino-C₁-C₇-alkyl, C₁-C₇-alkylsulfonylamino-C₁-C₇-alkyl, phenyl- or naphthylsulfonylamino-C₁-C₇-alkyl wherein phenyl or naphthyl is unsubstituted or substituted by one or more, especially one to three, C₁-C₇-alkyl moieties, phenyl- or naphthyl-C₁-C₇-alkylsulfonylamino-C₁-C₇-alkyl, pyrrolidino-C₁-C₇-alkyl, piperidino-C₁-C₇-alkyl, morpholino-C₁-C₇-alkyl, thiomorpholino-C₁-C₇-alkyl, N-C₁-C₇-alkyl-piperazino-C₁-C₇-alkyl, N-mono- or N,N-di-(C₁-C₇-alkyl)-amino-substituted or unsubstituted pyrrolidino-C₁-C₇-alkyl, halo, especially fluoro, chloro or bromo, hydroxy, C₁-C₇-alkoxy, phenyl-C₁-C₇-alkoxy wherein phenyl is unsubstituted or substituted by C₁-C₇-alkoxy and/or halo, halo-C₁-C₇-alkoxy, such as trifluoromethoxy, hydroxy-C₁-C₇-alkoxy, C₁-C₇-alkoxy-C₁-C₇-alkoxy, amino-C₁-C₇-alkoxy, N-C₁-C₇-alkanoylamino-C₁-C₇-alkoxy, N-unsubstituted-, N-mono- or N,N-di-(C₁-C₇-alkyl)carbamoyl-C₁-C₇-alkoxy, phenyl- or naphthyloxy, phenyl- or naphthyl-C₁-C₇-alkyloxy, C₁-C₇-alkanoyloxy, benzoyl- or naphthoyloxy, C₁-C₇-alkylthio, halo-C₁-C₇-alkthio, such as trifluoromethylthio, C₁-C₇-alkoxy-C₁-C₇-alkylthio, phenyl- or naphthylthio, phenyl- or naphthyl-C₁-C₇-alkylthio, C₁-C₇-alkanoylthio, benzoyl- or naphthoylthio, nitro, amino, mono- or di-(C₁-C₇-alkyl)-amino, mono- or di-(naphthyl- or phenyl-C₁-C₇-alkyl)-amino, C₁-C₇-alkanoylamino, benzoyl- or naphthoylamino, C₁-C₇-alkylsulfonylamino, phenyl- or naphthylsulfonylamino wherein phenyl or naphthyl is unsubstituted or substituted by one or more, especially one to three, C₁-C₇-alkyl moieties, phenyl- or naphthyl-C₁-C₇-alkylsulfonylamino, C₁-C₇-alkanoyl, C₁-C₇-alkoxy-C₁-C₇-alkanoyl, carboxyl, C₁-C₇-alkyl-carbonyl, C₁-C₇-alkoxy-carbonyl, phenyl- or naphthyloxycarbonyl, phenyl- or naphthyl-C₁-C₇-alkoxycarbonyl, carbamoyl,- N-mono- or N,N-di-(C₁-C₇-alkyl and/or mono-C₁-C₇-alkoxy-C₁-C₇alkyl and/or (mono- or di-(C₁-C₇-alkyl)-amino-C₁-C₇-alkyl)-aminocarbonyl, such as N- mono- or N,N-di-C₁-C₇-alkyl)-aminocarbonyl, N-C₁-C₇-alkoxy-C₁-C₇-alkylcarbamoyl, N-mono- or N,N-di-(naphthyl- or phenyl-C₁-C₇-alkyl)-aminocarbonyl, pyrrolidinocarbonyl, piperidinocarbonyl, morpholinocarbonyl, thiomorpholinocarbonyl, N-C₁-C₇-alkyl-piperazinocarbonyl, N-mono- or N,N-di-C₁-C₇-alkyl)-amino-substituted or unsubstituted pyrrolidino-C₁-C₇-alkyl, cyano, C₁-C₇-alkenylene or-alkinylene, C₁-C₇-alkylsulfonyl (= lower alkanesulfonyl), phenyl- or naphthylsulfonyl wherein phenyl or naphthyl is unsubstituted or substituted by one or more, especially one to three, C₁-C₇-alkyl moieties, phenyl- or naphthyl-C₁-C₇-alkylsulfonyl, sulfamoyl and N-mono or N,N-di-(C₁-C₇-alkyl. and phenyl-, naphthyl-, phenyl-C₁-C₇-alkyl- or naphthyl-C₁-C₇-alkyl)-aminosulfonyl.

Where R1, Y and R2 together form an unsubstituted or substituted heterocyclyl with 3 to 14 ring atoms bound and at least one nitrogen heteroatom which is bound via a ring nitrogen, the heterocycles are preferably selected from the nitrogen-comprising heterocycles mentionned above that may be unsubstituted or substituted as described for unsubstituted or substituted heterocyclyl above, especially from pyrrolidinyl, such as pyrrolidino, oxopyrrolidino, such as 2-oxopyrrolidino, piperidinyl, such as piperidino or piperidin-4-yl, N-lower alkylpiperidino, morpholino, thiomorpholino, S,S-dioxothiomorpholino and piperazinyl, such as piperazino, each of which are unsubstituted or substituted as defined for substituted heterocyclyl, especially by lower alkyl, such as methyl, amino, N-mono- or N,N-di-(loweralkyl)-amino or lower alkanesulfonyl, such as methanesulfonyl.

One group of compounds of the formula I is preferred wherein each X is hydrogen. In another preferred group of compounds of the formula I, X and X together are oxo.

Lower alkyl R3 is preferably methyl.

Acyl is preferably the moiety (remaining after the removal of the acidic hydrogen) of an organic carbonic or sulfonic acid with (without substitutents) 1 to 22 carbon atoms, and is preferably selected from the group consisting of unsubstituted or substituted C₆-C₁₄-arylaminocarbonyl (=C₆-C₁₄-aryl-NH-C(=O)-) which is especially preferred, unsubstituted or substituted heterocyclylaminocarbonyl (= heterocyclyl-N-C(=O)-) wherein heterocyclyl has 3 to 14 ring atoms which is especially preferred, unsubstituted or substituted C₆-C₁₄-arylaminosulfonyl (= aryl-NH-S(O)₂-), unsubstituted or substituted heterocyclylaminosulfonyl (= heterocylyl-NH-S(O)₂) wherein heterocyclyl has 3 to 14 ring atoms, unsubstituted or substituted lower-alkanesulfonyl (= lower-alkane-S(O)₂-) which is especially preferred, unsubstituted or substituted C₆-C₁₄-arylsulfonyl (= aryl-S(O)₂-) which is especially preferred, unsubstituted or substituted heterocyclylsulfonyl (= heterocyclyl-S(O)₂-) wherein heterocyclyl has 3 to 14 ring atoms, and unsubstituted or substituted C₆-C₁₄-arylcarbonyl (= aryl-C(=O)-).

In unsubstituted or substituted C₆-C₁₄-arylaminocarbonyl, unsubstituted or substituted C₆-C₁₄-aryl is preferably defined as above; more preferred is a moiety selected from phenylaminocarbonyl wherein phenyl is unsubstituted or substituted by one or more, especially up to two, moieties independently selected from lower alkyl, especially methyl, halo (very preferred), especially chloro; halo-lower alkyl, such as trifluoromethyl, lower alkoxy, such as methoxy, and cyano. Very preferred is 3-trifluoromethyl-phenylaminocarbonyl, more preferred 4-fluorophenylaminocarbonyl and most preferred 3- or 2-chlorophenylaminocarbonyl.

In unsubstituted or substituted heterocyclylaminocarbonyl wherein heterocyclyl has 3 to 14 ring atoms, unsubstituted or substituted heterocyclyl is preferably as defined above; more preferred is pyrazolyl-aminocarbonyl (especially pyrazol-5-ylaminocarbonyl) or isoxazolylaminocarbonyl (especially isoxazol-3-ylaminocarbonyl), where each pyrazolyl or isoxazolyl is unsubstituted or substituted by one or two moieties independently selected from the group consisting of lower alkyl, such as tert-butyl, and phenyl that is unsubstituted or substituted with halo, especially fluoro or preferably chloro, lower alkoxy, especially methoxy, piperazino-lower alkyl, especially piperazinomethyl, 4-lower alkylpiperazino-lower alkyl, such as 4-methylpiperazino-methyl, and morpholino-lower alkyl, epecially morpholinomethyl. Very preferred is 3-tert-butyl-1-(4-fluorophenyl)-pyrazol-5-ylaminocarbonyl, 3-tert-butyl-1-(4-methoxyphenyl)-pyrazol-5-ylaminocarbonyl, 3-tert-butyl-1-(4-(4-methyl-piperazinomethyl)-phenyl)-pyrazol-5-ylaminocarbonyl, 3-tert-butyl-1-(3-(4-methyl-piperazinomethyl)-phenyl)-pyrazol-5-ylaminocarbonyl, 3-tert -butyl-1-(4-(morpholinomethyl)-phenyl)-pyrazol-5-ylaminocarbonyl or 5-tert-butyl-isoxazol-3-ylaminocarbonyl.

In unsubstituted or substituted C₆-C₁₄-arylaminosulfonyl, unsubstituted or substituted C₆-C₁₄-aryl is preferably as described above. Very preferred is 3-trifluoromethyl-phenylaminosulfonyl, more preferred 4-fluorophenylaminosulfonyl and most preferred 3- or 2-chlorophenylaminosulfonyl.

In unsubstituted or substituted heterocyclylaminosulfonyl wherein heterocyclyl has 3 to 14 ring atoms, unsubstituted or substituted heterocylyl is preferably as defined above; more preferred is pyrazolyl-aminosulfonyl (especially pyrazol-5-ylaminosulfonyl) or isoxazolylaminosulfonyl (especially isoxazol-3-ylaminosulfonyl), where each pyrazolyl or isoxazolyl is unsubstituted or substituted by one or two moieties independently selected from the group consisting of lower alkyl, such as tert-butyl, and phenyl that is unsubstituted or substituted with halo, especially fluoro or preferably chloro, lower alkoxy, especially methoxy, piperazino-lower alkyl, especially piperazinomethyl, 4-lower alkylpiperazino-lower alkyl, such as 4-methylpiperazino-methyl, and morpholino-lower alkyl, epecially morpholinomethyl Very preferred is 3-tert-butyl-1-(4-fluorophenyl)-pyrazol-5-ylaminosulfonyl.

In unsubstituted or substituted lower-alkanesulfonyl, unsubstituted or substituted lower alkyl is preferably as defined above; more preferred is phenyl-lower alkanesulfonyl, such as phenylmethylsuffonyl or 2-phenylethylsulfonyl, wherein each phenyl is unsubstituted (preferred) or substituted with one or more, e.g. up to three, moieties independently selected from the group consisting of lower alkyl, e.g. methyl, halo, e.g. chloro or fluoro, halo-lower alkyl, e.g. trifluoromethyl, lower alkoxy, e.g. methoxy, and cyano. Very preferred is phenylmethylsulfonyl or 2-phenylethylsulfonyl.

In unsubstituted or substituted C₆-C₁₄-arylsulfonyl, unsubstituted or substituted C₆-C₁₄-aryl is preferably as defined above; more preferred is phenylsulfonyl wherein the phenyl is unsubstituted or substituted by one or more, e.g. up to three, more preferably up to two, moieties independently selected from the group consisting of lower alkyl, e.g. methyl, halo (preferred), such as chloro (very preferred) or fluoro, halo-lower alkyl, e.g. trifluoromethyl, lower alkoxy, e.g. methoxy; and cyano. Very preferred is 2,3-dimethylphenylsulfonyl, 2-, 3- or 4-methylphenylsulfonyl, 2-, 3- or 4-methoxyphenylsulfonyl, 2-methyl-4,5-dimethoxyphenylsulfonyl, 2,5-dimethoxyphenylsulfonyl, 2-, 3- or 4-trifluoromethylphenylsulfonyl, 2-chloro-5-trifluoromethylphenylsulfonyl, 2-chlorm4-trifluoromethylphenylsulfonyl, and especially 2,- 3- or 4-chlorophenylsulfonyl, 2,3-, 2,4-, 2,5-, 3,5- or 2,6-dichlorophenylsulfonyl, 2-chloro-4-cyanophenylsulfonyl or 4-fluoro-2-chlorophenylsulfonyl.

In unsubstituted or substituted heterocyclylsulfonyl wherein heterocyclyl has 3 to 14 ring atoms, unsubstituted or substituted heterocyclyl is preferably as described above; more preferred is isoxazolylsulfonyl wherein isoxazolyl is unsubstituted or substituted by one or more, e.g. up to two, independently selected lower alkyl moieties. Very preferred is 5-methyl- or 3,5-dimethyl-isoxazol-4-ylsulfonyl.

In unsubstituted or substituted C₆-C₁₄-arylcarbonyl, unsubstituted or substituted aryl is preferably as defined above; more preferred is benzoyl substituted by one or more, e.g. up to two, independently selected halo moieties, especially chloro. Very preferred is 2- or 3-chlorobenzoyl.

B₁ is N or CRo, B₂ is preferably CRm.

Ro and Rm lower alkyl is preferably methyl, halo (which is especially preferred) is especially chloro (very preferred) or fluoro, and lower alkoxy is preferably methoxy.

YR₁R₂ is preferably lower alkylamino, such as ethylamino, di-(lower alkyl)-amino, such as dimethylamino, unsubstituted amino-lower alkylamino, N-mono-, N,N-di- or N,N,N'-tri-(lower alkyl)-amino-lower alkyl-amino, such as 2-(N.N-dimethylamino)-ethylamino. 3-(N,N-dimethylamino)-ethylaminopropylamino, 4-(N,N-dimethylamino)-butylamino, 2-(N,N-dimethylamino)-ethyl-N'-methylamino or 3-(N,N-dimethylamino)-propyl-N'-methylamino, lower alkoxy-lower alkylamino, such as 2-methoxyethylamino or 3-methoxypropylamino, pyrrolidinyl-lower alkylamino, such as pyrrolidino-lower alkylamino, e.g. 2-pyrrolidinoethylamino or 3-pyrrolidinopropylamino, piperidinyl-lower alkylamino, such as piperidin-4-ylmethylamino, pyridyl-lower alkylamino, such as 2- or 3-pyridylmethylamino, C₃-C₆-cycloalkylamino, such as cyclopropylamino, piperidinylamino, such as piperidin-4-ylamino, N-lower alkylpiperidinylamino, such as N-methylpiperidin-4-ylamino, pyrrolidino, amino-, N-lower alkylamino- or N,N-di-lower alkylamino-pyrrolidino, such as 3-dimethylamino-pyrrolidino, amino-, N-lower alkylamino- or N,N-di-lower alkylamino-piperidino, such as 4-dimethylamino-piperidino, piperazino, N-lower alkylpiperazino, such as 4-methylpiperazino, N-lower alkanoyl-piperazino or N-lower alkanesulfonyl-piperazino, such as 4-methanesulfonylpiperazino.

Esterified hydroxyl (as Q in a compound of the formula XIII) is preferably acyloxy with acyl as defined above, especially as defined as preferred above. Examples are lower alkanoyloxy or benzoxyloxy.

Etherified hydroxyl (preferred over esterified hydroxyl as Q in a compound of the formula XIII) is preferably
- unsubstituted or substituted lower alkyloxy (a preferred substitutent) with unsubstituted or substituted lower alkyl as defined above; more especially lower alkoxy, such as methoxy, hydroxyl-lower alkoxy, such as 2-hydroxy-ethoxy, lower alkoxy-lower alkoxy, such as 2-methoxyethoxy, lower-alkoxy-lower-alkoxy-lower alkoxy, such as 2-(2-(methoxy)-ethoxy)-ethoxy, phenyl- or naphthyloxy, or phenyl- or naphthyl-lower alkoxy;
- unsubstituted or substituted C₃-C₁₀-cycloalkyloxy wherein unsubstituted or substituted C₃-C₁₀-cycloalkyl is preferably as defined above;
- unsubstituted or substituted C₆-C₁₄-aryloxy wherein unsubstituted or substituted C₆-C₁₄-aryl is preferably as defined above; or
- unsubstituted or substituted heterocyclyloxy with heterocyclyl with 3 to 14 ring atoms wherein unsubstituted or substituted heterocyclyl with 3 to 14 ring atoms is preferably as defined above.

Salts are especially the pharmaceutically acceptable salts of compounds of formula I. They can be formed where salt forming groups, such as basic or acidic groups, are present that can exist in dissociated form at least partially, e.g. in a pH range from 4 to 10 in aqueous environment, or can be isolated especially in solid form.

Such salts are formed, for example, as acid addition salts, preferably with organic or inorganic acids, from compounds of formula I with a basic nitrogen atom, especially the pharmaceutically acceptable salts. Suitable inorganic acids are, for example, halogen acids, such as hydrochloric acid, sulfuric acid, or phosphoric acid. Suitable organic acids are, for example, carboxylic, phosphonic, sulfonic or sulfamic acids, for example acetic acid, propionic acid, lactic acid, fumaric acid, succinic acid, citric acid, amino acids, such as glutamic acid or aspartic acid, maleic acid, hydroxymaleic acid, methylmaleic acid, benzoic acid, methane- or ethane-sulfonic acid, ethane-1,2-disulfonic acid, benzenesulfonic acid, 2-naphthalenesulfonic acid, 1,5-naphthalene-disulfonic acid, N-cyclohexylsulfamic acid. N-methyl-, N-ethyl- or N-propyl-sulfamic acid, or other organic protonic acids, such as ascorbic acid.

In the presence of negatively charged radicals, such as carboxy or sulfo, salts may also be formed with bases, e.g. metal or ammonium salts, such as alkali metal or alkaline earth metal salts, for example sodium, potassium, magnesium or calcium salts, or ammonium salts with ammonia or suitable organic amines, such as tertiary monoamines, for example triethylamine or tri(2-hydroxyethyl)amine, or heterocyclic bases, for example N-ethyl-piperidine or N,N'-dimethylpiperazine.

When a basic group and an acid group are present in the same molecule, a compound of formula I may also form internal salts.

For isolation or purification purposes it is also possible to use pharmaceutically unacceptable salts, for example picrates or perchlorates. For therapeutic use, only pharmaceutically acceptable salts or free compounds are employed (where applicable comprised in pharmaceutical preparations), and these are therefore preferred.

In view of the close relationship between the compounds in free form and in the form of their salts, including those salts that can be used as intermediates, for example in the purification or identification of the compounds or salts thereof, any reference to "compounds" (including also starting materials and "intermediates") hereinbefore and hereinafter, especially to the compound(s) of the formula I, is to be understood as referring also to one or more salts thereof or a mixture of a free compound and one or more salts thereof, each of which is intended to include also any solvate, metabolic precursor such as ester or amide of the compound of formula I, or salt of any one or more of these, as appropriate and expedient and if not explicitly mentioned otherwise. Different crystal forms may be obtainable and then are also included.

Where the plural form is used for compounds, salts, pharmaceutical preparations, diseases, disorders and the like, this is intended to mean also a single compound, salt, pharmaceutical preparation, disease or the like, and vice versa.

In some cases, a compound of the present invention comprises one or more chiral centers or show other asymmetry (leading to enantiomers) or may otherwise be able to exist in the form of more than one stereoisomer, e.g. due more than one chiral centers or more than one asymmetry or due to rings or double bonds that allow for Z/E (or cis-trans) isomerism (diastereomers). The present inventions includes both mixtures of two or more such isomers, such as mixtures of enantiomers, especially racemates, as well as preferably purified isomers, especially purified enantiomers or enantiomerically enriched mixtures.

The compounds of formula I (as well as some intermediates mentioned as preferred below) have valuable pharmacological properties and are useful in the treatment of kinase, especially Tie-2, dependent diseases, e.g., as drugs to treat one or more proliferative diseases.

The terms "treatment" or "therapy" (especially of tyrosine protein kinase dependent diseases or disorders) refer to the prophylactic or preferably therapeutic (including but not limited to palliative, curing, symptom-alleviating, symptom-reducing, kinase-regulating and/or kinase-inhibiting) treatment of said diseases, especially of the diseases mentioned below.

A warm-blooded animal (or patient) is preferably a mammal, especially a human.

Where subsequently or above the term "use" is mentioned (as verb or noun) (relating to the use of a compound of the formula I or a pharmaceutically acceptable salt thereof), this (if not indicated differently or suggested differently by the context) includes any one or more of the following embodiments of the invention, respectively (if not stated otherwise): the use in the treatment of a protein (especially tyrosine, more especially Tie-2) kinase dependent disease, the use for the manufacture of pharmaceutical compositions for use in the treatment of a protein kinase dependent disease, methods of use of one or more compounds of the formula I in the treatment of a protein kinase dependent and/or proliferative disease, pharmaceutical preparations comprising one or more compounds of the formula I for the treatment of said protein kinase dependent disease, and one or more compounds of the formula I in the treatment of said protein kinase dependent disease, as appropriate and expedient, if not stated otherwise. In particular, diseases to be treated and are thus preferred for "use" of a compound of formula I are selected from (especially tyrosine) protein kinase dependent ("dependent" meaning also "supported", not only "solely dependent") diseases mentioned below, especially proliferative diseases mentioned below, more especially any one or more of these or other diseases that depend on Tie-2, e.g. aberrantly highly-expressed, constitutively activated, normal and/or mutated Tie-2 kinase.

The (especially important and preferred) efficacy of compounds of the formula I as inhibitors or Tie-2 kinase can be demonstrated as follows:

### Tie-2 receptor autophosphorylation

The inhibition of Tie-2 receptor autophosphorylation can be confirmed with an *in vitro* experiment in cells such as transfected COS cells (ATCC Number: CRL-1651), which permanently express human Tie-2 (SwissProt AccNo Q02763), are seeded in complete culture medium (with 10% fetal calf serum = FCS) in 6-well cell-culture plates and incubated at 37°C under 5% CO₂ until they show about 90% confluency. The compounds to be tested are then diluted in culture medium (without FCS, with 0.1% bovine serum albumin) and added to the cells. Controls comprise medium without test compounds. After 40min of incubation at 37°C, ortho vanadate is added to give the final concentration of 10 mM. After a further incubation for 20 minutes at 37°C, the cells are washed twice with ice-cold PBS (phosphate-buffered saline) and immediately lysed in 100 µl lysis buffer per well. The lysates are then centrifuged to remove the cell nuclei, and the protein concentrations of the supernatants are determined using a commercial protein assay (BIORAD). The lysates can then either be immediately used or, if necessary, stored at -20°C.

A sandwich ELISA is carried out to measure the Tie-2 phosphorylation: a monoclonal antibody to Tie-2 (for example anti-Tie2 clone AB33, Upstate, Cat Nr. 05-584 or comparable monoclonal antibody) is immobilized using 0.1 ml of a 2µg/ml solution on black ELISA plates (OptiPlate™ HTRF-96 from Packard). The plates are then washed and the remaining free protein-binding sites are saturated with 3% TopBlock® (Juro, Cat. # TB232010) in phosphate buffered saline with Tween 20® (polyoxyethylen(20)sorbitane monolaurate, ICI/Uniquema) (PBST). The cell lysates (100 µg protein per well) are then incubated in these plates overnight at 4°C together with an antiphosphotyrosine antibody coupled with alkaline phosphatase (PY20:AP from Zymed). The (plates are washed again and the) binding of the antiphosphotyrosine antibody to the captured phosphorylated receptor is then demonstrated using a luminescent AP substrate (CDP-Star, ready to use, with Emerald II; Applied Biosystems). The luminescence is measured in a Packard Top Count Microplate Scintillation Counter. The difference between the signal of the positive control (stimulated with vanadate) and that of the negative control (not stimulated) corresponds to maximum Tie-2 phosphorylation (= 100 %). The activity of the tested substances is calculated as percent inhibition of maximum Tie-2 phosphorylation, and the concentration of substance that induces half the maximum inhibition is defined as the IC₅₀ (inhibitory dose for 50% inhibition). For compounds of the formula I, XII or XIII, preferably IC₅₀ values in the range from 0.005to 10 µM can be found, e.g. more preferably from 0.005 to 6 µM.

### KDR Autophosphorylation

The activity of the compounds of the invention as inhibitors of KDR protein-tyrosine kinase activity can be tested as follows: The inhibition of VEGF-induced receptor autophosphorylation can be confirmed in cells such as transfected CHO cells, which permanently express human VEGF-R2 receptor (KDR), and are seeded in complete culture medium (with 10% fetal calf serum = FCS) in 6-well cell-culture plates and incubated at 37°C under 5% CO₂ until they show about 80% confluency. The compounds to be tested are then diluted in culture medium (without FCS, with 0.1% bovine serum albumin) and added to the cells. Controls comprise medium without test compounds. After 2h incubation at 37°C, recombinant VEGF is added; the final VEGF concentration is 20 ng/ml. After a further incubation period of five minutes at 37°C, the cells are washed twice with ice-cold PBS (phosphate-buffered saline) and immediately lysed in 100 µl lysis buffer per well. The lysates are then centrifuged to remove the cell nuclei, and the protein concentrations of the supernatants are determined using a commercial protein assay (BIORAD). The lysates can then either be immediately used or, if necessary, stored at -20°C. With this assay it can be shown that the compounds of the present invention can show IC₅₀ values for inhibition that are higher (less inhibition) than in the Tie-2 assay. Especially compounds of the formula I wherein R5 is an unsubstituted or substituted (aryl, heterocyclyl or alkane)sulfonyl are selective for Tie-2, while other compounds of the formula I may also be useful as dual inhibitors for both KDR and Tie-2..

A good selectivity can also be found using *in vitro* assays known in the art against one or more kinases selected from the group consisting of CDK1; IGF-R, insulin receptor kinase, Eph-B4, Raf (e.g. b- and/or c-Raf), Flt-3, Her-1 and FGF-R3. Test systems for many of these are known in the art, see e.g. WO 2005/070431.

The results indicate an advantageous selectivity profile of compounds of the formula I with a quite specific inhibition for Tie-2 kinase, where selectivity does not necessarily mean that only Tie-2 kinase is inhibited to an advantageous and pharmaceutically relevant extent - instead also other kinases, e.g. c-Abl, Bcr-Abl, c-Kit, c-Raf, Flt-1, Flt-3, KDR, Her-1, PDGFR-kinase, c-Src, RET-receptor kinase, FGF-R1, FGF-R2, FGF-R3, FGF-R4, Ephrin receptor kinases (e. g., EphB2 kinase, EphB4 kinase and related Eph kinases), casein kinases (CK-1, CK-2, G-CK), Pak, ALK, ZAP70, Jak1, Jak2, Axl, Cdk1, cdk4, cdk5, Met, FAK, Pyk2, Syk, Insulin receptor kinase, or (especially constitutively activating) mutations of kinases (active-ting kinases) such as of Bcr-Abl, c-Kit, c-Raf, Flt-3, FGF-R3, PDGF-receptors, RET, and Met, may also be inhibited to an extent to support usefulness in connection with the Tie-2 inhibition.

The efficiency of the compounds of the formula I as inhibitors of tumor growth can be demonstrated as follows:

For example, in order to test whether a compound of the formula linhibits VEGF-mediated angiogenesis in vivo, its effect on the angiogenic response induced by VEGF in a growth factor implant model in mice can be tested: A porous Teflon chamber (volume 0.5 mL) is filled with 0.8 % w/v agar containing heparin (20 units/ml) with or without growth factor (2 µg/ml human VEGF) is implanted subcutaneously on the dorsal flank of C57/C6 mice. The mice are treated with the test compound (e.g. 25, 50 or 100 mg/kg p.o. once daily) or vehicle starting on the day of implantation of the chamber and continuing for 4 days after. At the end of the treatment, the mice are killed, and the chambers are removed. The vascularized tissue growing around the chamber is carefully removed and weighed, and the blood content is assessed by measuring the hemoglobin content of the tissue (Drabkins method; Sigma, Deisenhofen, Germany). It has been shown previously that these growth factors induce dose-dependent increases in weight and blood content of this tissue growing (characterized histologically to contain fibroblasts and small blood vessels) around the chambers and that this response is blocked by antibodies that specifically neutralize VEGF (see Wood JM et al., Cancer Res. 60(8), 2178-2189, (2000); and Schlaeppi et al., J. Cancer Res. Clin. Oncol. 125, 336-342, (1999)).

In view of the high expression of the Tie-2 antagonist angiopoietin-2 expression of which is up-regulated at sites where angiogenesis takes place, this result is corroborating surprising former findings. In addition, although VEGF has been used to stimulate angiogenesis in the *in vivo* model, selective Tie-2 inhibitors are sufficient to inhibit angiogenesis. Therefore the compounds of the present invention can support treatments inhibiting VEGF-driven angiogenesis or replace them, especially where they are not successful, and thus are a very good addition to the arsenal of antitumor drugs and therapies.

Angiogenesis is regarded as a prerequisite for those tumors which grow beyond a maximum diameter of about 1-2 mm; up to this limit, oxygen and nutrients may be supplied to the tumor cells by diffusion. Every tumor, regardless of its origin and its cause, is thus dependent on angiogenesis for its growth after it has reached a certain size. Three principal mechanisms play an important role in the activity of angiogenesis inhibitors against tumors: 1) Inhibition of the growth of vessels, especially capillaries, into avascular resting tumors, with the result that there is no net tumor growth owing to the balance that is achieved between apoptosis and proliferation; 2) Prevention of the migration of tumor cells owing to the absence of blood flow to and from tumors; and 3) Inhibition of endothelial cell proliferation, thus avoiding the paracrine growth-stimulating effect exerted on the surrounding tissue by the endothelial cells normally lining the vessels.

In a preferred sense of the invention, a disease or disorder dependent on activity of a protein (preferably tyrosine) kinase, especially Tie-2, where a compound of the formula I can be used is one or more of a proliferative disease (meaning one dependent on inadequate including a hyperproliferative condition, such as one or more of leukemia, hyperplasia, fibrosis (especially pulmonary, but also other types of fibrosis, such as renal fibrosis or hepatic cirrhosis), angiogenesis, psoriasis, atherosclerosis and smooth muscle proliferation in the blood vessels, such as stenosis or restenosis following angioplasty. Further, a compound of the formula I may be used for the treatment of thrombosis and/or scleroderma.

Preferred is the use of a compound of the formula I in the therapy (including prophylaxis) of a proliferative disorder (especially which is dependent on (for example inadequate) Tie-2 activity) selected from tumor or cancer diseases, especially against preferably a benign or especially malignant tumor or cancer disease, more preferably solid tumors, e.g. carcinoma of the brain, kidney, liver, adrenal gland, bladder, breast, stomach (especially gastric tumors), ovaries, cervix, endometrium, colon, rectum, prostate, pancreas, lung (e.g. small or large cell lung carcinomas), vagina, thyroid, sarcoma, glioblastomas, myeloma, especially multiple myeloma or gastrointestinal cancer, especially colon carcinoma or colorectal adenoma, skin cancer, e.g. melanoma, Kaposi's sarcoma, a tumor of the neck and head, e.g. squameous carcinoma of the head and neck, including neoplasias, especially of epithelial character, e.g. in the case of mammary carcinoma; an epidermal hyperproliferation (other than cancer), especially psoriasis; prostate hyperplasia; malignant pleural mesotherioma; lymphoma; or further liquid tumors, e.g. leukemia.

A compound of formula I or its use makes it possible to bring about the regression of tumors and to prevent the formation of tumor metastases and the growth of (also micro)metastases.

Compounds of the formula I, in regard of their ability to inhibit Tie-2 kinase, and thus to modulate angiogenesis, are especially appropriate for the use against diseases or disorders related to the inadequate activity of Tie-2 kinase, especially an overexpression thereof.

The compounds of the formula I are especially of use to prevent or treat the mentioned and other diseases that are triggered by persistent angiogenesis, such as restenosis, e.g., stent-induced restenosis; Crohn's disease; Hodgkin's disease; malignant nephrosclerosis; thrombotic microangiopathic syndromes; (e.g. chronic) transplant rejections and glomerulopathy; mesangial cell-proliferative diseases; injuries of the nerve tissue; for inhibiting the re-occlusion of vessels after balloon catheter treatment, for use in vascular prosthetics or after inserting mechanical devices for holding vessels open, such as, e.g., stents, as immunosuppressants, as an aid in scar-free wound healing, and for treating age spots and contact dermatitis, diseases caused by ocular neovascularisation, especially (e.g. ischemic) retinopathies such as diabetic retinopathy, neovascular glaucoma or (e.g. age-related) macula degeneration, Von Hippel Lindau disease, hemangioblastoma, (haem)angioma, mesangial cell proliferative disorders such as chronic or acute renal diseases, e.g. diabetic nephropathy, obesity, malignant nephrosclerosis, thrombotic microangiopathy syndromes or transplant rejection, or especially inflammatory renal disease, such as glomerulonephritis, especially mesangioproliferative glomerulonephritis, haemolytic-uraemic syndrome, diabetic nephropathy, hypertensive nephroscterosis, atheroma, arterial restenosis, autoimmune and/or inflammatory diseases, e.g. acute inflammation, rheumatoid arthritis, inflammatory bowel disease, rheumatoid inflammatory diseases or other chronic inflammatory disorders, diabetes, endometriosis, chronic asthma, arterial or post-transplantational atherosclerosis, neurodegenerative disorders, and especially neoplastic diseases such as cancers (especially solid tumours but also leukemias as mentioned above), myelodysplastic syndrome, AML (acute myeloid leukemia), AMM (agnogenic myeloid metaplasia), mesothelioma, glioma and glioblastoma.

Preferably, the invention relates to the use of compounds of the formula I, or pharmaceutically acceptable salts thereof, in the treatment of solid tumors as mentioned herein.

Where above or subsequently the term "use" is mentioned, this includes any one or more of the following embodiments of the invention, respectively: the use of a compound of the formula I in the treatment of (especially tyrosine) protein kinase dependent diseases, its use for the manufacture of pharmaceutical compositions for use in the treatment of said diseases, methods of use of a compound of the formula I in the treatment of said diseases, pharmaceutical preparations comprising a compound of the formula I for the treatment of said diseases, and a compound of the formula I for use in the treatment of said diseases, as appropriate and expedient, if not stated otherwise. In particular, diseases to be treated and are thus preferred for USE of a compound of formula (I) are selected from (especially tyrosine) protein kinase dependent ("dependent" meaning also "supported", not only "solely dependent") diseases mentioned above, especially corresponding proliferative diseases, more especially diseases that depend on Tie-2.

### Process of Manufacture

A compound of formula I can be prepared analogously to methods that, for other compounds, are in principle known in the art, so that for the novel compounds of the formula I the process is novel as analogy process, preferably by reacting
a) a compound of the formula II, 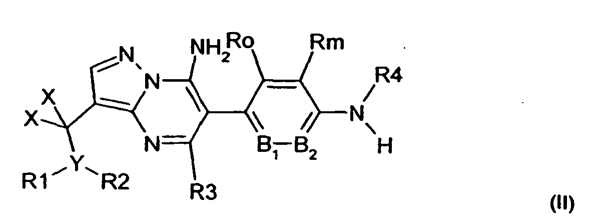 wherein R1, R2, X, R3, B₁, B₂, Ro, Rm and R4 are as defined for a compound of the formula I, with an acid of the formula III,

   R5-OH (III)

   wherein R5 is as defined for a compound of the formula I, or a reactive derivative thereof capable of introducing a moiety R5 as defined for a compound of the formula I, or
b) for the manufacture of a compound of the formula I wherein the two symbols X together are oxo (=O) and the remaining symbols are as defined for a compound of the formula I, an acid of the formula IV, 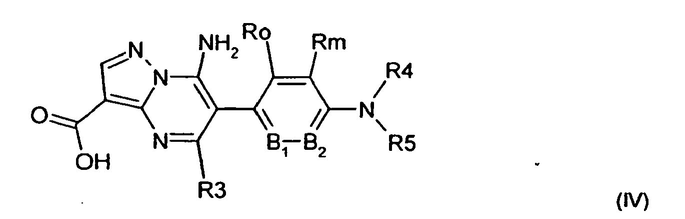 wherein R2, Ro, Rm, B₁, B₂, R4 and R5 are as defined for a compound of the formula I, or a reactive derivative thereof, with a compound of the formula V, 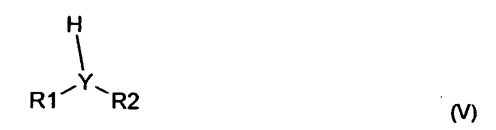 wherein R1, R2 and Y are as defined for a compound of the formula I, or
c) for the manufacture of a compound of the formula I wherein each X is hydrogen and the other symbols are as defined for a compound of the formula I, a compound of the formula VI, 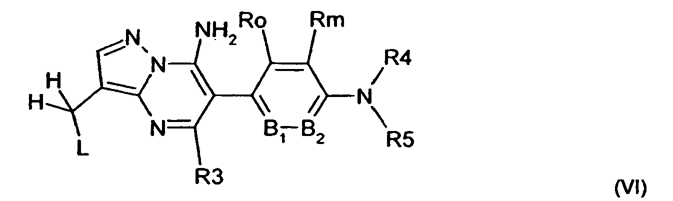
wherein L is a leaving group and R3, Ro, Rm, R4, R5, B₁ and B₂ are as defined for a compound of the formula I, with a compound of the formula V as defined under b);
and, if desired, transforming a compound of formula I into a different compound of formula I, transforming a salt of an obtainable compound of formula I into the free compound or a different salt, transforming an obtainable free compound of formula I into a salt thereof, and/or separating an obtainable mixture of isomers of a compound of formula I into individual isomers.

The reaction under a) preferably takes place under customary conditions for the formation of amide bonds, and the acid of the formula III is either used as such and a reactive derivative is formed in situ, e.g. by dissolving the compounds of formulae II and III in a suitable solvent, for example *N,N*-dimethylformamide, *N,N*-dimethylacetamide, *N*-methyl-2-pyrrolidone, methylene chloride, tetrahydrofurane or a mixture of two or more such solvents, and/or at least one suitable base, for example triethylamine, diisopropylethylamine (DIEA), *N*-methylmorpholine or pyridine, together with a suitable coupling agent that forms a preferred reactive derivative of the carbonic acid of formula III *in situ,* for example dicyclohexylcarbodiimide/1-hydroxybenzotriazole (DCC/ HOBT); *O*-(1,2-dihydro-2-oxo-1-pyridyl)-*N*,*N*,*N*'.*N*'-tetramethyluronium tetrafluoroborate (TPTU); O-benzotriazol-1-yl)-N,N,N', N'-tetramethyluronium tetrafluoroborate (TBTU); or 1-(3-dimethylaminopropyl)-3-ethylcarbodiimide hydrochloride (EDC) (for a review of other possible coupling agents, see e.g. Klauser; Bodansky, Synthesis 1972, 453-463), preferably reacting at a temperature of between approximately -20 and 50 °C, especially between 0 °C and room temperature, to yield a compound of formula I. Alternatively, the acid of the formula III is used in the form of a reactive derivative, e.g. as the acid halide, such as chloride, as an anhydride, as an active ester, or, if the moiety R5 to be introduced is a substituted aminocarbonyl moiety, especially unsubstituted or substituted C₆-C₁₄-arylaminoarbonyl or unsubstituted or substituted heterocyclylaminocarbonyl, using a corresponding isocyanate precursor where, during the reaction, the isocyanato group forms the aminocarbonyl group, preferably in the presence of a base and/or a solvent and at preferred temperatures as just described.

For the reaction under b), either a carbonic acid or a reactive derivative is either an acid halide, such as chloride, an anhydride or an active ester of a carbonic acid of the formula I, or preferably the active derivative is formed in situ. Appropriate solvents, reaction temperatures and/or bases to be added and other reaction conditions are e.g. as defined under reaction a).

A leaving group L in reaction c) is preferably halo or arylsulfonyloxy, such as tolylsulfonyloxy, or alkanesulfonyloxy, such as methanesulfonyloxy. The reaction preferably takes place under customary substitution conditions, e.g. in the presence of an appropriate solvent or solvent mixture, such as tetrahydrofurane, at preferred termperatures in the range from 0 to 50 °C, e.g. at room temperature.

### Optional Reactions and Conversions

Compounds of the formula I, or protected forms thereof directly obtained according to any one of the preceding procedures or after introducing protecting groups anew, which are included subsequently as starting materials for conversions as well even if not mentioned specifically, can be converted into different compounds of the formula I according to known procedures, where required followed by removal of protecting groups.

For example, in a compound of the formula I wherein the two substituents X together are oxo, the oxo can be converted into thioxo e.g. in the presence of an appropriate thionation agent, such as Lawesson's reagent under appropriate customary conditions.

In a compound of the formula I wherein R4 is hydrogen and the other symbols have the meanings defined under formula I, a moiety R4 = unsubstituted or substituted alkyl can be introduced by reaction of a compound of the formula I wherein R4 is hydrogen with an alkylating agent, e.g. a compound of the formula VII,

R4-G (VII)

wherein R4 is unsubstituted or substituted alkyl and G is a leaving group, such as halo, especially chloro, bromo or iodo, arylsulfonyloxy, such as toluolsulfonyloxy, or alkanesulfonyloxy, such as methansulfonyloxy, under customary reaction conditions and in the presence of appropriate solvents. If required, the 7-amino group at the central pyrazolo[1,5-a]pyrimidin ring can be protected before (also aready on an intermediate stage) and deprotected after the alkylation in a customary way.

In the examples, appropriate reaction conditions can be found that may be used for analogous conversions of different compounds of the formula I.

Salts of compounds of formula I having at least one salt-forming group may be prepared in a manner known *per se*. For example, salts of compounds of formula I having acid groups may be formed, for example, by treating the compounds with metal compounds, such as alkali metal salts of suitable organic carboxylic acids, e.g. the sodium salt of 2-ethylhexanoic acid, with organic alkali metal or alkaline earth metal compounds, such as the corresponding hydroxides, carbonates or hydrogen carbonates, such as sodium or potassium hydroxide, carbonate or hydrogen carbonate, with corresponding calcium compounds or with ammonia or a suitable organic amine, stoichiometric amounts or only a small excess of the salt-forming agent preferably being used. Acid addition salts of compounds of formula I are obtained in customary manner, e.g. by treating the compounds with an acid or a suitable anion exchange reagent. Internal salts of compounds of formula I containing acid and basic salt-forming groups, e.g. a free carboxy group and a free amino group, may be formed, e.g. by the neutralisation of salts, such as acid addition salts, to the isoelectric point, e.g. with weak bases, or by treatment with ion exchangers.

A salt of a compound of the formula I can be converted in customary manner into the free compound; metal and ammonium salts can be converted, for example, by treatment with suitable acids, and acid addition salts, for example, by treatment with a suitable basic agent. In both cases, suitable ion exchangers may be used.

Stereoisomeric mixtures, e.g. mixtures of diastereomers, can be separated into their corresponding isomers in a manner known per se by means of appropriate separation methods. Diastereomeric mixtures for example may be separated into their individual diastereomers by means of fractionated crystallization, chromatography, solvent distribution, and similar procedures. This separation may take place either at the level of one of the starting compounds or in a compound of formula I itself. Enantiomers may be separated through the formation of diastereomeric salts, for example by salt formation with an enantiomer-pure chiral acid, or by means of chromatography, for example by HPLC, using chromatographic substrates with chiral ligands.

Intermediates and final products can be worked up and/or purified according to standard methods, e.g. using chromatographic methods, distribution methods, (re-) crystallization, and the like.

### Starting Materials

Starting Materials, including intermediates, for compounds of the formula I, such as the compounds of the formulae II, III, IV, V and VI, can be prepared, for example, according to methods that are known in the art, according to methods described in the examples and the section below with the title Examples, or to methods analogous to those described in the examples or the section below with the title Examples, and/or they are known or commercially available.

In the subsequent description of starting materials and intermediates and their synthesis (as well as in general parts of the Examples), R1, R2, R3, R4, R5, X, Y, Ro, Rm, B₁ and B₂ have the meanings given above for the corresponding starting materials or herein for compounds of the formula I or especially in the Examples for the respective starting materials or intermediates, if not indicated otherwise directly or by the context. Protecting groups, if not specifically mentioned, can be introduced and removed at appropriate steps in order to prevent functional groups, the reaction of which is not desired in the corresponding reaction step or steps, employing protecting groups, methods for their introduction and their removal are as described above or below, e.g. in the references mentioned under "General Process Conditions". The person skilled in the art will readily be able to decide whether and which protecting groups are useful or required.

Intermediates of the formula II wherein X and X together form oxo (=O) and the other symbols are as defined for a compound of the formula I can be prepared as described for intermediates INT7 (which fall under formula I) in the General schemes in the Examples section, or in analogy thereto.

Intermediates of the formula II wherein each X is hydrogen can be obtained by reacting a compound of the formula INT2 (see General Scheme 1 and 2 in the Examples Section), if required under protection of the amino group(s) and deprotection at an appropriate stage, in the presence of an appropriate reductant, such as Lithium-Aluminium-hydride (LAH), in an appropriate solvent, such as tetrahydrofurane, e.g. at temperatures from 0 to 50°C, to give a compound of the formula VIII, 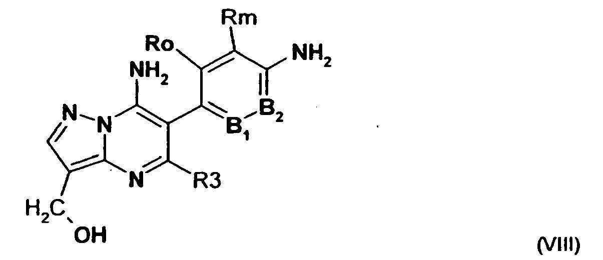 wherein R3, Ro, Rm, B₁ and B₂ are as defined for a compound of the formula I, which can then be converted to a compound of the formula II wherein each X is hydrogen by introduction of the leaving group L e.g. with an arylsulfonyl- or alkanesulfonylhalogenide (to give an arylsulfonyloxy or alkanesulfonyloxy group L) or with an inorganic acid chloride, such as thionylchloride, in an appropriate solvent, such as tetrahydrofurane, e.g. at temperatures from 0 to 50°C, e.g. at ambient temperature.

An intermediate of the formula IV can, for example, be obtained as or in analogy to the intermediates INT4-1, INT4-2, INT4-3 and INT4-4 and the preceding steps including the precursors by the methods given in the Reaction Schemes in the Examples section.

An intermediate of the formula VI can, for example, be obtained by reducing a compound of the formula IX, 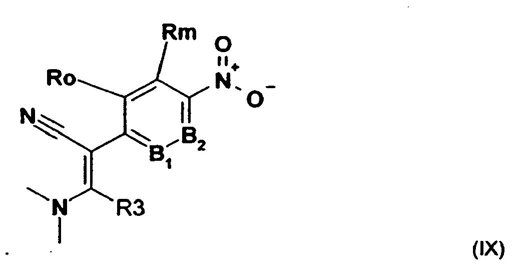 (obtainable e.g. as described in WO 2005/054238), wherein R3, Ro, Rm, B₁ and B₂ are as defined for a compound of the formula I, in the presence of an appropriate reductanct, e.g. hydrogen in the presence of a catalyst such as Raney-Nickel in an appropriate solvent, such as methanol at temperatures e.g. in the range from 0 to 50°C, to the corresponding amine of the formula X, 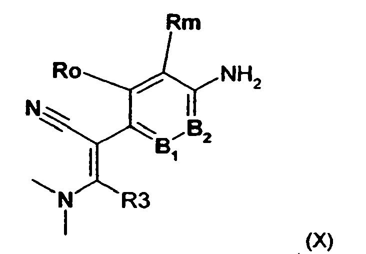 which can then be acylated in analogy to manufacturing process variant a) with a compound of the formula III and then if desired replacing a hydrogen R4 by unsubstituted or substituted alkyl R4 by reaction with a compound of the formula VII under alkylation conditions as described for conversion reactions of compounds of the formula I to give a compound of the formula XI, 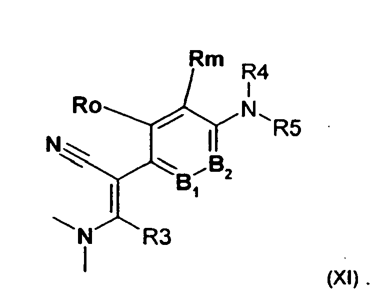 which is then reacted with 5-amino-1H-pyrazole-4-carboxylic acid lower alkyl (e.g. ethyl) ester to a compound of the formula XII 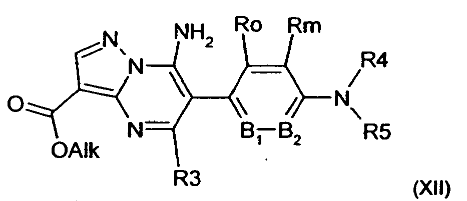 wherein Alk is unsubstituted or substituted lower alkyl, preferably lower alkyl, e.g. ethyl (which is an ester of a compound of the formula IV) which can then be reduced to a compound of the formula XIII, 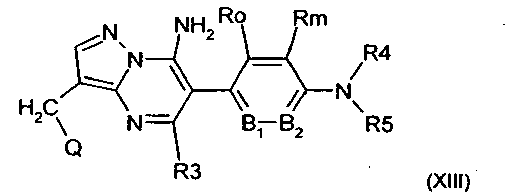 wherein Q is hydroxyl (which can be converted to a compound of the formula XIII wherein Q is esterified hydroxyl by introduction of acyl, e.g. with an acyl halogenide, in the presence of a tertiary nitrogen base, or under other acylation conditions comparable to those for reaction b) above between compounds of the formulae IV and V) in the presence of an appropriate reductant, such as Lithium-Aluminium-hydride (LAH), in an appropriate solvent, such as tetrahydrofurane, e.g. at temperatures from 0 to 50°C, which can then be converted to a compound of the formula VI by introduction of the leaving group L e.g. with an arylsulfonyl- or alkanesulfonylhalogenide (to give an arylsulfonyloxy or alkanesulfonyloxy group L) or with an inorganic acid chloride, such as thionylchloride, in an appropriate solvent, such as tetrahydrofurane, e.g. at temperatures from 0 to 50°C, e.g. at ambient temperature.

For the manufacture of a compound of the formula XIII wherein Q is etherified alkoxy, it is possible to react the etherifiying alcohol with a compound of the formula VI under comparable reaction conditions as given above under c) for the reaction of compounds of the formulae VI and V.

Other starting materials, e.g. those of the formula III, V or VII, are known in the art, commercially available and/or can be prepared according to standard procedures, e.g. in analogy to or by methods described in the Examples.

### General process conditions

The following applies in general to all processes mentioned hereinbefore and hereinafter, while reaction conditions specifically mentioned above or below are preferred:

In any of the reactions mentioned hereinbefore and hereinafter, protecting groups may be used where appropriate or desired, even if this is not mentioned specifically, to protect functional groups that are not intended to take part in a given reaction, and they can be introduced and/or removed at appropriate or desired stages. Reactions comprising the use of protecting groups are therefore included as possible wherever reactions without specific mentioning of protection and/or deprotection are described in this specification.

Within the scope of this disclosure only a readily removable group that is not a constituent of the particular desired end product of formula I is designated a "protecting group", unless the context indicates otherwise. The protection of functional groups by such protecting groups, the protecting groups themselves, and the reactions appropriate for their removal are described for example in standard reference works, such as J. F. W. McOmie, "Protective Groups in Organic Chemistry", Plenum Press, London and New York 1973, in T. W. Greene and P. G. M. Wuts, "Protective Groups in Organic Synthesis", Third edition, Wiley, New York 1999, in "The Peptides"; Volume 3 (editors: E. Gross and J. Meienhofer), Academic Press, London and New York 1981, in "Methoden der organischen Chemie" (Methods of Organic Chemistry), Houben Weyl, 4th edition, Volume 15/I, Georg Thieme Verlag, Stuttgart 1974, in H.-D. Jakubke and H. Jeschkeit, "Aminosäuren. Peptide, Proteine" (Amino acids, Peptides, Proteins), Verlag Chemie, Weinheim, Deerfield Beach, and Basel 1982, and in Jochen Lehmann, "Chemie der Kohlenhydrate: Monosaccharide und Derivate" (Chemistry of Carbohydrates: Monosaccharides and Derivatives), Georg Thieme Verlag, Stuttgart 1974. A characteristic of protecting groups is that they can be removed readily (i.e. without the occurrence of undesired secondary reactions) for example by solvolysis, reduction, photolysis or alternatively under physiological conditions (e.g. by enzymatic cleavage).

All the above-mentioned process steps can be carried out under reaction conditions that are known per se, preferably those mentioned specifically, in the absence or, customarily, in the presence of solvents or diluents, preferably solvents or diluents that are inert towards the reagents used and dissolve them, in the absence or presence of catalysts, condensation or neutralizing agents, for example ion exchangers, such as cation exchangers, e.g. in the H⁺ form, depending on the nature of the reaction and/or of the reactants at reduced, normal or elevated temperature, for example in a temperature range of from about -100 °C to about 190°C, preferably from approximately -80°C to approximately 150°C, for example at from -80 to -60°C, at room temperature, at from -20 to 40 °C or at reflux temperature, under atmospheric pressure or in a closed vessel, where appropriate under pressure, and/or in an inert atmosphere, for example under an argon or nitrogen atmosphere.

The solvents from which those solvents that are suitable for any particular reaction may be selected include those mentioned specifically or, for example, water, esters, such as lower alkyl-lower alkanoates, for example ethyl acetate, ethers, such as aliphatic ethers, for example diethyl ether, or cyclic ethers, for example tetrahydrofurane or dioxane, liquid aromatic hydrocarbons, such as benzene or toluene, alcohols, such as methanol, ethanol or 1- or 2-propanol, nitriles, such as acetonitrile, halogenated hydrocarbons, e.g. as methylene chloride or chloroform, acid amides, such as dimethylformamide or dimethyl acetamide, bases, such as heterocyclic nitrogen bases, for example pyridine or N-methylpyrrolidin-2-one, carboxylic acid anhydrides, such as lower alkanoic acid anhydrides, for example acetic anhydride, cyclic, linear or branched hydrocarbons, such as cyclohexane, hexane or isopentane, or mixtures of these, for example aqueous solutions, unless otherwise indicated in the description of the processes. Such solvent mixtures may also be used in working up, for example by chromatography or partitioning.

Intermediates and final products can be worked up and/or purified according to standard methods, e.g. using chromatographic methods, distribution methods, (re-) crystallization, distillation (under normal or reduced pressure), steam distillation and the like.

The invention relates also to those forms of the process in which a compound obtainable as intermediate at any stage of the process is used as starting material and the remaining process steps are carried out, or in which a starting material is formed under the reaction conditions or is used in the form of a derivative, for example in protected form or in the form of a salt, or a compound obtainable by the process according to the invention is produced under the process conditions and processed further in situ. In the process of the present invention those starting materials are preferably used which result in compounds of formula I described as being preferred. Special preference is given to reaction conditions that are identical or analogous to those mentioned in the Examples. The invention also relates to novel starting materials and also especially to a compound of the formula XII (also a starting material) or a compound of the formula XIII (a starting material with Q = hydroxyl or a derivative thereof), for the formulae see above.

### Preferred embodiments according to the invention:

In the following preferred embodiments as well as in preceding and following embodiments of more general scope, any one or more or all general expressions can be replaced by the corresponding_more specific definitions provided above and below, thus yielding stronger preferred embodiments of the invention.

Preferred is a compound of the formula I, wherein
R5 is unsubstituted or substituted C₆-C₁₄-arylaminocarbonyl which is especially preferred, unsubstituted or substituted heterocyclylaminocarbonyl which is especially preferred and wherein heterocyclyl has 3 to 14 ring atoms, unsubstituted or substituted C₆-C₁₄-arylaminosulfonyl, unsubstituted or substituted heterocyclylaminosulfonyl wherein heterocyclyl has 3 to 14 ring atoms, unsubstituted or substituted lower-alkanesulfonyl which is especially preferred, unsubstituted or substituted C₆-C₁₄-arylsulfonyl which is especially preferred, unsubstituted or substituted heterocyclylsulfonyl wherein heterocyclyl has 3 to 14 ring atoms, or unsubstituted or substituted C₆-C₁₄-arylcarbonyl,
and the other symbols R1, R2, Y, X, R3, R4, B₁, B₂, Ro and Rm have the meanings given in claim 1,
or a (preferably pharmaceutically acceptable) salt thereof.

Also preferred is a compound of the formula I, wherein
R5 is phenylaminocarbonyl wherein phenyl is unsubstituted or substituted by one or more moieties independently selected from lower alkyl, halo (very preferred), especially chloro; halo-lower alkyl, lower alkoxy and cyano;
pyrazolyl-aminocarbonyl or isoxazolylaminocarbonyl where pyrazolyl or isoxazolyl is unsubstituted or substituted by one or two moieties independently selected from the group consisting of lower alkyl and phenyl that is unsubstituted or substituted with halo, lower alkoxy, piperazino-lower alkyl, 4-lower alkylpiperazino-lower alkyl and morpholino-lower alkyl; pyrazolyl-aminosulfonyl or isoxazolylaminosulfonyl, where each pyrazolyl or isoxazolyl is unsubstituted or substituted by one or two moieties independently selected from the group consisting of lower alkyl and phenyl that is unsubstituted or substituted with halo, lower alkoxy, piperazino-lower alkyl, 4-lower alkylpiperazino-lower alkyl and morpholino-lower alkyl; phenyl-lower alkanesulfonyl, wherein phenyl is unsubstituted (preferred) or substituted with one or more, e.g. up to three, moieties independently selected from the group consisting of lower alkyl, halo (especially preferred), halo-lower alkyl, lower alkoxy and cyano; phenylsulfonyl wherein the phenyl is unsubstituted or substituted by one or more moieties independently selected from the group consisting of lower alkyl, halo (preferred), halo-lower alkyl, lower alkoxy and cyano;
and the other symbols R1, R2, X, Y, R3, R4, B₁, B₂, Ro and Rm have the meanings given for a compound of the formula I in claim 1,
or a (preferably pharmaceutically acceptable) salt thereof.

More preferred is a compound of the formula I wherein
R5 is 3-trifluoromethyl-phenylaminocarbonyl, 4-fluorophenylaminocarbonyl, 3- or 2-chlorophenylaminocarbonyl, 3-tert-butyl-1-(4-fluorophenyl)-pyrazol-5-ylaminocarbonyl, 3-tert-butyl-1-(4-methoxyphenyl)-pyrazol-5-ylaminocarbonyl, 3-tert-butyl-1-(4-(4-methylpiperazinomethyl)-phenyl)-pyrazol-5-ylaminocarbonyl, 3-tert-butyl-1-(3-(4-methylpiperazinomethyl)-phenyl)-pyrazol-5-ylaminocarbonyl, 3-tert-butyl-1-(4-(morpholinomethyl)-phenyl)-pyrazol-5-ylaminocarbonyl, 5-tert-butyl-isoxazol-3-ylaminocarbonyl, 3-tert-butyl-1-(4-fluorophenyl)-pyrazol-5-ylaminosulfonyl, phenylmethylsulfonyl or 2-phenylethylsulfonyl, 2,3-dimethylphenylsulfonyl, 2-, 3- or 4-methylphenylsulfonyl, 2-, 3- or 4-methoxyphenylsulfonyl, 2-methyl-4,5-dimethoxyphenylsulfonyl, 2,5-dimethoxyphenylsulfonyl, 2-, 3- or 4-trifluoromethylphenylsulfonyl, 2-chloro-5-trifluoromethylphenylsulfonyl, 2-chloro-4-trifluoromethylphenylsulfonyl, or especially 2,- 3- or 4-chlorophenylsulfonyl, 2,3-, 2,4-, 2,5-, 3,5- or 2,6-dichlorophenylsulfonyl, 2-chloro-4-cyanophenylsulfonyl or 4-fluoro-2-chlorophenylsulfonyl; and the other symbols R1, R2, X, Y, R3, R4, B_{1,} B₂, Ro and Rm have the meanings given in claim 1, or a (preferably pharmaceutically acceptable) salt thereof.

Very preferred is also a compound of the formula I according to claim 1 or any preceding paragraph in this section on preferred embodiments wherein
R1R2Y - taken together - is lower alkylamino, di-(lower alkyl)-amino, unsubstituted amino-lower alkylamino, N-mono-, N,N-di- or N,N,N'-tri-(lower alkyl)-amino-lower alkyl-amino, lower alkoxy-lower alkylamino, pyrrolidinyl-lower alkylamino, oxopyrrolidinyl-lower alkylamino, piperidinyl-lower alkylamino, N-lower alkylpiperidinyl-lower alkylamino, pyridyl-lower alkylamino, C₃-C₆-cycloalkylamino, piperidinylamino, N-lower alkylpiperidinylamino, pyrrolidino, amino-, N-lower alkylamino- or N,N-di-lower alkylamino-pyrrolidino, amino-, N-lower alkylamino- or N,N-di-lower alkylamino-piperidino, piperazino, N-lower alkylpiperazino, N-lower alkanoyl-piperazino, N-lower alkanesulfonyl-piperazino, morpholino, thiomorpholino or S,S-dioxothiomorpholino;
R5 is as defined in any one of claims 1 to 4;
and the other symbols R3, R4, X, B₁, B₂, Ro and Rm have the meanings given in claim 1,
or a (preferably pharmaceutically acceptable) salt thereof.

Highly preferred is a compound of the formula I according to claim 1 or any preceding paragraph in this section on preferred embodiments wherein
R1, R2 and Y are as defined in claim 1 or any one of the preceding paragraphs,
R5 is as defined in claim 1 or any one of the preceding paragraphs,
R3 is hydrogen or methyl,
R4 is hydrogen,
each X stands for hydrogen,
B₁ is N or CRo,
B₂ is CRm,
and each of Ro and Rm, independently of the other, is hydrogen, methyl, fluoro, chloro or methoxy, preferably chloro,
or a pharmaceutically acceptable salt thereof;
or a compound of the formula I according to claim 1 or any preceding paragraph in this section on preferred embodiments wherein
R1, R2 and Y are as defined in claim 1 any one of the preceding paragraphs,
R5 is as defined in claims 1 or any one of the preceding paragraphs;
R3 is hydrogen or methyl,
R4 is hydrogen,
both X together form oxo,
B₁ is N or CRo,
B₂ is CRm,
and each of Ro and Rm, independently of the other, is hydrogen, methyl, fluoro, chloro or methoxy, preferably chloro,
or a pharmaceutically acceptable salt thereof.

The invention also relate to a compound of the formula XII, or a (especially pharmaceutically acceptable) salt thereof, wherein the symbols are as defined for a compound of the formula I (especially as in one of the preferred embodiments thereof as given in any one of the preceding paragraphs of the present Section on "Preferred embodiments according to the inventtion").

The invention also relate to a compound of the formula XIII, or a (especially pharmaceutically acceptable) salt thereof, wherein Q is hydroxyl or esterified or etherified hydroxyl (especially hydroxyl, lower alkoxy or lower alkoxy-lower alkoxy) and the other symbols are as defined for a compound of the formula I (especially as in one of the preferred embodiments thereof as given in any one of the preceding paragraphs of the present Section on "Preferred embodiments according to the invention").

Most preferred is a compound of the formula I, XII or XIII or a (preferably pharmaceutically acceptable) salt thereof, as exemplified herein-below under 'Examples', or its USE as defined above.

### Pharmaceutical Compositions

The invention relates also to pharmaceutical compositions comprising a (preferably novel) compound of formula I, to their use in the therapeutic (in a broader aspect of the invention also prophylactic) treatment or a method of treatment of a disease or disorder that depends on inadequate protein (especially Tie-2) kinase activity, especially the preferred disorders or diseases mentioned above, to the compounds for said use and to pharmaceutical preparations and their manufacture, especially for said uses. More generally, pharmaceutical preparations are useful in case of compounds of the formula I.

The pharmacologically acceptable compounds of the present invention may be present in or employed, for example, for the preparation of pharmaceutical compositions that comprise an effective amount of a compound of the formula I, or a pharmaceutically acceptable salt thereof, as active ingredient together or in admixture with one or more inorganic or organic, solid or liquid, pharmaceutically acceptable carriers (carrier materials).

The invention relates also to a pharmaceutical composition that is suitable for administration to a warm-blooded animal, especially a human (or to cells or cell lines derived from a warm-blooded animal, especially a human, e.g. lymphocytes), for the treatment (this, in a broader aspect of the invention, also including prevention of (= prophylaxis against)) a disease that responds to inhibition of protein (especially Tie-2) kinase activity, comprising an amount of a compound of formula I or a pharmaceutically acceptable salt thereof, preferably which is effective for said inhibition, together with at least one pharmaceutically acceptable carrier.

The pharmaceutical compositions according to the invention are those for enteral, such as nasal, rectal or oral, or parenteral, such as intramuscular or intravenous, administration to warm-blooded animals (especially a human), that comprise an effective dose of the pharmacologically active ingredient, alone or together with a significant amount of a pharmaceutically acceptable carrier. The dose of the active ingredient depends on the species of warm-blooded animal, the body weight, the age and the individual condition, individual pharmacokinetic data, the disease to be treated and the mode of administration.

The invention relates also to method of treatment for a disease that responds to inhibition of a disease that depends on inadequate activity of a protein (especially Tie-2) kinase; which comprises administering a prophylactically or especially therapeutically effective amount of a compound of formula I, or a pharmaceutically acceptable salt thereof, , especially to a warm-blooded animal, for example a human, that, on account of one of the mentioned diseases, requires such treatment.

The dose of a compound of the formula I or a pharmaceutically acceptable salt thereof to be administered to warm-blooded animals, for example humans of approximately 70 kg body weight, preferably is from approximately 3 mg to approximately 10 g, more preferably from approximately 10 mg to approximately 1.5 g, most preferably from about 100 mg to about 1000 mg /person/day, divided preferably into 1-3 single doses which may, for example, be of the same size. Usually, children receive half of the adult dose.

The pharmaceutical compositions comprise from approximately 1% to approximately 95%, preferably from approximately 20% to approximately 90%, active ingredient. Pharmaceutical compositions according to the invention may be, for example, in unit dosage form, such as in the form of ampoules, vials, suppositories, dragées, tablets or capsules.

The pharmaceutical compositions of the present invention are prepared in a manner known per se, for example by means of conventional dissolving, lyophilizing, mixing, granulating or confectioning processes.

Solutions of the active ingredient, and also suspensions, and especially isotonic aqueous solutions or suspensions, are preferably used, it being possible, for example in the case of lyophilized compositions that comprise the active ingredient alone or together with a carrier, for example mannitol, for such solutions or suspensions to be produced prior to use. The pharmaceutical compositions may be sterilized and/or may comprise excipients, for example preservatives, stabilizers, wetting and/or emulsifying agents, solubilizers, salts for regulating the osmotic pressure and/or buffers, and are prepared in a manner known per se, for example by means of conventional dissolving or lyophilizing processes. The said solutions or suspensions may comprise viscosity-increasing substances, such as sodium carboxymethylcellulose, carboxymethylcellulose, dextran, polyvinylpyrrolidone or gelatin.

Suspensions in oil comprise as the oil component the vegetable, synthetic or semi-synthetic oils customary for injection purposes. There may be mentioned as such especially liquid fatty acid esters that contain as the acid component a long-chained fatty acid having from 8-22, especially from 12-22, carbon atoms, for example lauric acid, tridecylic acid, myristic acid, pentadecylic acid, palmitic acid, margaric acid, stearic acid, arachidic acid, behenic acid or corresponding unsaturated acids, for example oleic acid, elaidic acid, erucic acid, brasidic acid or linoleic acid, if desired with the addition of antioxidants, for example vitamin E, β-carotene or 3,5-di-*tert*-butyl-4-hydroxytoluene. The alcohol component of those fatty acid esters has a maximum of 6 carbon atoms and is a mono- or poly-hydroxy, for example a mono-, di- or tri-hydroxy, alcohol, for example methanol, ethanol, propanol, butanol or pentanol or the isomers thereof, but especially glycol and glycerol. The following examples of fatty acid esters are therefore to be mentioned: ethyl oleate, isopropyl myristate, isopropyl palmitate, "Labrafil M 2375" (polyoxyethylene glycerol trioleate, Gattefossé, Paris), "Miglyol 812" (triglyceride of saturated fatty acids with a chain length of C8 to C12, Hüls AG, Germany), but especially vegetable oils, such as cottonseed oil, almond oil, olive oil, castor oil, sesame oil, soybean oil and groundnut oil.

The injection or infusion compositions are prepared in customary manner under sterile conditions; the same applies also to introducing the compositions into ampoules or vials and sealing the containers.

Pharmaceutical compositions for oral administration can be obtained by combining the active ingredient with solid carriers, if desired granulating a resulting mixture, and processing the mixture, if desired or necessary, after the addition of appropriate excipients, into tablets, dragée cores or capsules. It is also possible for them to be incorporated into plastics carriers that allow the active ingredients to diffuse or be released in measured amounts.

Suitable carriers are especially fillers, such as sugars, for example lactose, saccharose, mannitol or sorbitol, cellulose preparations and/or calcium phosphates, for example tricalcium phosphate or calcium hydrogen phosphate, and binders, such as starch pastes using for example corn, wheat, rice or potato starch, gelatin, tragacanth, methylcellulose, hydroxypropylmethylcellulose, sodium carboxymethylcellulose and/or polyvinylpyrrolidone, and/or, if desired, disintegrators, such as the above-mentioned starches, and/or carboxymethyl starch, crosslinked polyvinylpyrrolidone, agar, alginic acid or a salt thereof, such as sodium alginate. Excipients are especially flow conditioners and lubricants, for example silicic acid, talc, stearic acid or salts thereof, such as magnesium or calcium stearate, and/or polyethylene glycol. Dragée cores are provided with suitable, optionally enteric, coatings, there being used, inter alia, concentrated sugar solutions which may comprise gum arabic, talc, polyvinylpyrrolidone, polyethylene glycol and/or titanium dioxide, or coating solutions in suitable organic solvents, or, for the preparation of enteric coatings, solutions of suitable cellulose preparations, such as ethylcellulose phthalate or hydroxypropylmethylcellulose phthalate. Capsules are dry-filled capsules made of gelatin and soft sealed capsules made of gelatin and a plasticizer, such as glycerol or sorbitol. The dry-filled capsules may comprise the active ingredient in the form of granules, for example with fillers, such as lactose, binders, such as starches, and/or glidants, such as talc or magnesium stearate, and if desired with stabilizers. In soft capsules the active ingredient is preferably dissolved or suspended in suitable oily excipients, such as fatty oils, paraffin oil or liquid polyethylene glycols, it being possible also for stabilizers and/or antibacterial agents to be added. Dyes or pigments may be added to the tablets or dragée coatings or the capsule casings, for example for identification purposes or to indicate different doses of active ingredient.

A compound of the formula I may also be used to advantage in combination with other antiproliferative agents. Such antiproliferative agents include, but are not limited to aromatase inhibitors; antiestrogens; topoisomerase I inhibitors; topoisomerase II inhibitors; microtubule active agents; alkylating agents; histone deacetylase inhibitors; compounds which induce cell differentiation processes; cyclooxygenase inhibitors; MMP inhibitors; mTOR inhibitors; antineoplastic antimetabolites; platin compounds; compounds targeting/decreasing a protein or lipid kinase activity and further anti-angiogenic compounds; compounds which target, decrease or inhibit the activity of a protein or lipid phosphatase; gonadorelin agonists; anti-androgens; methionine aminopeptidase inhibitors; bisphosphonates; biological response modifiers; antiproliferative antibodies; heparanase inhibitors; inhibitors of Ras oncogenic isoforms; telomerase inhibitors; proteasome inhibitors; agents used in the treatment of hematologic malignancies; compounds which target, decrease or inhibit the activity of FIt-3; Hsp90 inhibitors; and temozolomide (TEMODAL®).

The term "aromatase inhibitor" as used herein relates to a compound which inhibits the estrogen production, i.e. the conversion of the substrates androstenedione and testosterone to estrone and estradiol, respectively. The term includes, but is not limited to steroids, especially atamestane, exemestane and formestane and, in particular, non-steroids, especially aminoglutethimide, roglethimide, pyridoglutethimide, trilostane, testolactone, ketokonazole, vorozole, fadrozole, anastrozole and letrozole. Exemestane can be administered, e.g., in the form as it is marketed, e.g. under the trademark AROMASIN. Formestane can be administered, e.g., in the form as it is marketed, e.g. under the trademark LENTARON. Fadrozole can be administered, e.g., in the form as it is marketed, e.g. under the trademark AFEMA. Anastrozole can be administered, e.g., in the form as it is marketed, e.g. under the trademark ARIMIDEX. Letrozole can be administered, e.g., in the form as it is marketed, e.g. under the trademark FEMARA or FEMAR. Aminoglutethimide can be administered, e.g., in the form as it is marketed, e.g. under the trademark ORIMETEN. A combination of the invention comprising a chemotherapeutic agent which is an aromatase inhibitor is particularly useful for the treatment of hormone receptor positive tumors, e.g. breast tumors.

The term "antiestrogen" as used herein relates to a compound which antagonizes the effect of estrogens at the estrogen receptor level. The term includes, but is not limited to tamoxifen, fulvestrant, raloxifene and raloxifene hydrochloride. Tamoxifen can be administered, e.g., in the form as it is marketed, e.g. under the trademark NOLVADEX. Raloxifene hydrochloride can be administered, e.g., in the form as it is marketed, e.g. under the trademark EVISTA.

Fulvestrant can be formulated as disclosed in US 4,659,516 or it can be administered, e.g., in the form as it is marketed, e.g. under the trademark FASLODEX. A combination of the invention comprising a chemotherapeutic agent which is an antiestrogen is particularly useful for the treatment of estrogen receptor positive tumors, e.g. breast tumors.

The term "anti-androgen" as used herein relates to any substance which is capable of inhibiting the biological effects of androgenic hormones and includes, but is not limited to, bicalutamide (CASODEX), which can be formulated, e.g. as disclosed in US 4,636,505.

The term "gonadorelin agonist" as used herein includes, but is not limited to abarelix, goserelin and goserelin acetate. Goserelin is disclosed in US 4,100,274 and can be administered, e.g., in the form as it is marketed, e.g. under the trademark ZOLADEX. Abarelix can be formulated, e.g. as disclosed in US 5,843,901.

The term "topoisomerase I inhibitor as used herein includes, but is not limited to topotecan, gimatecan, irinotecan, camptothecian and its analogues, 9-nitrocamptothecin and the macromolecular camptothecin conjugate PNU-166148 (compound A1 in W099/ 17804). Irinotecan can be administered, e.g. in the form as it is marketed, e.g. under the trademark CAMPTO-SAR. Topotecan can be administered, e.g., in the form as it is marketed, e.g. under the trademark HYCAMTIN.

The term "topoisomerase II inhibitor" as used herein includes, but is not limited to the anthracyclines such as doxorubicin (including liposomal formulation, e.g. CAELYX), daunorubicin, epirubicin, idarubicin and nemorubicin, the anthraquinones mitoxantrone and Io-soxantrone, and the podophillotoxines etoposide and teniposide. Etoposide can be administered, e.g. in the form as it is marketed, e.g. under the trademark ETOPOPHOS. Teniposide can be administered, e.g. in the form as it is marketed, e.g. under the trademark VM 26-BRISTOL. Doxorubicin can be administered, e.g. in the form as it is marketed, e.g. under the trademark ADRIBLASTIN or ADRIAMYCIN. Epirubicin can be administered, e.g. in the form as it is marketed, e.g. under the trademark FARMORUBICIN. Idarubicin can be administered, e.g. in the form as it is marketed, e.g. under the trademark ZAVEDOS. Mitoxantrone can be administered, e.g. in the form as it is marketed, e.g. under the trademark NOVANTRON.

The term "microtubule active agent" relates to microtubule stabilizing, microtubule destabilizing agents and microtublin polymerization inhibitors including, but not limited to taxanes, e.g. paclitaxel and docetaxel, vinca alkaloids, e.g., vinblastine, especially vinblastine sulfate, vincristine especially vincristine sulfate, and vinorelbine, discodermolides, cochicine and epothilones and derivatives thereof, e.g. epothilone B or a derivative thereof. Paclitaxel may be administered e.g. in the form as it is marketed, e.g. TAXOL. Docetaxel can be administered, e.g., in the form as it is marketed, e.g. under the trademark TAXOTERE. Vinblastine sulfate can be administered, e.g., in the form as it is marketed, e.g. under the trademark VINBLASTIN R.P.. Vincristine sulfate can be administered, e.g., in the form as it is marketed, e.g. under the trademark FARMISTIN. Discodermolide can be obtained, e.g., as disclosed in US 5,010,099. Also included are Epothilone derivatives which are disclosed in WO 98/10121, US 6,194,181, WO 98/25929, WO 98/08849, WO 99/43653, WO 98/22461 and WO 00/31247. Especially preferred are Epothilone A and/or B.

The term "alkylating agent" as used herein includes, but is not limited to, cyclophosphamide, ifosfamide, melphalan or nitrosourea (BCNU or Gliadel). Cyclophosphamide can be administered, e.g., in the form as it is marketed, e.g. under the trademark CYCLOSTIN. Ifosfamide can be administered, e.g., in the form as it is marketed, e.g. under the trademark HOLOXAN.

The term "histone deacetylase inhibitors" or "HDAC inhibitors" relates to compounds which inhibit the histone deacetylase and which possess antiproliferative activity. This includes compounds disclosed in WO 02/22577, especially N-hydroxy-3-[4[[(2-hydroxyethyl)[2-(1H-indol-3-yl)ethyl]-amino]methyl]phenyl]-2E-2-propenamide, N-hydroxy-3-[4-[[2-(2-methyl-1*H-*indol-3-yl)-ethyl]-amino]methyl]phenyl]-2*E*-2-propenamide and pharmaceutically acceptable salts thereof. It further especially includes Suberoylanilide hydroxamic acid (SAHA).

The term "antineoplastic antimetabolite" includes, but is not limited to, 5-Fluorouracil or 5-FU, capecitabine, gemcitabine, DNA demethylating agents, such as 5-azacytidine and decitabine, methotrexate and edatrexate. Capecitabine can be administered, e.g., in the form as it is marketed, e.g. under the trademark XELODA. Gemcitabine can be administered, e.g., in the form as it is marketed, e.g. under the trademark GEMZAR. Also included is the monoclonal antibody trastuzumab which can be administered, e.g., in the form as it is marketed, e.g. under the trademark HERCEPTIN.

The term "platin compound" as used herein includes, but is not limited to, carboplatin, cis-platin, cisplatinum and oxaliplatin. Carboplatin can be administered, e.g., in the form as it is marketed, e.g. under the trademark CARBOPLAT. Oxaliplatin can be administered, e.g., in the form as it is marketed, e.g. under the trademark ELOXATIN.

The term "compounds targeting/decreasing a protein or lipid kinase activity and further anti-angiogenic compounds" as used herein includes, but is not limited to: protein tyrosine kinase and/or serine and/or threonine kinase inhibitors or lipid kinase inhibitors, e.g.:
a) compounds targeting, decreasing or inhibiting the activity of the platelet-derived growth factor-receptors (PDGFR), such as compounds which target, decrease or inhibit the activity of PDGFR, especially compounds which inhibit the PDGF receptor, e.g. a N-phenyl-2-pyrimidine-amine derivative, e.g. imatinib, SU101, SU6668, and GFB-111;
b) compounds targeting, decreasing or inhibiting the activity of the fibroblast growth factor-receptors (FGFR);
c) compounds targeting, decreasing or inhibiting the activity of the insulin-like growth factor receptor 1 (IGF-1R), such as compounds which target, decrease or inhibit the activity of IGF-IR, especially compounds which inhibit the IGF-1R receptor, such as those compounds disclosed in WO 02/092599;
d) compounds targeting, decreasing or inhibiting the activity of the Turk receptor tyrosine kinase family;
e) compounds targeting, decreasing or inhibiting the activity of the Ax1 receptor tyrosine kinase family;
f) compounds targeting, decreasing or inhibiting the activity of the c-Met receptor;
g) compounds targeting, decreasing or inhibiting the activity of the c-Kit receptor tyrosine kinases - (part of the PDGFR family), such as compounds which target, decrease or inhibit the activity of the c-Kit receptor tyrosine kinase family, especially compounds which inhibit the c-Kit receptor, e.g. imatinib;
h) compounds targeting, decreasing or inhibiting the activity of members of the c-Abl family and their gene-fusion products (e.g. BCR-Abl kinase), such as compounds which target, decrease or inhibit the activity of c-Abl family members and their gene fusion products, e.g. a N-phenyl-2-pyrimidine-amine derivative, e.g. imatinib; PD180970; AG957; NSC 680410; or PD173955 from ParkeDavis;
i) compounds targeting, decreasing or inhibiting the activity of members of the protein kinase C (PKC) and Raf family of serine/threonine kinases, members of the MEK, SRC, JAK, FAK, PDK and Ras/MAPK family members, or PI(3) kinase family, or of the PI(3)-kinase-related kinase family, and/or members of the cyclin-dependent kinase family (CDK) and are especially those staurosporine derivatives disclosed in US 5,093,330, e.g. midostaurin; examples of further compounds include e.g. UCN-01, safingol, BAY 43-9006, Bryostatin 1, Perifosine; Ilmofosine; RO 318220 and RO 320432; GO 6976; Isis 3521; LY333531/LY379196; isochinoline compounds such as those disclosed in WO 00/09495; FTIs; PD184352 or QAN697 (a P13K inhibitor);
j) compounds targeting, decreasing or inhibiting the activity of a protein-tyrosine kinase, such as imatinib mesylate (GLIVEC/GLEEVEC) or tyrphostin. A tyrphostin is preferably a low molecular weight (Mr < 1500) compound, or a pharmaceutically acceptable salt thereof, especially a compound selected from the benzylidenemalonitrile class or the S-arylbenzenemalonirile or bisubstrate quinoline class of compounds, more especially any compound selected from the group consisting of Tyrphostin A23/RG-50810; AG 99; Tyrphostin AG 213; Tyrphostin AG 1748; Tyrphostin AG 490; Tyrphostin B44; Tyrphostin B44 (+) enantiomer Tyrphostin AG 555; AG 494; Tyrphostin AG 556, AG957 and adaphostin (4-{[(2,5-dihydroxyphenyl)methyl]amino}benzoic acid adamantyl ester, NSC 680410, adaphostin);
k) compounds targeting, decreasing or inhibiting the activity of the epidermal growth factor family of receptor tyrosine kinases (EGFR, ErbB2, ErbB3, ErbB4 as homo- or heterodimers), such as compounds which target, decrease or inhibit the activity of the epidermal growth factor receptor family are especially compounds, proteins or antibodies which inhibit members of the EGF receptor tyrosine kinase family, e.g. EGF receptor, ErbB2, ErbB3 and ErbB4 or bind to EGF or EGF related ligands, and are in particular those compounds, proteins or monoclonal antibodies generically and specifically disclosed in WO 97/02266, e.g. the compound of ex. 39, or in EP 0 564 409, WO 99/03854, EP 0520722, EP 0 566 226, EP 0 787 722, EP 0 837 063, US 5,747,498. WO 98/10767, WO 97/30034, WO 97/49688. WO 97/38983 and, especially, WO 96/30347 (e.g. compound known as CP 358774), WO 96/33980 (e.g. compound ZD 1839) and WO 95/03283 (e.g. compound ZM105180); e.g. trastuzumab (HerpetinR), cetuximab, Iressa, OSI-774, CI-1033, EKB-569, GW-2016, E1.1, E2.4, E2.5, E6.2, E6.4, E2.11, E6.3 or E7.6.3, and 7H-pyrrolo-[2,3-d]pyrimidine derivatives which are disclosed in WO 031013541; and
l) compounds targeting, decreasing or inhibiting the activity of the vascular endothelial growth factor-receptors (VEGFR), such as PTK-787 or Avastin,

Further anti-angiogenic compounds include compounds having another mechanism for their activity, e.g. unrelated to protein or lipid kinase inhibition e.g. thalidomide (THALOMID) and TNP-470 or RAD001.

Compounds which target, decrease or inhibit the activity of a protein or lipid phosphatase are e.g. inhibitors of phosphatase 1, phosphatase 2A, PTEN or CDC25, e.g. okadaic acid or a derivative thereof.

Compounds which induce cell differentiation processes are e.g. retinoic acid, α- γ- or δ-tocopherol or α- γ- or δ-tocotrienol.

The term "cyclooxygenase inhibitor" as used herein includes, but is not limited to, e.g. Cox-2 inhibitors, 5-alkyl substituted 2-arylaminophenylacetic acid and derivatives, such as celecoxib (CELEBREX), rofecoxib (VIOXX), etoricoxib, valdecoxib or a 5-alkyl-2-arylaminophenylacetic acid, e.g. 5-methyl-2-(2'-chloro-6'-fluoroanilino)phenyl acetic acid, lumiracoxib.

The term "mTOR inhibitors" relates to compounds which inhibit the mammalian target of rapamycin (mTOR) and which possess antiproliferative activity such as sirolimus (Rapamune®), everolimus (Certican™), CCI-779 and ABT578.

The term "bisphosphonates" as used herein includes, but is not limited to, etridonic, clodronic, tiludronic, pamidronic, alendronic, ibandronic, risedronic and zoledronic acid. "Etridonic acid" can be administered, e.g., in the form as it is marketed, e.g. under the trademark DIDRONEL. "Clodronic acid" can be administered, e.g., in the form as it is marketed, e.g. under the trademark BONEFOS. "Tiludronic acid" can be administered, e.g., in the form as it is marketed, e.g. under the trademark SKELID. "Pamidronic acid" can be administered, e.g. in the form as it is marketed, e.g. under the trademark AREDIA™. "Alendronic acid" can be administered, e.g., in the form as it is marketed, e.g. under the trademark FOSAMAX. "Ibandronic acid" can be administered, e.g., in the form as it is marketed, e.g. under the trademark BONDRANAT. "Risedronic acid" can be administered, e.g., in the form as it is marketed, e.g. under the trademark ACTONEL. "Zoledronic acid" can be administered, e.g. in the form as it is marketed, e.g. under the trademark ZOMETA.

The term "heparanase inhibitor" as used herein refers to compounds which target, decrease or inhibit heparin sulphate degradation. The term includes, but is not limited to, PI-88.

The term "biological response modifier" as used herein refers to a lymphokine or interferons, e.g. interferon γ.

The term "inhibitor of Ras oncogenic isoforms", e.g. H-Ras, K-Ras, or N-Ras, as used herein refers to compounds which target, decrease or inhibit the oncogenic activity of Ras e.g. a "farnesyl transferase inhibitor, e.g. L-744832, DK8G557 or R115777 (Zamestra).

The term "telomerase inhibitor" as used herein refers to compounds which target, decrease or inhibit the activity of telomerase. Compounds which target, decrease or inhibit the activity of telomerase are especially compounds which inhibit the telomerase receptor, e.g. telomestatin.

The term "methionine aminopeptidase inhibitor as used herein refers to compounds which target, decrease or inhibit the activity of methionine aminopeptidase. Compounds which target, decrease or inhibit the activity of methionine aminopeptidase are e.g. bengamide or a derivative thereof.

The term "proteasome inhibitor" as used herein refers to compounds which target, decrease or inhibit the activity of the proteasome. Compounds which target, decrease or inhibit the activity of the proteasome include e.g. PS-341 and MLN 341.

The term "matrix metalloproteinase inhibitor" or ("MMP inhibitor) as used herein includes, but is not limited to collagen peptidomimetic and nonpeptidomimetic inhibitors, tetracycline derivatives, e.g. hydroxamate peptidomimetic inhibitor batimastat and its orally bioavailable analogue marimastat (BB-2516), prinomastat (AG3340), metastat (NSC 683551) BMS-279251, BAY 12-9566, TAA211, MM1270B or AAJ996.

The term "agents used in the treatment of hematologic malignancies" as used herein includes, but is not limited to FMS-like tyrosine kinase inhibitors e.g. compounds targeting, decreasing or inhibiting the activity of Flt-3; interferon, 1-b-D-arabinofuransylcytosine (ara-c) and bisulfan; and ALK inhibitors e.g. compounds which target, decrease or inhibit anaplastic lymphoma kinase.

The term "compounds which target, decrease or inhibit the activity of Flt-3" are especially compounds, proteins or antibodies which inhibit Flt-3, e.g. PKC412, midostaurin, a staurosporine derivative, SU11248 and MLN518.

The term "HSP90 inhibitors" as used herein includes, but is not limited to, compounds targeting, decreasing or inhibiting the intrinsic ATPase activity of HSP90; degrading, targeting, decreasing or inhibiting the HSP90 client proteins via the ubiquitin proteasome pathway.

Compounds targeting, decreasing or inhibiting the intrinsic ATPase activity of HSP90 are especially compounds, proteins or antibodies which inhibit the ATPase activity of HSP90 e.g., 17-allylamino,17-demethoxygeldanamycin (17AAG), a geldanamycin derivative; other geldanamycin related compounds; radicicol and HDAC inhibitors.

The term "antiproliferative antibodies" as used herein includes, but is not limited to trastuzumab (Herceptin™), Trastuzumab-DM1, erlotinib (Tarceva™), bevacizumab (Avastin™), rituximab (Rituxan®), PRO64553 (anti-CD40) and 2C4 Antibody. By antibodies is meant e.g. intact monoclonal antibodies, polyclonal antibodies, multispecific antibodies formed from at least 2 intact antibodies, and antibodies fragments so long as they exhibit the desired biological activity.

For the treatment of acute myeloid leukemia (AML), compounds of formula 1 can be used in combination with standard leukemia therapies, especially in combination with therapies used for the treatment of AML. In particular, compounds of formula I can be administered in combination with e.g. famesyl transferase inhibitors and/or other drugs useful for the treatment of AML, such as Daunorubicin, Adriamycin, Ara-C, VP-16, Teniposide, Mitoxantrone, Idarubicin, Carboplatinum and PKC412.

The structure of the active agents identified by code nos., generic or trade names may be taken from the actual edition of the standard compendium "The Merck Index" or from databases, e.g. Patents International (e.g. IMS World Publications).

The above-mentioned compounds, which can be used in combination with a compound of the formula I, can be prepared and administered as described in the art such as in the documents cited above.

A compound of the formula I may also be used to advantage in combination with known therapeutic processes, e.g., the administration of hormones or especially radiation.

A compound of formula I may in particular be used as a radiosensitizer, especially for the treatment of tumors which exhibit poor sensitivity to radiotherapy.

By "combination", there is meant either a fixed combination in one dosage unit form, or a kit of parts for the combined administration where a compound of the formula I and a combination partner may be administered independently at the same time or separately within time intervals that especially allow that the combination partners show a cooperative, e.g. synergistic, effect, or by making use of administration schedules representing any combination thereof.

### EXAMPLES:

The following examples serve to illustrate the invention without limiting the scope thereof:

### Abbreviations

- Ac: acetyl
- aq.: aqueous
- Boc: tert-butoxycarbonyl
- Brine: saturated sodium chloride solution
- Celite: trademark of Celite Corp. for filtering aid based on kieselguhr
- conc.: concentrated
- DCM: dichloromethane
- DEAD: diethyl azodicarboxylate
- DMF: N,N-dimethylformamide
- DMSO: dimethylsulfoxide
- DMT-MM: 4-(4,6-dimethoxy-1,3,5-triazin-2-yl)-4-methylmorpholinium chloride
- EDC: 1-(3-Dimethylaminopropyl)-3-ethylcarbodiimide hydrochloride
- ES-MS: electrospray mass spectrometry
- Et: ethyl
- EtOAc: ethyl acetate
- h: hour(s)
- HOAt: 1-hydroxy-7-azabenzotriazole
- HPLC: high-pressure liquid chromatography
- HyFlo: diatomaceous earth based filtering aid
- IPr: isopropyl
- LAH: lithium aluminium hydride
- Me: methyl
- min: minute(s)
- mL: milliliter(s)
- MS: Mass Spectrometry
- NaOMe: sodium methoxylate
- NMR: nuclear magnetic resonance
- Ph: phenyl
- RT: room temperature
- TBTU: O-(benzotriazol-1-yl)-N,N,N',N'-tetramethylammonium tetrafluoroborate
- TFA: trifluoroacetic acid
- THF: tetrahydrofurane
- TMS: trimethylsilyl
- WSCD: = EDC
- Prop-sol: Propylphosphonic anhydride solution in DMF

### Synthesis

Flash chromatography is performed by using silica gel (Merck; 40 - 63 µm). For thin layer chromatography, pre-coated silica gel (Merck 60 F254; Merck KgaA, Darmstadt, Germany)) plates are used. ¹NMR measurements are performed on a Varian Gemini 400 or Varian Gemini 300 spectrometer using tetramethylsilane as internal standard. Chemical shifts (δ) are expressed in ppm downfield from tetramethylsilane. Electrospray mass spectra are obtained with a Fisons Instruments VG Platform II. Commercially available solvents and chemicals are used for syntheses.

### HPLC condition A

Column: Nucleosil 100-3 C18, 70 x 4.0 mm.
Flow rate: 1.0 ml/min
Mobile phase: A) TFA/water (0.1/100, v/v), B) TFA/acetonitrile (0.1/1 00,v/v)
Gradient: linear gradient from 20% B to 100% B in 7min
Detection: UV at 215nm

### HPLC condition B

Column: Speed ROD RP18e, 50 x 4.6 mm.
Flow rate: 2.0 ml/min
Mobile phase: A) TFA/water (0.1/100, v/v), B) TFA/acetonitrile (0.1/100,v/v)
Gradient: linear gradient from 0% B to 100% B in 2 min then 100% B 2 min
Detection: UV at 215nm

### HPLC condition C

Column: YMC-pack ODS-AQ, 50 x 4.6 mm.
Flow rate: 2.5 ml/min
Mobile phase: A) TFA/water (0.1/100, v/v). B) TFA/acetonitrile (0.1/100,v/v)
Gradient: linear gradient from 10% B to 80% B in 6 min then 80% B 2 min
Detection: UV at 215nm

### HPLC condition D

Column: YMC-pack ODS-AQ, 50 x 4.6 mm.
Flow rate: 3.0 ml/min
Mobile phase: A) TFA/water (0.1/100, v/v), B) TFA/acetonitrile (0.1/100,v/v)
Gradient: linear gradient from 10% B to 80% B in 5 min then 80% B 1.5 min
Detection: UV at 215nm

The HPLC conditions A, B, C and D can be identified by the subscript prefixes of the T_{Ret} values given in the examples. For instance, B in _{B}t*_{Ret}* = .... Min means condition-B in the case of HPLC.

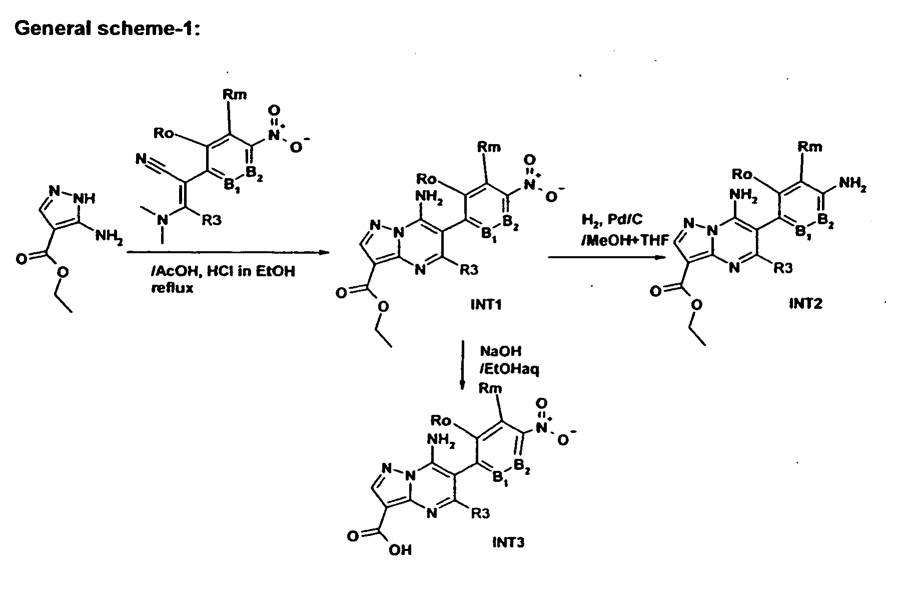

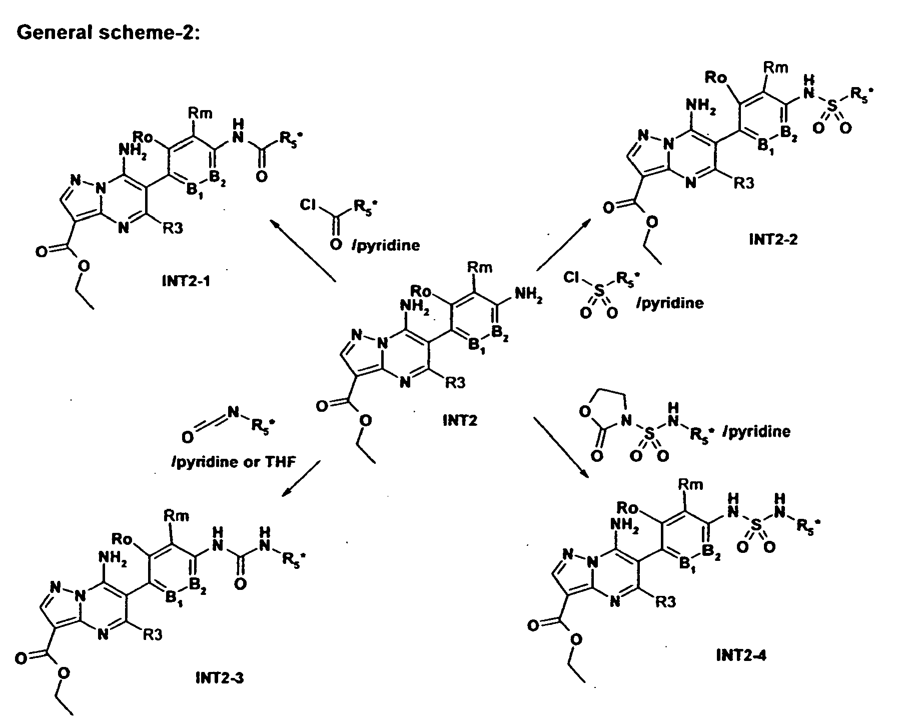

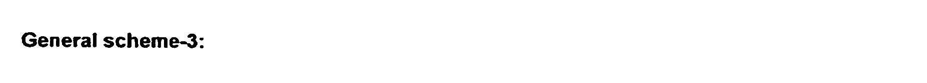

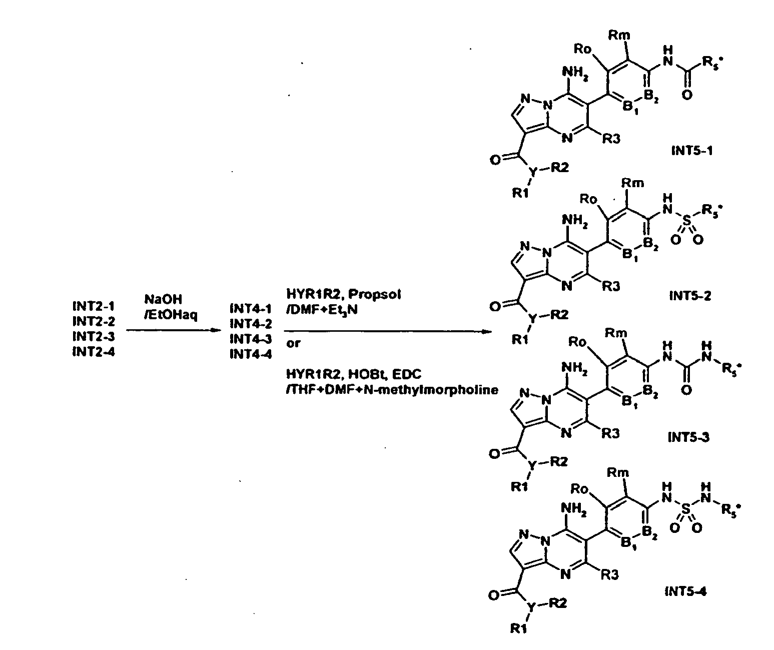

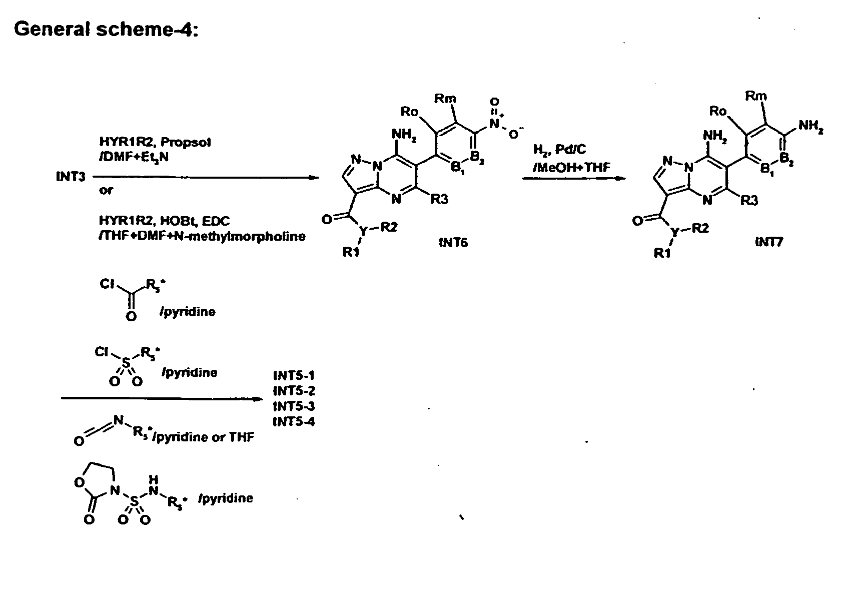

Intermediates INT6, INT7 can be obtained as a racemic mixture when racemic or chiral amine will be used, or optical resolution of INT6 and INT7 using an appropriate chiral acid (such as tartaric acid etc) may afford corresponding enantiomeric pure INT6 and INT7. And the final product INT5-1, INT5-2, INT5-3 and INT5-4 can be separated into the pure enantiomers by common techniques like chiral chromatography.

R1, R2, Ro, Rm, B₁, B₂ and Y (which is more preferably N) are preferably as described under formula I and more preferably as described in the examples, R₅* is a moiety complementing the formula given in the reaction schemes to give a corresponding moiety R₅ in a compound of the formula I, especially as described in the examples.

### Example 1 N-{4-[7-Amino-3-(3-dimethylamino,pyrrolidine-1-carbonyl)-pyrazolo[1,5-a]pyrimidin-6-yl]-phenyl}-2,3-dichloro-benzenesulfonamide

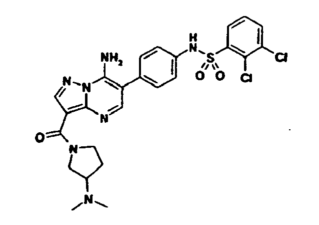

A mixture of 7-amino-6-[4-(2,3-dichtoro-benzenesulfonylamino)-phenyl]-pyrazolo[1,5-a]pyrimidine-3-carboxylic acid (100 mg, 0.21 mmol), 3-(dimethylamino)-pyrrolidine (48 mg, 0.42 mmol), EDC (61 mg, 0.32 mmol) and HOAt (44 mg, 0.32 mmol) in DMF (1.1 mL), Et₃N (0.29 mL, 2.1 mmol) is stirred for 20 h at room temperature and for 2 h at 40 °C. EtOAc is added and the organic layer is washed with brine, dried over MgSO₄ and evaporated in vacuo. Silica gel flash chromatography of the residue affords the title compound as colorless crystal; ES-MS: M+ = 576.8; HPLC: _{A}t_{Ret} = 2.94 min.

### Intermediate 1.1 7-Amino-6-[4-(2,3-dichloro-benzenesulfonylamino)-phenyl]-pyrazolo[1,5-a]pyrimidine-3-carboxylic acid

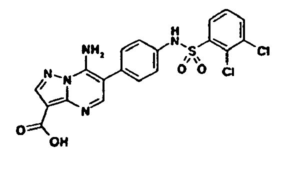

A mixture of 7-amino-6-[4-(2,3-dichloro-benzenesulfonylamino)-phenyl]-pyrazolo[1,5-a]pyrimidine-3-carboxylic acid ethyl ester (1.03 g, 2.03 mmol) and NaOH (2g) in EtOH (20 mL) and H₂O (5 mL) is refluxed for 2 hours. The resulting mixture is poured into a mixture of ice and water, and after neutralized by AcOH, the product is isolated by filtration and washed with water, and dried under reduced pressure to the title compound as colorless crystal; ES-MS: M = 478.0; HPLC: _{A}t*_{Ret}* = 3.26 min.

### Intermediate 1.2 7-Amino-6-[4-(2,3-tlichloro-benzenesulfonylamino)-phenyl]-pyrazolo[1,5-a]pyrimidine-3-carboxylic acid ethyl ester

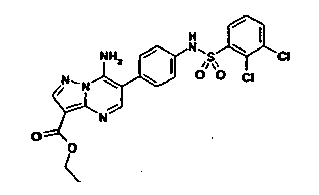

A mixture of 7-amino-6-(4-amino-phenyl)-pyrazolo[1,5-a]pyrimidine-3-carboxylic acid ethyl ester (1.46 g, 4.37 mmol) and 2,3-dichlorobenzenesulfonyl chloride (1.29 g, 5.24 mmol) in pyridine (20 mL) is stirred for 5 hours at room temperature. The resulting mixture is poured into a mixture of ice and water, and the product is isolated by filtration and washed with water, and dried under reduced pressure to give the title compound as yellow powder; ES-MS: M+H = 507.8; HPLC: _{A}t*_{Ret}* = 3.79 min.

### Intermediate 1.3 7-Amino-6-(4-amino-phenyl)-pyrazolo[1,5-a]pyrimidine-3-carboxylic acid ethyl ester

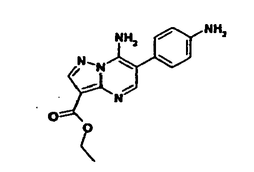

A mixture of 7-amino-6-(4-nitro-phenyl)-pyrazolo[1,5-a]pyrimidine-3-carboxylic acid ethyl ester (2.0 g, 5.5 mmol) and 5% Pd/C (0.1 g) in EtOH (200 mL) and THF (100 mL) is shaken under a H₂-atmosphere (1 bar). After 24 h, the reaction mixture is filtered through Celite and carefully washed with THF. Concentration *in vacuo* affords the title compound as colorless crystal; ES-MS: M+H = 298. 1; HPLC: _{A}t*_{Ret}* = 1.28 min.

### Intermediate 1.4 7-Amino-6-(4-nitro-phenyl)-pyrazZol[1,5-a]pyrimidine-3-carboxylic acid ethyl ester

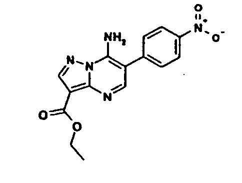

A mixture of 3-dimethylamino-2-(4-nitro-phenyl)-acrylonitrile (3.0 g, 13.8 mmol) (see e.g. Bulletin des Societes Chimiques Belges 1994, 103(12), 697-703) and 5-amino-1H-pyrazole-4-carboxylic acid ethyl ester (2.14 g, 13.8 mmol) in AcOH (25 mL) and EtOH solution of 1.25M HCl (25 ml) is refluxed for 14 hours. The resulting mixture is concentrated *in vacuo,* and the product is isolated by filtration and washed with CH₃CN, and dried under reduced pressure to afford the title compound as colorless crystal; ES-MS: M+H = 328.0; HPLC: _{A}t*_{Ret}* = 2.98 min.

### Example 2 1-{4-[7-Amino-3-(3-dimethylamino-pyrrolidine-1-carbonyl)-pyrazolo[1,5-a]pyrimidin-6-yl]-3-methyl-phenyl}-3-(2-chloro-phenyl)-urea

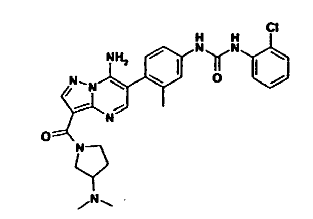

A mixture of [7-amino-6-(4-amino-2-methyl-phenyl)-pyrazolo[1,5-a]pyrimidin-3-yl]-(3-dimethylamino-pyrrolidin-1-yl)-methanone (80 mg, 0.21 mmol) and 2-chlorophenylisocyanate (42 mg, 0.27 mmol) in pyridine (1.1 mL) is stirred for 3 h at room temperature. EtOAc is added and the organic layer is washed with brine, dried over MgSO₄ and evaporated *in vacuo*. Silica gel flash chromatography of the residue affords the title compound as colorless crystal; ES-MS: M+ = 533.0; HPLC: _{A}t*_{Ret}* = 3.06 min.

### Intermediate 2.1 [7-Amino-6-(4-amino-2-methyl-phenyl)-pyrazolo[1,5-a]pyrimidin-3-yl]-(3-dimethylamino-pyrrolidin-1-yl)-methanone

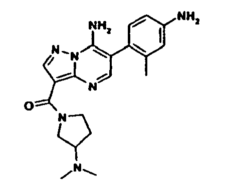

A mixture of [7-amino-6-(2-methyl-4-nitro-phenyl)-pyrazolo[1,5-a]pyrimidin-3-yl]-(3-dimethylamino-pyrrolidin-1-yl)-methanone (3.5 g, 8.55 mmol) and 10% Pd/C (350 mg) in MeOH (200 mL) is shaken under a H₂-atmosphere (1 bar). After 3 h, the reaction mixture is filtered through Celite and carefully washed with MeOH and THF. Concentration *in vacuo* affords the title compound as colorless powder; ES-MS: M+H = 380.1; HPLC: _{A}t*_{Ret}* = 0.90 min.

### Intermediate 2.2 [7-Amino-6-(2-methyl-4-nitro-phenyl)-pyrazolo[1,5-a]pyrimidin-3-yl]-(3-dimethylamino-pyrrolidin-1-yl)-methanone

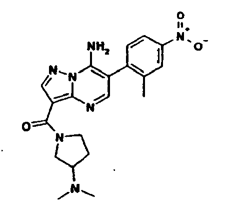

A mixture of 7-amino-6-(2-methyl-4-nitro-phenyl)-pyrazolo[1,5-a]pyrimidine-3-carboxylic acid (200 mg, 0.63 mmol), 3-(dimethytamino)-pyrrolidine (110 mg, 0.96 mmol), a catalytic amount of DMAP and Prop-sol (0.74 mL, 2.52 mmol) in DMF (4 mL), Et₃N (4 mL) is stirred for 17 h at room temperature. EtOAc is added and the organic layer is washed with brine, dried over MgSO₄ and evaporated *in vacuo.* Silica gel flash chromatography of the residue affords the title compound as colorless powder; ES-MS: M+H = 410.1; HPLC: _{A}t*_{Ret}* = 1.67 min.

### Intermediate 2.3 7-Amino-6-(2-methyl-4-nitro-phenyl)-pyrazolo[1,5-a]pyrimidine-3-carboxylic acid

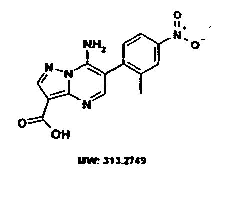

A mixture of Intermediate 2.4 (4.0 g, 11.7 mmol) in EtOH (36 mL) and aqueous 4M NaOH (15 mL) is refluxed for 6.5 hours. The resulting mixture is poured into a mixture of ice and water, and after neutralized by AcOH, the product is isolated by filtration and washed with water, and dried under reduced pressure to Intermediate 2.3 as white crystal; ES-MS: M = 313.1; HPLC: _{A}t*_{Ret}* = 2.23 min.

### Intermediate 2.4

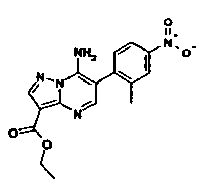

A mixture of 3-dimethylamino-2-(3-methyl-4-nitro-phenyl)-acrylonitrile (1.2 g, 5.19 mmol) (see e.g. Bulletin des Societes Chimiques Belges 1994, 103(12), 697-703) and 5-Amino-1H-pyrazole-4-carboxylic acid ethyl ester (810 mg, 5.19 mmol) in AcOH (15 mL) and EtOH solution of 1.25M HCl (15 ml) is refluxed for 5.5 h. The resulting mixture is concentrated *in vacuo,* and the product is isolated by filtration and washed with CH₃CN, and dried under reduced pressure to Intermediate 2.4 as colorless crystal; ES-MS: M+H = 232.1; HPLC: _{A}t*_{Ret}* = 3.12 min.

### Intermediate 2.5

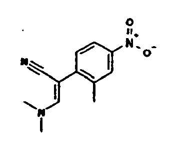

**Intermediate 2.6** (1.61 g, 9.13 mmol) is dissolved in toluene (20 mL) and N,N-dimethyl formamide dimethyl acetal (2.4 mL, 18.2 mmol) is added at rt. The reaction is then heated to 120 °C and stirred for 12 h. It is subsequently cooled again and all volatiles are removed under reduced pressure. The remaining crude product is dried under high vacuum to give the title compound as a yellow oil and directly used for the next step. ES-MS: M+H = 232.1.

### Intermediate 2.6

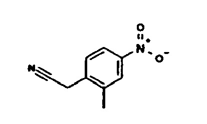

The title compound is prepared in the same method starting from 4-fluoro-3-methylnitrobenzene (see W02005/054238) and 2,3-dichlorobenzene sulfonylchloride. ES-MS: M-H = 174.9, HPLC: _{A}t*_{Ret}* = 3.90 min.

**Table 1: the following examples are prepared in analogy to examples 1 or 2 and/or as decribed herein, using the appropriately substituted starting materials:**

| | | | |
|---|---|---|---|
| 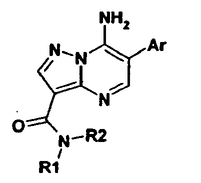 | | | |

| Example | 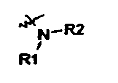 | 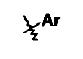 | Analytical data MS/HPLC/m.p |
|---|---|---|---|
| 3 | 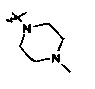 | 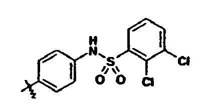 | M+H = 561.8 _{A}t*_{Ret}* = 3.01 min |
| 4 | 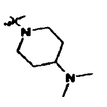 | 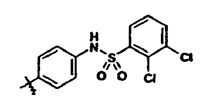 | M+H = 589.9 _{A}t*_{Ret}* = 3.00 min |
| 5 | 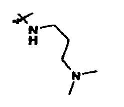 | 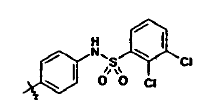 | M+H = 563.9 _{A}t*_{Ret}* = 3.26 min |
| 6 | 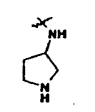 | 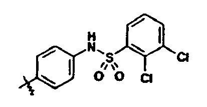 | M+H = 548.8 _{A}t*_{Ret}* = 3.15 min |
| 7 | 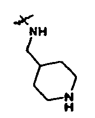 | 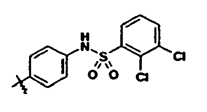 | M+H = 576.9 _{A}t*_{Ret}*= 3.20 min |
| 8 | 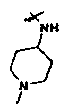 | 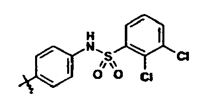 | M+H = 575.8 _{A}t*_{Ret}* = 3.20 min |
| 9 | 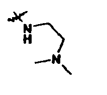 | 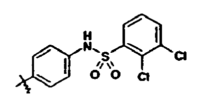 | M+H = 549.8 _{A}t*_{Ret}* = 3.24 min |
| 10 | 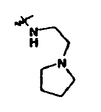 | 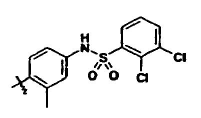 | M+H = 559.8 _{A}t*_{Ret}* = 3.41 min |
| 11 | 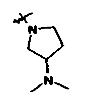 | 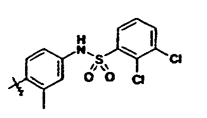 | M+H = 589.8 _{A}t*_{Ret}* = 3.05 min |
| 12 | 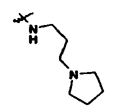 | 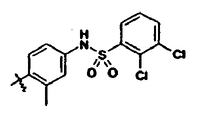 | M+H = 603.8 _{A}t*_{Ret}* = 3.43 min |
| 13 | 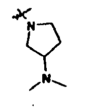 | 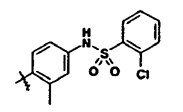 | M+H = 555.9 _{A}t*_{Ret}* = 2.72 min |
| 14 | 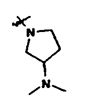 | 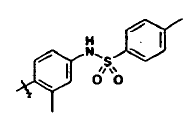 | M+H = 534.1 _{C}t*_{Ret}* = 4.45 min |
| 15 | 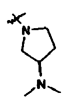 | 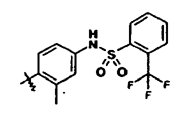 | M+H = 588.0 _{C}t*_{Ret}* = 4.58 min |
| 16 | 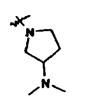 | 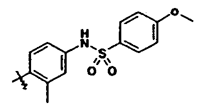 | M+H = 550:1 _{C}t*_{Ret}* = 4.34 min |
| 17 | 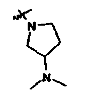 | 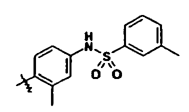 | M+H = 534.1 _{C}t*_{Ret}* = 4.03 min |
| 18 | 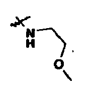 | 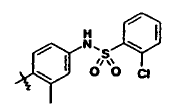 | M+ = 514.9 _{C}t*_{Ret}* = 4.13 min |
| 19 | 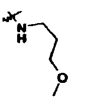 | 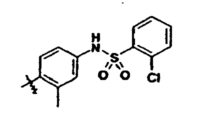 | M+ = 528.9 _{C}t*_{Ret}* = 3.85 min |
| 20 | 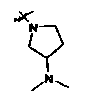 | 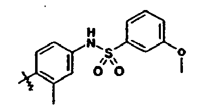 | M+H = 550.1 _{C}t*_{Ret}* = 4.71 min |
| 21 | 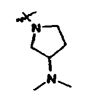 | 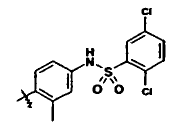 | M+ = 588.0 _{C}t*_{Ret}* = 4.80 min |
| 22 | 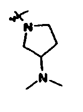 | 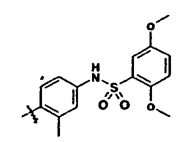 | M+H = 580.1 _{C}t*_{Ret}* = 2.78 min |
| 23 | 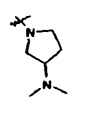 | 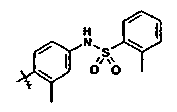 | M+H = 534.1 _{C}t*_{Ret}* = 4.41 min |
| 24 | 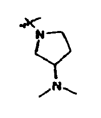 | 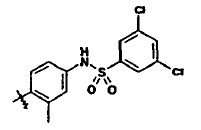 | M+ = 588.0 _{C}t*_{Ret} =* 4.04 min |
| 25 | 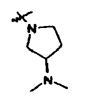 | 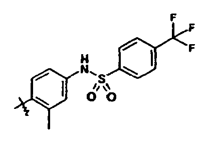 | M+H = 588.1 _{C}t*_{Ret}* = 3.99 min |
| 26 | 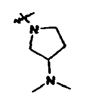 | 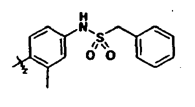 | M+H = 534.0 _{C}t*_{Ret}* = 4.22 min |
| 27 | 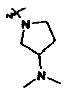 | 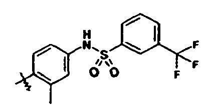 | M+H = 588.0 _{C}t*_{Ret}* = 3.97 min |
| 28 | 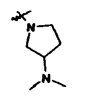 | 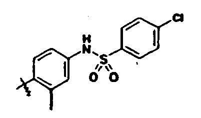 | M+ = 554.0 _{C}t*_{Ret}* = 5.69 min |
| 29 | 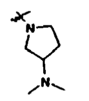 | 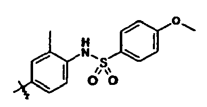 | M+H = 550.1 _{C}t*_{Ret}* = 3.03 min |
| 30 | 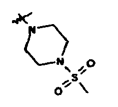 | 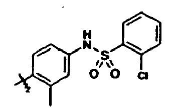 | M+ = 603.9 _{C}t*_{Ret}* = 4.27 min |
| 31 | 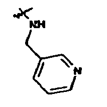 | 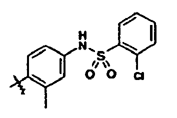 | M+ = 547.9 _{C}t*_{Ret}* = 3.21 min |
| 32 | 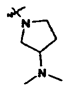 | 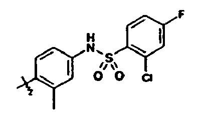 | M+ = 572.0 _{C}t*_{Ret}* = 3.73 min |
| 33 | 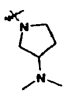 | 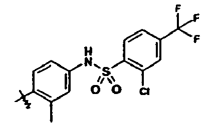 | M+ = 622.0 _{C}t*_{Ret}* = 4.34 min |
| 34 | 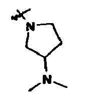 | 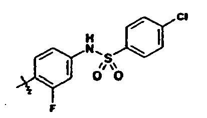 | M+ = 558.0 _{C}t*_{Ret}* = 3.17 min |
| 35 | 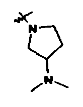 | 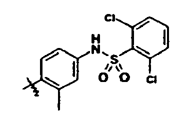 | M+ = 588.0 _{C}t*_{Ret}* = 3.18 min |
| 36 | 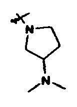 | 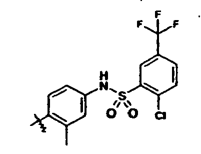 | M+ = 622.0 _{C}t*_{Ret}* = 3.44 min |
| 37 | 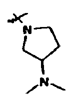 | 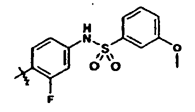 | M+H = 554.0 _{C}t*_{Ret}* = 2.88 min |
| 38 | 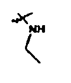 | 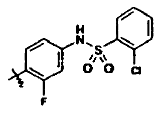 | M+ = 490.0 279-280 °C |
| 39 | 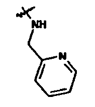 | 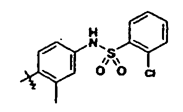 | M+ = 548.0 _{C}t*_{Ret}* = 3.33 min |
| 40 | 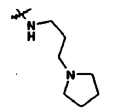 | 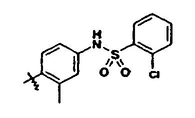 | M+ = 568.1 _{C}t*_{Ret}* = 3.60 min |
| 41 | 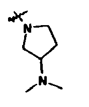 | 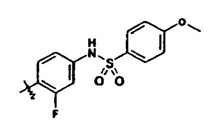 | M+H = 554.0 _{C}t*_{Ret}* = 3.21 min |
| 42 | 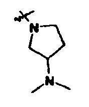 | 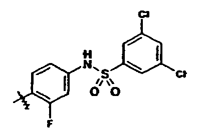 | M+ = 592.0 _{C}t*_{Ret}* = 3.51 min |
| 43 | 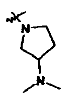 | 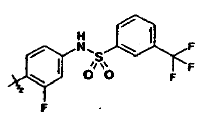 | M+H = 592.0 _{C}t*_{Ret}* = 2.73 min |
| 44 | 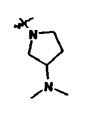 | 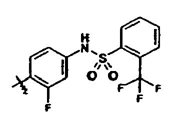 | M-H = 590.3 _{C}t*_{Ret}* = 3.18 min |
| 45 | 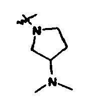 | 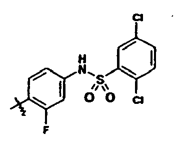 | M+ = 592.0 _{C}t*_{Ret}* = 3.28 min |
| 46 | 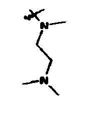 | 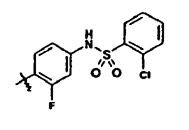 | M+ = 547.0 164-165 °C |
| 47 | 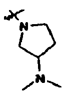 | 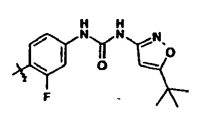 | M+ = 549.0 _{B}t*_{Ret}* = 1.95min |
| 48 | 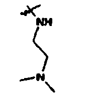 | 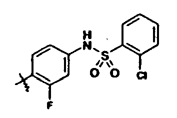 | M+H = 549.5 157-158 °C |
| 49 | 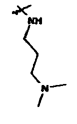 | 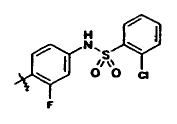 | M+H = 563.5 159-160 °C |
| 50 | 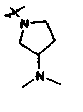 | 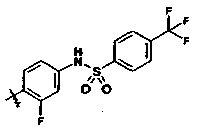 | M+H = 592.1 _{C}t*_{Ret}* = 3.93 min |
| 51 | 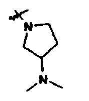 | 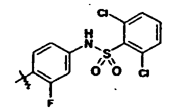 | M+ = 592.0 _{C}t*_{Ret}* = 3.72 min |
| 52 | 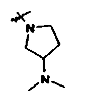 | 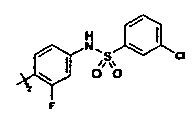 | M+ = 558.0 _{C}t*_{Ret}* = 3.70 min |
| 53 | 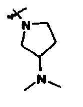 | 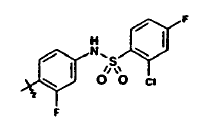 | M+ = 576.3 _{C}t*_{Ret}* = 3.34 min |
| 54 | 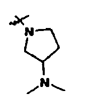 | 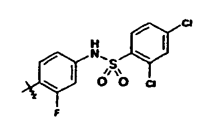 | M+ = 592.0 _{C}t*_{Ret}* = 3.90 min |
| 55 | 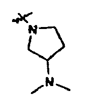 | 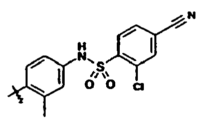 | M+ = 579.0 _{C}t*_{Ret}* = 3.30 min |
| 56 | 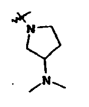 | 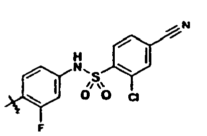 | M+ = 583.0 _{C}t*_{Ret}* = 3.35 min |
| 57 | 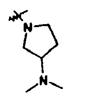 | 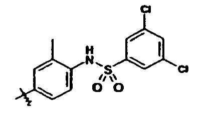 | M+ = 588.0 _{C}t*_{Ret}* = 4.16 min |
| 58 | 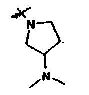 | 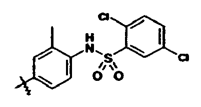 | M+ = 588.0 _{C}t*_{Ret}* = 2.57 min |
| 59 | 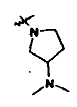 | 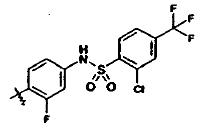 | M+ = 626.0 _{C}t*_{Ret}* = 407 min |
| 60 | 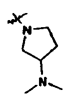 | 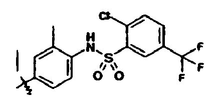 | M-H = 621.9 _{C}t*_{Ret}* = 4.22 min |
| 61 | 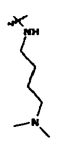 | 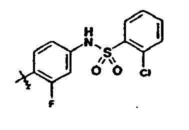 | M+H = 577.9 138-140 °C |
| 62 | 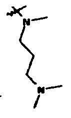 | 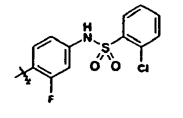 | M+H = 577.9 150-152 °C |
| 63 | 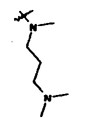 | 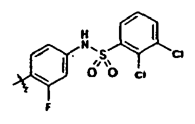 | M+ = 595.5 143-146 °C |
| 64 | 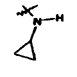 | 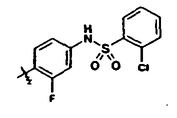 | M+ = 502.0 270-271 °C |
| 65 | 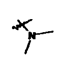 | 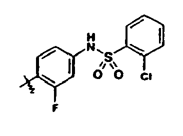 | M+ = 490.0 279-280 °C |
| 66 | 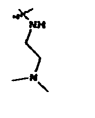 | 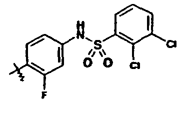 | M+ = 567.5 168-170 °C |
| 67 | 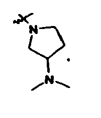 | 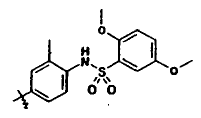 | M+ = 580.1 _{C}t*_{Ret}* = 2.17 min |
| 68 | 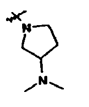 | 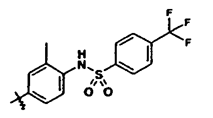 | M+ = 588.0 _{C}t*_{Ret}* = 4.03 min |
| 69 | 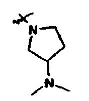 | 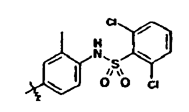 | M+ = 588.0 _{C}t*_{Ret}* = 2.36 min |
| 70 | 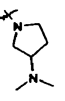 | 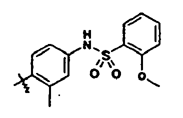 | M+ H = 550.1 _{C}t*_{Ret}* = 2.53 min |
| 71 | 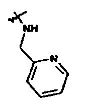 | 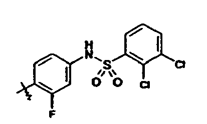 | M+ = 586.0 _{C}t*_{Ret}* = 3.87 min |
| 72 | 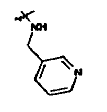 | 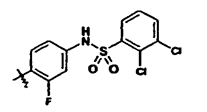 | M+ = 586.0 _{C}t*_{Ret}* = 2.84 min |
| 73 | 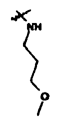 | 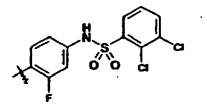 | M+ = 567.0 _{C}t*_{Ret}* = 2.56 min |
| 74 | 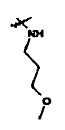 | 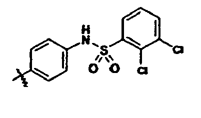 | M+ = 549.0 _{C}t*_{Ret}* = 4.29 min |
| 75 | 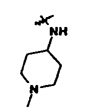 | 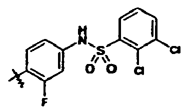 | M+ = 592.1 _{C}t*_{Ret}* = 3.74 min |
| 76 | 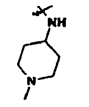 | 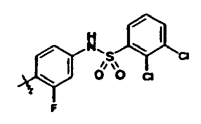 | M+ = 592.1 _{C}t*_{Ret}* = 3.74 min |
| 77 | 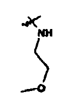 | 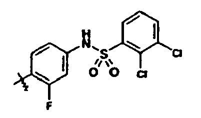 | M+ H = 554.9 _{C}t*_{Ret}* = 4.14 min |
| 78 | 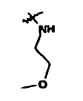 | 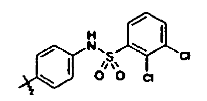 | M+ = 535.0 _{C}t*_{Ret}* = 4.14 min |
| 79 | 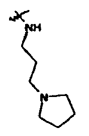 | 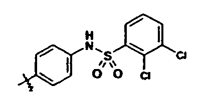 | M+ = 588.2 _{C}t*_{Ret}* = 3.78 min |
| 80 | 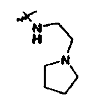 | 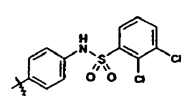 | M+ = 574.1 _{C}t*_{Ret}* = 3.81 min |
| 81 | 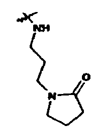 | 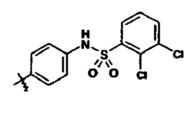 | M+ = 601.9 _{C}t*_{Ret}* = 3.11 min |
| 82 | 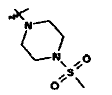 | 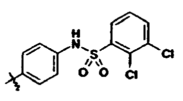 | M-H = 622.2 _{C}t*_{Ret}* = 3.78 min |
| 83 | 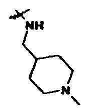 | 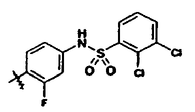 | M+ = 606.1 _{C}t*_{Ret}* = 3.78 min |
| 84 | 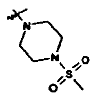 | 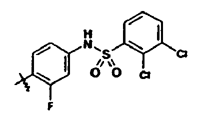 | M+ = 642.0 _{C}t*_{Ret}* = 4.06 min |
| 85 | 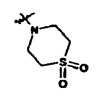 | 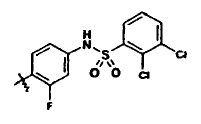 | M+ = 612.9 _{C}t*_{Ret}* = 2.94 min |
| 86 | 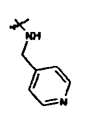 | 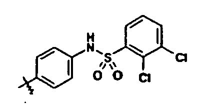 | M+ = 568.0 _{C}t*_{Ret}* = 3.78 min |
| 87 | 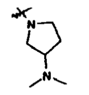 | 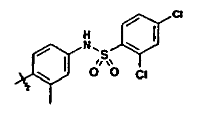 | M+ = 588.0 _{C}t*_{Ret}* = 2.45 min |
| 88 | 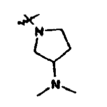 | 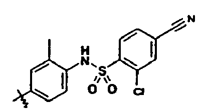 | M+ = 579.1 _{C}t*_{Ret}* = 3.07 min |
| 89 | 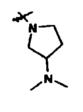 | 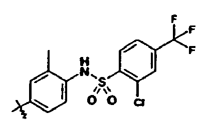 | M+ = 622.1 _{C}t*_{Ret}* = 3.76 min |
| 90 | 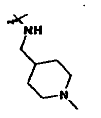 | 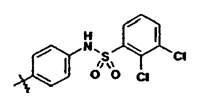 | M+ = 588.1 _{C}t*_{Ret}* = 3.79 min |
| 91 | 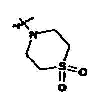 | 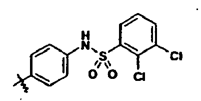 | M+ = 593.2 _{C}t*_{Ret}* = 3.70 min |
| 92 | 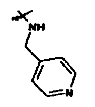 | 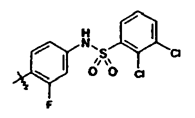 | M+ = 586.1 _{C}t*_{Ret}* = 3.92 min |
| 93 | 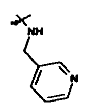 | 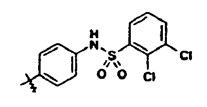 | M+ = 606.1 _{C}t*_{Ret}* = 3.78 min |
| 94 | 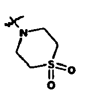 | 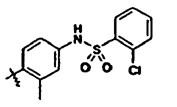 | M+ = 574.9 _{O}t*_{Ret}* = 2.55 min |

### Example 95 N-[4-(7-Amino-3-{[(3-dimethylamino-propyl)-methyl-amino]-methyl}-pyrazolo[1,5-a]pyrimidin-6-yl)-3-chlorophenyl]-2-chloro-benzenesulfoneamide

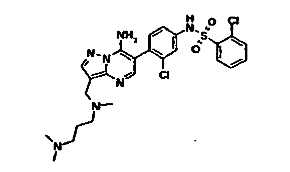

N-[4-(7-Amino-3-chloromethyl-pyrazolo[1,5-a]pyrimidin-6-yl)-3-chlorophenyl]-benzene sulfonamide (195 mg, 0.40 mmol) is dissolved in THF (4 mL) and N,N,N trimethyl-1,3-propyldiamine (0.077mL, 0.52 mmol) is added at ambient temperature. The reaction is stirred for 2h and then concentrated. The solid residue is dissolved in H₂O and basified with dilute NaOH leading to formation of a white precipitate of the title compound which is isolated by filtration and dried under vacuum. ¹H NMR (DMSO-d6) 8.33 (s, 1H). 8.04 (s, 1H), 7.99 (s, 1H), 7.60 (s, 1H), 7.45-7.38 (m, 4H), 6.98 (m, 2H), 6.48 (d, 1H), 4.61 (s, 1H), 3.02 (m, 2H), 3.01 (s, 6H), 2.29 (s, 3H), 2.19-2.14 (m, 2H), 2.01 -1.93 (m, 2H).

### Intermediate 95.1 N-[4-(7-Amino-3-chloromethyl-pyrazolo[1,5-a]pyrimidin-6-yl)-3-chlorophenyl]-benzenesulfonamide

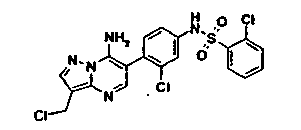

N-[4-(7-Amino-3-hydroxymethyl-pyrazolo[1,5-a]pyrimidin-6-yl)-3-chlorophenyl]-benzenesulfone amide (150 mg, 0.32 mmol) is dissolved in THF (3 mL) and treated with thionylchloride (0.154 mL, 1.29 mmol) at ambient temperature. The reaction mixture is stirred for 1.5 h and then submitted to aqueous workup. To give the title compound as a yellow solid, which is directly subjected to the next step.

### Intermediate 95.2 N-[4-(7-Amino-3-hydroxymethyl-pyrazolo[1,5-a]pyrimidin-6-yl)-3-chlorophenyl]-benzenesulfonamide

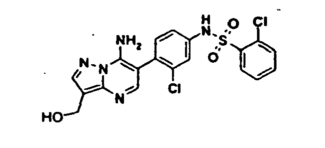

7-Amino-6-[2-chloro-benzene sulfonylamino)3-chloro-phenyl]-pyrazolo[1,5-a]pyrimidine-3-carboxylic acid ethyl ester (500 mg, 0.98 mmol) is dissolved in THF (6 mL) at ambient temperature. It is treated with LAH (112 mg, 2.97 mmol) in small portions and stirred for 2h. The reaction mixture is submitted to aqueous workup. The organic layers are combined dried and concentrated to give the title compound as a yellow solid. ES-MS: M+ = 463.6, HPLC: _{C}t_{Ret} = 1.00 min.

### Intermediate 95.3 7-Amino-6-[2-chloro-benzene sulfonylamino)-phenyl]-pyrazolo[1,5-a]pyrimidine-3-carboxylic acid ethyl ester

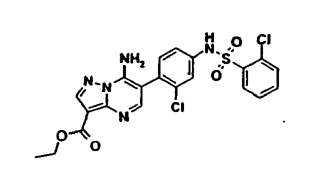

2-Chloro-N-[3-chloro-4-((-1-cyano-2-dimethylamino-vinyl)phenyl]-benzene sulfonamide (3.3 g, 8.0 mmol) is dissolved in HCl/EtOH (1.25 M) and treated with 5-amino-1H-pyrazole-4-carboxylic acid ethyl ester (1.2 g, 8.0 mmol) at ambient temperature. The reaction mixture is then stirred at 100°C overnight and allowed to cool to ambient temperature again. It is diluted with H₂O, leading to formation of a yellow precipitated of the title compound which is isolated by filtration and dried under vacuum to give a yellow powder. ES-MS: M+ = 506.1; ¹H NMR (MeOH-d4) 8.49 (s, 1H), 8.23, (d, 1H), 8.05 (s, 1H), 7.63 (d, 2H), 7.57-7.53 (m, 1H), 7.42 (s, 1H), 7.35 (d, 1H), 7.27 (d, 1H), 4.39 (d, 2H), 1.42 (t, 3H).

### Intermediate 95.4 2-Chloro-N-[3-chloro-4((-1-cyano-2-dimethylamino-vinyl)phenyl]-benzene sulfonamide

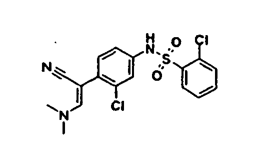

(Z)-2-(4-Amino-2-chloro-phenyl)-3-dimethylaminoacrylonitrile (1.57 g, 7.1 mmol) is dissolved in pyridine (30 mL). 2-Chlorobenzene sulfonylchloride (0.98 mL, 7.1 mmol) is added dropwise at ambient temperature and the reaction mixture is stirred for 45 min. It is concentrated under reduced pressure and the residual crude product purified by flash chromatography (SiO₂, gradient CH₂Cl₂/MeOH: 0-5 % MeOH) to give the title compound as a yellow solid. ES-MS: M+ = 341.9, HPLC: _{C}t_{Ret} = 2.27 min.

### Intermediate 95.5 (Z)-2-(4-Amino-2-chloro-phenyl)-3-dimethylaminoacrylonitrile

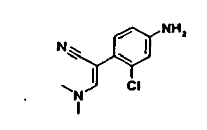

(Z)-2-(2-Chloro-4-nitro-phenyl)-3-dimethylaminoacrylonitrile (2.9 g, 11.5 mmol) is dissolved in MeOH (150 mL) and submitted to hydrogenation over Raney -Nickel (0.8 g) at ambient pressure and temperature for 4 h. The reaction mixture is then filtered over a pad of celite . The filtrate is concentrated and dried under vacuum to give the title compound as a brown solid. ES-MS: M+ = 222.1, HPLC: _{C}t_{Ret} = 1.61 min

### Intermediate 95.6 (Z)-2-(2-Chloro-4-nitro-phenyl)-3-dimethylaminoacrylonitrile

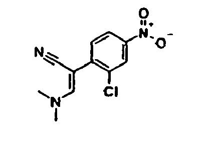

2-Chloro-4-nitro-phenyl)-acetonitrile (see W02005/054238, ex 57b; 5.5 g, 26 mmol) is dissolved in toluene (55 mL) and treated with dimethoxymethyl-dimethyl amine (3.0 g, 26 mmol). The reaction mixture is subsequently heated to reflux for 6h, cooled to rt again and concentrated to give the title compound as a brown oil which was directly used for the next step. ES-MS: M+ = 253.7; ¹H NMR (CDCl₃) 7.96 (d, 1H), 7.87 (d, 1H), 7.53 (s, 1H), 7.37 (s, 1H).

### Example 96 N-[4-(7-Amino-3-{[(3-dimethylamino-propyl)-methyl-amino]-methyl}-pyrazolo[1,5-a]pyrimidin-6-yl)-3-chlorophenyl]-2-chloro-benzenesulfoneamide

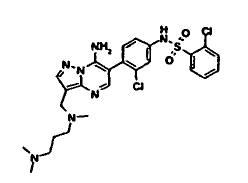

N-(4-(7-Amino-3-chloromethyl-pyrazolo[1,5-a]pyrimidin-6-yl)-3-chlorophenyl]-benzene sulfoneamide (195 mg, 0.40 mmol) is dissolved in THF (4 mL) and N,N,N trimethyl-1,3-propyldiamine (0.077mL, 0.52 mmol) is added at ambient temperature. The reaction is stirred for 2h and then concentrated . The solid residue is dissolved in H₂O and basified with dilute NaOH leading to formation of a white precipitate of the title compound which is isolated by filtration and dried under vacuum. ¹H NMR (DMSO-d6) 8.33 (s, 1H), 8.04 (s, 1H), 7.99 (s, 1H), 7.60 (s, 1H), 7.45-7.38 (m, 4H), 6.98 (m, 2H), 6.48 (d, 1H), 4.61 (s, 1H), 3.02 (m, 2H), 3.01 (s, 6H), 2.29 (s, 3H), 2.19-2.14 (m, 2H), 2.01 -1.93 (m, 2H).

### Intermediate 96.1 N-[4-(7-Amino-3-chloromethyl-pyrazolo[1,5-a]pyrimidin-6-yl)-3-chlorophenyo-benzenesulfoneamide

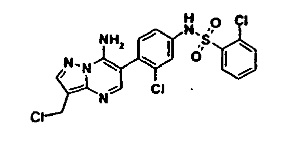

N-[4-(7-Amino-3-hydroxymethyl-pyrazolo[1,5-a]pyrimidin-6-yl)-3-chlorophenyl]-benzenesulfone amide (150 mg, 0.32 mmol) is dissolved in THF (3 mL) and treated with thionylchloride (0.154 mL, 1.29 mmol) at ambient temperature. The reaction mixture is stirred for 1.5 h and then submitted to aqueous workup. To give the title compound as a yellow solid, which is directly submitted to the next step.

### Intermediate 96.2 (compound of the formula XIII) N-[4-(7-Amino-3-hydroxymethylpyrazolo[1,5-a]pyrimidin-6-yl)-3-chlorophenyl]-benzenesulfonamide

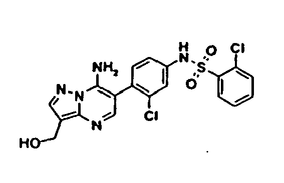

7-Amino-6-[2-chloro-benzene sulfonylamino)-phenyl]-pyrazolo[1,5-a]pyrimidine-3-carboxylic acid ethyl ester (500 mg, 0.98 mmol) is dissolved in THF (6 mL) at ambient temperature. It is treated with LAH (112 mg, 2.97 mmol) in small portions and stirred for 2h. The reaction mixture is submitted to aqueous workup. The organic layers are combined, dried and concentrated to give the title compound as a yellow solid. ES-MS: M+ = 463.6, HPLC: _{c}t_{Ret}= 1.00 min.

### Intermediate 96.3 (Compound of the formula XII) 7-Amino-6-[2-chloro-benzene sulfonylamino)-3-chloro phenyl]-pyrazolo[1,5-a]pyrimidine-3-carboxylic acid ethyl ester

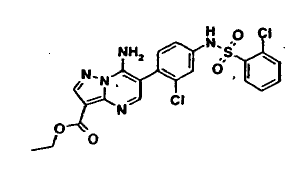

2-Chloro-N-(3-chloro-4-((-1-cyano-2-dimethylamino-vinyl)phenyl]-benzene sulfonamide (3.3 g, 8.0 mmol) is dissolved in HCl/ETOH (1.25 M) and treated with 5-amino-1H-pyrazole-4-carboxylic acid ethyl ester (1.2 g, 8.0 mmol) at ambient temperature. The reaction mixture is then stirred at 100°C overnight and allowed to cool to ambient temperature again. It is diluted with H₂O, leading to formation of a yellow precipitate of the title compound which is isolated by filtration and dried under vacuum to give a yellow powder. ES-MS: M+ = 506.1; ¹H NMR (MeOH-d4) 8.49 (s, 1H), 8.23, (d, 1H), 8.05 (s, 1H), 7.63 (d, 2H), 7.57-7.53 (m, 1H), 7.42 (s, 1H), 7.35 (d, 1H), 7.27 (d, 1H), 4.39 (d, 2H), 1.42 (t, 3H).

### Intermediate 96.4 2-Chloro-N-[3-chloro-4-((-1-cyano-2-dimethylamino-vinyl)phenyl]-benzene sulfonamide

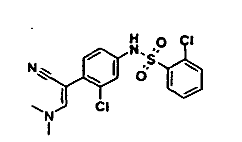

(Z)-2-(4-Amino-2-chloro-phenyl)-3-dimethylaminoacrylonitrile (1.57g, 7.1 mmol) is dissolved in pyridine (30 mL). 2-Chlorobenzene sulfonylchloride (0.98 mL, 7.1 mmol) is added dropwise at ambient temperature and the reaction mixture is stirred for 45 min. It is concentrated under reduced pressure and the residual crude product purified by flash chromatography (SiO₂. gradient CH₂Cl₂/MeOH: 0-5 % MeOH) to give the title compound as a yellow solid. ES-MS: M+ = 341.9, HPLC: _{c}t_{Ret} = 2.27 min.

### Intermediate 96.5 (2)-2-(4-Amino-2-chloro-phenyl)-3-dimethylaminoacrylonitrile

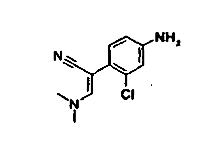

(Z)-2-(2-Chloro-4-nitro-phenyl)-3-dimethylaminoacrylonitrile (2.9 g, 11.5 mmol) is dissolved in MeOH (150 mL) and submitted to hydrogenation over Raney -Nickel (0.8 g) at ambient pressure and temperature for 4 h. The reaction mixture is then filtered over a pad of celite . The filtrate is concentrated and dried under vacuum to give the title compound as a brown solid. ES-MS: M+ = 222.1, HPLC: _{c}t_{Rel} = 1.61 min

### Intermediate 96.6 (Z)-2-(2-Chloro-4-nitro-phenyl-3-dimethylaminoacrylonitrile

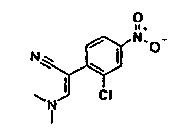

2-Chloro-4-nitro-phenyl)-acetonitrile (see W02005/054238, ex 57b; 5.5 g, 26 mmol) is dissolved in toluene (55 mL) and treated with dimethoxymethyl-dimethyl amine (3.0 g, 26 mmol). The reaction mixture is subsequently heated to reflux for 6h, cooled to rt again and concentrated to give the title compound as a brown oil which was directly used for the next step. ES-MS: M+ = 253.7; ¹H NMR (CDCl₃) 7.96 (d, 1H), 7.87 (d, 1H), 7.53 (s, 1H), 7.37 (s, 1H).

### Example 97 (compound of the formula XII - also useful as intermediate for compounds of the formula I): 7-Amino-6-[2-chloro-benzene sulfonylamino)-3-fluoro-phenyl]-pyrazolo[1 ,5-a]pyrimidine-3-carboxylic acid ethyl ester

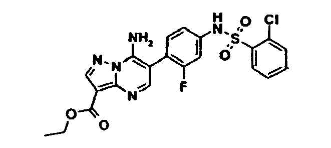

The title compound is prepared in analogy to intermediate 96.3 starting from 2-fluoro-4-nitrophenyl)-acetonitrile (see WO2005/054238). ES-MS: M+ = 490.9, m.p. 271-273 °C

### Example 98 (compound of the formula XIII - also useful as intermediate for compounds of the formula I): N-[4-(7-Amino-3-hydroxymethyl-pyrazolo[1,5-a]pyrimidin-6-yl)-3-fluorophenyl]-2-chloro-benzenesulfonamide

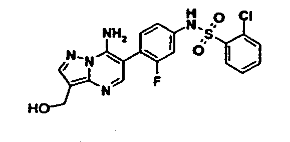

The title compound is prepared in analogy to intermediate 96.2 from 7-amino-6-[2-chlorobenzene sulfonylamino)-3-fluoro-phenyl]-pyrazolo[1,5-a]pyrimidine-3-carboxylic acid ethyl ester ES-MS: M+ = 448.6. m.p. 204-208 °C

### Example 99 (compound of the formula XII - also useful as intermediate for compounds of the formula I): 7-Amino-6-[2,3-dichloro-benzene sulfonylamino)-3-fluoro-phenyl]-pyrazolo[1,5-a]pyrimidine-3-carboxylic acid ethyl ester

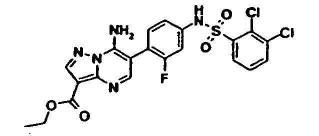

The title compound is prepared in analogy to intermediate 96.3 starting from 2-fluoro-4-nitrophenyl)-acetonitrile (see WO2005/054238) and 2,3-dichlorobenzene sulfonylchloride. ES-MS: M+ = 525.4, m.p. 200-205 °C

### Example 100 (compound of the formula XIII - also useful as intermediate for compounds of the formula I): N-[4-(7-Amino-3-hydroxymethyl-pyrazolo[1,5-a]pyrimidin-6-yl)-3-fluorophenyl]-2,3-dichloro-benzenesulfonamide

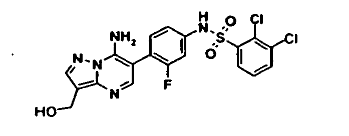

The title compound is prepared in analogy to intermediate 96.2 from 7-amino-6-[2,3-dichlorobenzene sulfonylamino)-3-fluoro-phenyl]-pyrazolo[1,5-a]pyrimidine-3-carboxylic acid ethyl ester. ES-MS: M+ = 481.6; HPLC: _{B}t_{Ret} = 2.00 min.

### Example 101 (example for a compound of the formula XIII with etherified hydroxy): N-[4-(7-Amino-3-methoxymethyl-pyrazolo[1,5-a]pyrimidin-6-yl)-3-fluorophenyo-2,3-dichloro-benzenesulfonamide

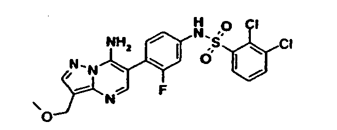

The title compound is prepared in analogy to example 96 by direct quenching of N-[4-(7-amino-3-chloromethyl-pyrazolo[1,5-a]pyrimidin-6-yl)-2,3-dichlorophenyl]-benzenesulfone-amide with MeOH at rt . ES-MS: M+ = 495.6 ; ¹H NMR (MeOH-d₄) 8.19 (d, 1H), 8.09 (s, 1H), 7.96 (s, 1H), 7.80 (d, 1H), 7.49 (dd, 1H), 7.33 (dd, 1H), 7.11-7.09 (m, 1H), 7.08 (s, 1H), 4.63 (s, 2H), 3.36 (s, 3H). HPLC: _{B}t_{Rel} = 2.03 min.

### Intermediate 101.1: N-[4-(7-Amino-3-chloromethyl-pyrazolo[1,5-a]pyrimidin-6-yl)-2,3-dichlorophenyl]-benzenesulfoneamide

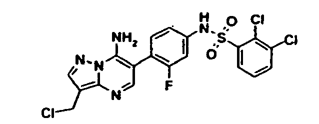

prepared in analogy to intermediate 96.1 from N-[4-(7-amino-3-hydroxymethyl-pyrazolo[1,5-a]pyrimidin-6-yl)-3-fluorophenyl]-2,3-dichloro-benzenesulfonamide and directly used for the next step.

### Example 102: (example for a compound of the formula XIII with etherified hydroxy) N-[4-(7-Amino-3-(2-methoxyethoxymethyl)-pyrazolo[1,5-a]pyrimidin-6-yl)-3-fluorophenyl]-2,3-dichloro-benzenesulfonamide

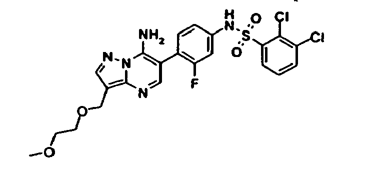

The title compound is prepared in analogy to example 96 by direct quenching of N-[4-(7-amino-3-chloromethyl-pyrazolo[1,5-a]pyrimidin-6-yl)-2,3-dichlorophenyl]-benzenesulfone-amide with methoxyethanol at rt. ES-MS: M+ = 539.8 ; ¹H NMR (MeOH-d₄) 8.19 (d, 1H), 8.11 (s, 1H), 7.96 (s, 1H), 7.81 (d, 1H), 7.49 (dd, 1H), 7.33 (dd, 1H), 7.11-7.09 (m, 1H), 7.08 (s, 1H), 4.71 (s, 2H), 3.66-3.63 (m, 2H), 3.55-3.52 (m, 2H) 3.28 (s, 3H). HPLC: **_{B}t_{Ret}** = 2.09 min.

### Example 103: (example for a compound of the formula XIII) N-[4-(7-Amino-3-hydroxymethyl-pyrazolo[1,5-a]pyrimidin-6-yl)-phenyl]-2,3-dichlorobenzenesulfonamide

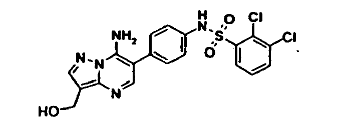

The title compound is prepared in analogy to intermediate 96.2 from 7-amino-6-[2,3-dichlorobenzene sulfonylamino)-phenyl]-pyrazolo[1,5-a]pyrimidine-3-carboxylic acid ethyl ester (intermediate 1.1). ES-MS: M+ = 463.7 ; HPLC: _{B}t_{Ret} = 1.99 min

### Example 104: N-[4-(7-Amino-3-dimethylaminomethyl-pyrazolo[1,5-a]pyrimidin-6-yl)-phenyl]-2,3-dichloro-benzenesulfonamide

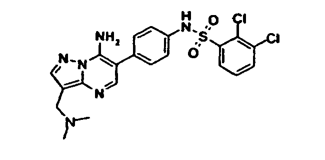

The title compound is prepared in analogy to example 96 by direct quenching of N-[4-(7-amino-3-chloromethy)-pyrazolo[1,5-a]pyrimidin-6-yl)-2,3-dichlorophenyl]-benzene-sulfoneamide with 2M solution of dimethylamine in THF at rt. ES-MS: M+ = 445.7 ; HPLC: _{B}t_{Ret} = 1.94 min.

### Example 105: Soft Capsules

5000 soft gelatin capsules, each comprising as active ingredient 0.05 g of one of the compounds of formula I mentioned in any one of the preceding examples, are prepared as follows:

**Composition**

| | |
|---|---|
| Active ingredient | 250 g |
| Lauroglycol | 2 litres |

Preparation process: The pulverized active ingredient is suspended in Lauroglykol* (propylene glycol laurate, Gattefossé S.A., Saint Priest, France) and ground in a wet pulverizer to produce a particle size of about 1 to 3 µm. 0.419 g portions of the mixture are then introduced into soft gelatin capsules using a capsule-filling machine.

### Example 106: Tablets comprising compounds of the formula I

Tablets, comprising, as active ingredient, 100 mg of any one of the compounds of formula I of Examples 1 to 132 are prepared with the following composition, following standard procedures:

**Composition**

| | |
|---|---|
| Active Ingredient | 100 mg |
| crystalline lactose | 240 mg |
| Avicel | 80 mg |
| PVPPXL | 20 mg |
| Aerosil | 2 mg |
| magnesium stearate | 5 mg |
| | 447 mg |

Manufacture: The active ingredient is mixed with the carrier materials and compressed by means of a tabletting machine (Korsch EKO, Stempeldurchmesser 10 mm). Avicel® is microcrystalline cellulose (FMC, Philadelphia, USA). PVPPXL is polyvinylpolypyrrolidone, cross-linked (BASF, Germany). Aerosil® is silicium dioxide (Degussa, Germany).

## Claims

1. A compound of the formula I, 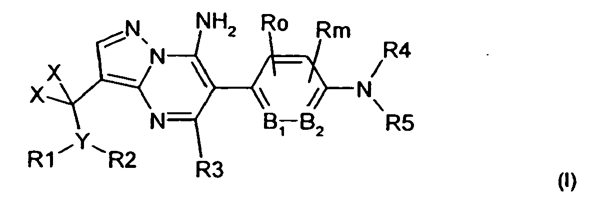 wherein
either
each of R1 and R2 is, independently of the other, unsubstituted or substituted C₁-C₇ alkyl, unsubstituted or substituted C₃-C₁₀-cycloalkyl, unsubstituted or substituted C₆-C₁₄-aryl or unsubstituted or substituted heterocyclyl with 3 to 14 ring atoms and Y is N,
or R1, Y and R2 together form an unsubstituted or substituted heterocyclyl with 3 to 14 ring atoms and at least one nitrogen heteroatom which is bound via a ring nitrogen;
each of the two X stands for hydrogen or both together form oxo or thioxo;
R3 is hydrogen or C₁-C₇ alkyl;
R4 is hydrogen or unsubstituted or substituted C₁-C₇ alkyl;
R5 is acyl;
B₁ is N or CRo,
B₂ is N or CRm,
and each of Ro and Rm, independently of the other(s), is hydrogen, C₁-C₇ alkyl, halo or C₁-C₇ alkoxy;
or a salt thereof.

2. A compound of the formula I according to claim 1 wherein
R5 is unsubstituted or substituted C₆-C₁₄-arylaminocarbonyl which is especially preferred, unsubstituted or substituted heterocyclylaminocarbonyl which is especially preferred and wherein heterocyclyl has 3 to 14 ring atoms, unsubstituted or substituted C₆-C₁₄-arylaminosulfonyl, unsubstituted or substituted heterocyclylaminosulfonyl wherein heterocyclyl has 3 to 14 ring atoms, unsubstituted or substituted lower-alkanesulfonyl which is especially preferred, unsubstituted or substituted C₆-C₁₄-arylsulfonyl which is especially preferred, unsubstituted or substituted heterocyclylsulfonyl wherein heterocyclyl has 3 to 14 ring atoms, or unsubstituted or substituted C₆-C₁₄-arylcarbonyl,
and the other symbols R1, R2, Y, X, R3, R4, B₁, B₂, Ro and Rm have the meanings given in claim 1,
or a (preferably pharmaceutically acceptable) salt thereof.

3. A compound of the formula I according to claim 1, wherein
R5 is phenylaminocarbonyl wherein phenyl is unsubstituted or substituted by one or more moieties independently selected from C₁-C₇ alkyl, halo (very preferred), especially chloro;
halo-C₁-C₇ alkyl, C₁-C₇ alkoxy and cyano;
pyrazolyl-aminocarbonyl or isoxazolylaminocarbonyl where pyrazolyl or isoxazolyl is unsubstituted or substituted by one or two moieties independently selected from the group consisting of C₁-C₇ alkyl and phenyl that is unsubstituted or substituted with halo, C₁-C₇ alkoxy, piperazino-C₁-C₇ alkyl, 4-C₁-C₇ alkylpiperazino-C₁-C₇ alkyl and morpholino-C₁-C₇ alkyl; pyrazolyl-aminosulfonyl or isoxazolylaminosulfonyl, where each pyrazolyl or isoxazolyl is unsubstituted or substituted by one or two moieties independently selected from the group consisting of C₁-C₇ alkyl and phenyl that is unsubstituted or substituted with halo, C₁-C₇ alkoxy, piperazino-C₁-C₇ alkyl, 4-C₁-C₇ alkylpiperazino-C₁-C₇ alkyl and morpholino-C₁-C₇ alkyl; phenyl C₁-C₇ alkanesulfonyl, wherein phenyl is unsubstituted (preferred) or substituted with one or more, e.g. up to three, moieties independently selected from the group consisting of C₁-C₇ alkyl, halo (especially preferred), halo-C₁-C₇ alkyl, C₁-C₇ alkoxy and cyano; phenylsulfonyl wherein the phenyl is unsubstituted or substituted by one or more moieties independently selected from the group consisting of C₁-C₇ alkyl, halo (preferred), halo-C₁-C₇ alkyl, C₁-C₇ alkoxy and cyano;
and the other symbols R1, R2, X, Y, R3, R4, B₁, B₂, Ro and Rm have the meanings given in claim 1,
or a (preferably pharmaceutically acceptable) salt thereof.

4. A compound of the formula I according to claim 1 wherein
R5 is 3-trifluoromethyl-phenylaminocarbonyl, 4-fluorophenylaminocarbonyl, 3- or 2-chlorophenylaminocarbonyl, 3-tert-butyl-1-(4-fluorophenyl)-pyrazol-5-ylaminocarbonyl, 3-tert-butyl-1-(4-methoxyphenyl)-pyrazol-5-ylaminocarbonyl, 3-tert-butyl-1-(4-(4-methylpiperazinomethyl)-phenyl)-pyrazol-5-ylaminocarbonyl, 3-tert-butyl-1-(3-(4-methylpiperazinomethyl)-phenyl)-pyrazol-5-ylaminocarbonyl, 3-tert-butyl-1-(4-(morpholinomethyl)-phenyl)-pyrazol-5-ylaminocarbonyl, 5-tert-butyl-isoxazol-3-ylaminocarbonyl, 3-tert-butyl-1-(4-fluorophenyl)-pyrazol-5-ylaminosulfonyl, phenylmethylsulfonyl or 2-phenylethylsulfonyl, 2,3-dimethylphenylsulfonyl, 2-, 3- or 4-methylphenylsulfonyl, 2-, 3- or 4-methoxyphenylsulfonyl, 2-methyl-4,5-dimethoxyphenylsulfonyl, 2,5-dimethoxyphenylsulfonyl, 2-, 3- or 4-trifluoromethylphenylsulfonyl, 2-chloro-5-trifluoromethylphenylsulfonyl, 2-chloro-4-trifluoromethylphenylsulfonyl, or especially 2,- 3- or 4-chlorophenylsulfonyl, 2,3-, 2,4-, 2,5-, 3,5- or 2,6-dichlorophenylsulfonyl, 2-chloro-4-cyanophenylsulfonyl or 4-fluoro-2-chlorophenylsulfonyl; and the other symbols R1, R2, X, Y, R3, R4, B₁, B₂, Ro and Rm have the meanings given in claim 1,
or a (preferably pharmaceutically acceptable) salt thereof.

5. A compound of the formula I according to any one of claims 1 to 4 wherein
R1R2Y - taken together - is C₁-C₇ alkylamino, di-(C₁-C₇alkyl)-amino, unsubstituted amino- C₁-C₇ alkylamino, N-mono-, N,N-di- or N,N,N'-tri-(lower alkyl)-amino-C₁-C₇ alkyl-amino, C₁-C₇ alkoxy-C₁-C₇ alkylamino, pyrrolidinyl-C₁-C₇ alkylamino, oxopyrrolidinyl-C₁-C₇ alkylamino, piperidinyl-C₁-C₇ alkylamino, (N-C₁-C₇ alkylpiperidinyl)-C₁-C₇ alkylamino, pyridyl-C₁-C₇ alkylamino, C₃-C₆-cycloalkylamino, piperidinylamino, N-C₁-C₇ alkylpiperidinylamino, pyrrolidino, amino-, N-C₁-C₇ alkylamino- or N,N-di-C₁-C₇ alkylamino-pyrrolidino, amino-, N-C₁-C₇ alkylamino- or N,N-di-C₁-C₇ alkylamino-piperidino, piperazino, N-C₁-C₇ alkylpiperazino, N-C₁-C₇ alkanoyl-piperazino, N-C₁-C₇ alkanesulfonyl-piperazino, morpholino, thiomorpholino or S,S-dioxothiomorpholino;
R5 is as defined in any one of claims 1 to 4;
and the other symbols R3, R4, X, B₁, B₂, Ro and Rm have the meanings given in claim 1,
or a (preferably pharmaceutically acceptable) salt thereof.

6. A compound of the formula I according to any one of claims 1 to 5 wherein
R1, R2 and Y are as defined in any one of claims 1 or 5,
R 5 is as defined in any one of claims 1 to 4.
R3 is hydrogen or methyl,
R4 is hydrogen,
each X stands for hydrogen,
B₁ is N or CRo,
B₂ is CRm,
and each of Ro and Rm, independently of the other, is hydrogen, methyl, fluoro, chloro or methoxy, preferably chloro,
or a pharmaceutically acceptable salt thereof.

7. A compound of the formula I according to any one of claims 1 to 5 wherein
R1, R2 and Y are as defined in any one of claims 1 or 5.
R5 is as defined in any one of claims 1 to 4.
R3 is hydrogen or methyl,
R4 is hydrogen,
both X together form oxo,
B₁ is N or CRo,
B₂ is CRm,
and each of Ro and Rm, independently of the other, is hydrogen, methyl, fluoro, chloro or methoxy, preferably chloro,
or a pharmaceutically acceptable salt thereof.

8. A compound of the formula I according to claim 1 selected from the group of compounds with the names
N-{4-[7-amino-3-(3-dimethylamino-pyrrolidine-1-carbonyl)-pyrazolo[1,5-a]pyrimidin-6-yl]-phenyl}-2,3-dichloro-benzenesulfonamide
1-{4-[7-amino-3-(3-dimethylamino-pyrrolidine-1-carbonyly-pyrazolo[1,5-a]pyrimidin-6-yl]-3-methyl-phenyl}-3-(2-chloro-phenyl)-urea
N-[4-(7-amino-3{[(3-dimethylamino-propyl)-methyl-amino]-methyl}-pyrazolo[1,5-a]pyrimidin-6-yl)-3-chlorophenyl]-2-chloro-benzenesulfoneamide, and
N-[4-(7-amino-3-{[(3-dimethylamino-propyl)-methyl-amino]-methyl}-pyrazolo[1,5-a]pyrimidin-6-yl)-3-chlorophenyl]-2-chloro-benzenesulfoneamide
N-[4-(7-amino-3-dimethylaminomethyl-pyrazolo[1,5-a]pyrimidin-6-yl)-phenyl]-2,3-dichloro-benzenesulfonamide;
or a pharmaceutically acceptable salt thereof.

9. A compound of the formula I according to claim 1, selected from the group of compounds represented in the following table: 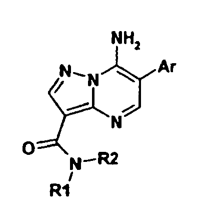
| compound | 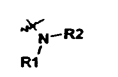 | 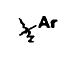 |
|---|---|---|
| 3 | 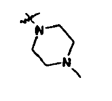 | 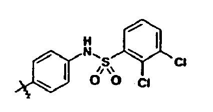 |
| 4 | 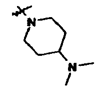 | 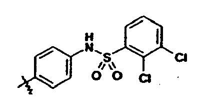 |
| 5 | 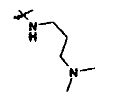 | 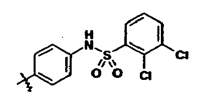 |
| 6 | 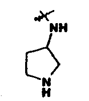 | 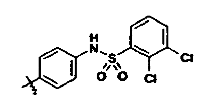 |
| 7 | 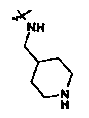 | 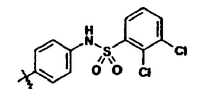 |
| 8 | 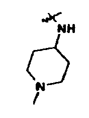 | 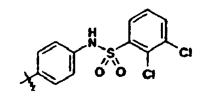 |
| 9 | 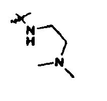 | 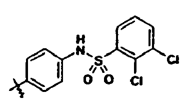 |
| 10 | 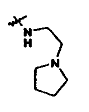 | 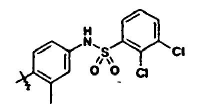 |
| 11 | 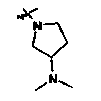 | 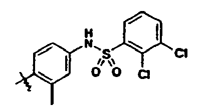 |
| 12 | 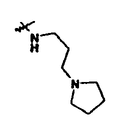 | 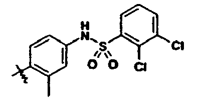 |
| 13 | 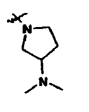 | 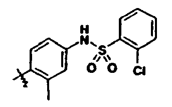 |
| 14 | 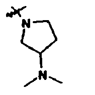 | 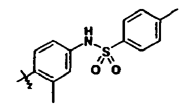 |
| 15 | 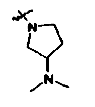 | 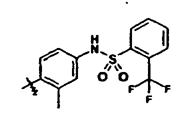 |
| 16 | 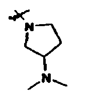 | 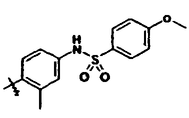 |
| 17 | 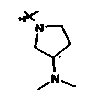 | 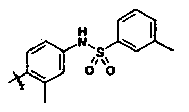 |
| 18 | 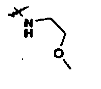 | 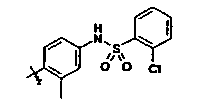 |
| 19 | 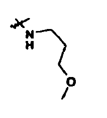 | 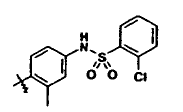 |
| 20 | 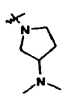 | 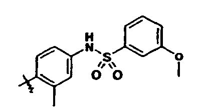 |
| 21 | 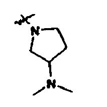 | 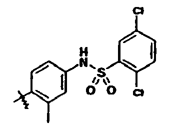 |
| 22 | 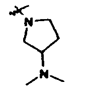 | 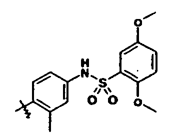 |
| 23 | 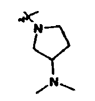 | 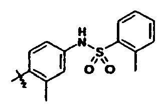 |
| 24 | 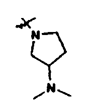 | 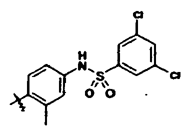 |
| 25 | 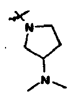 | 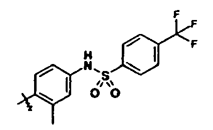 |
| 26 | 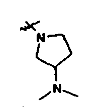 | 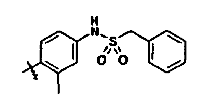 |
| 27 | 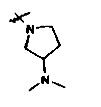 | 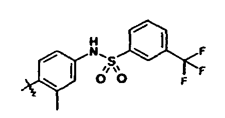 |
| 28 | 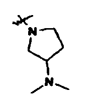 | 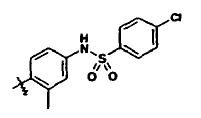 |
| 29 | 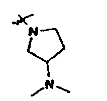 | 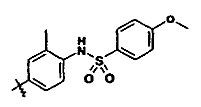 |
| 30 | 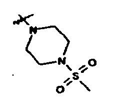 | 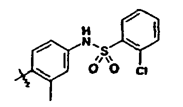 |
| 31 | 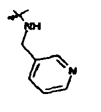 | 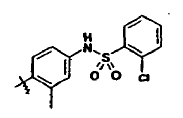 |
| 32 | 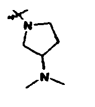 | 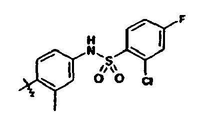 |
| 33 | 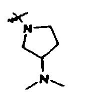 | 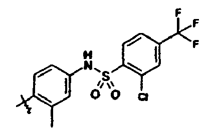 |
| 34 | 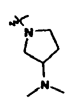 | 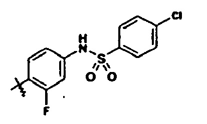 |
| 35 | 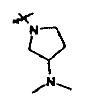 | 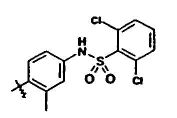 |
| 36 | 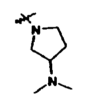 | 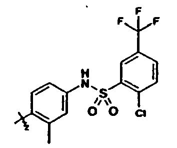 |
| 37 | 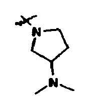 | 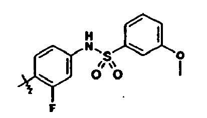 |
| 38 | 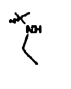 | 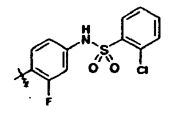 |
| 39 | 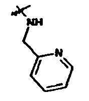 | 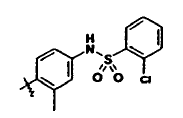 |
| 40 | 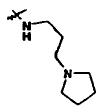 | 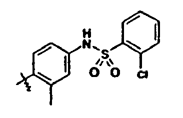 |
| 41 | 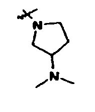 | 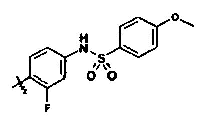 |
| 42 | 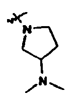 | 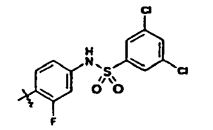 |
| 43 | 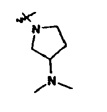 | 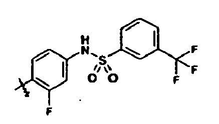 |
| 44 | 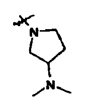 | 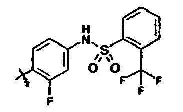 |
| 45 | 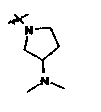 | 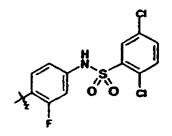 |
| 46 | 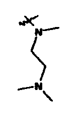 | 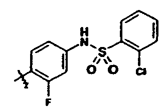 |
| 47 | 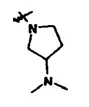 | 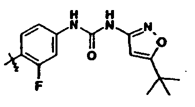 |
| 48 | 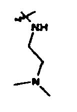 | 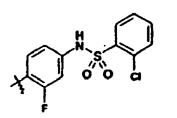 |
| 49 | 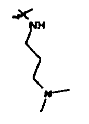 | 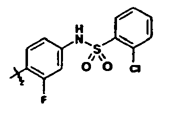 |
| 50 | 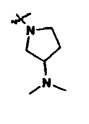 | 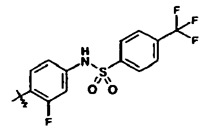 |
| 51 | 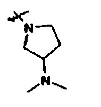 | 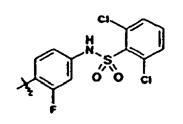 |
| 52 | 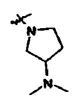 | 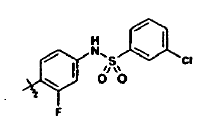 |
| 53 | 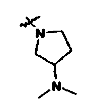 | 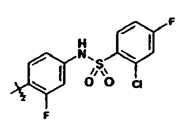 |
| 54 | 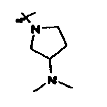 | 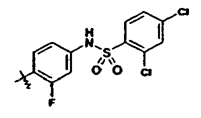 |
| 55 | 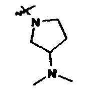 | 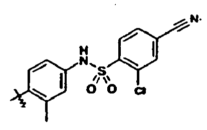 |
| 56 | 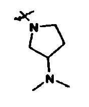 | 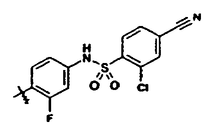 |
| 57 | 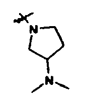 | 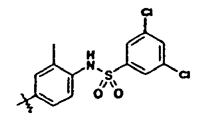 |
| 58 | 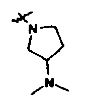 | 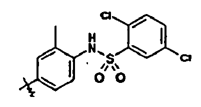 |
| 59 | 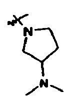 | 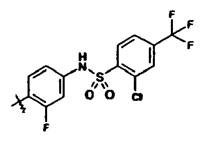 |
| 60 | 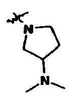 | 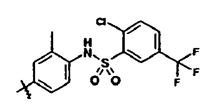 |
| 61 | 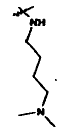 | 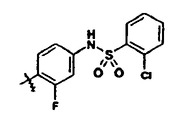 |
| 62 | 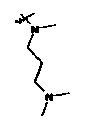 | 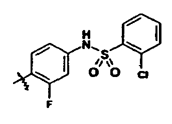 |
| 63 | 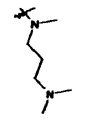 | 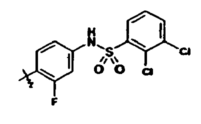 |
| 64 | 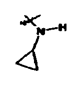 | 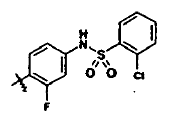 |
| 65 | 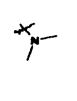 | 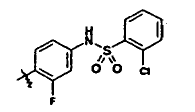 |
| 66 | 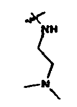 | 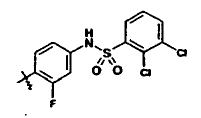 |
| 67 | 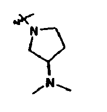 | 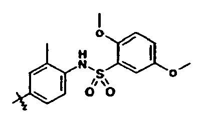 |
| 68 | 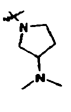 | 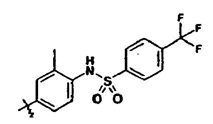 |
| 69 | 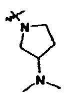 | 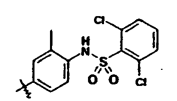 |
| 70 | 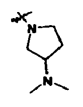 | 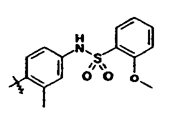 |
| 71 | 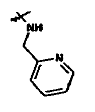 | 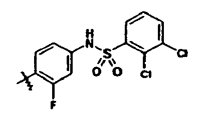 |
or a pharmaceutically acceptable salt thereof.

10. A process for the manufacture of a compound of the formula I according to any one of claims 1 to 9, comprising reacting
a) a compound of the formula II, 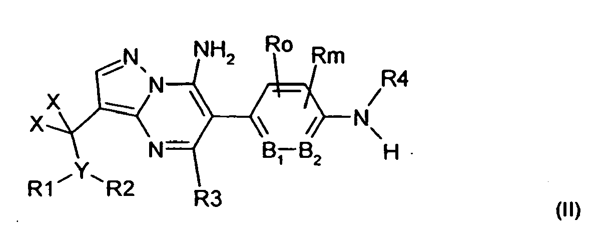 wherein R1, R2, X, R3, B₁, B₂, Ro, Rm and R4 are as defined for a compound of the formula I, with an acid of the formula III,
R5-OH (III)
wherein R5 is as defined for a compound of the formula I, or a reactive derivative thereof capable of introducing a moiety R5 as defined for a compound of the formula I, or
b) for the manufacture of a compound of the formula I wherein the two symbols X together are oxo (=O) and the remaining symbols are as defined for a compound of the formula I, an acid of the formula IV, 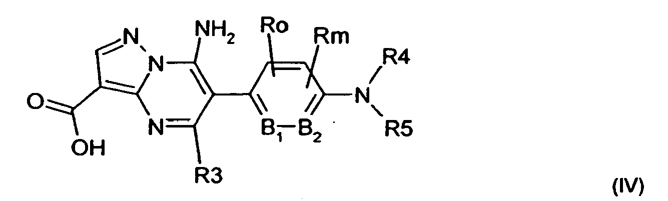 wherein R2, Ro, Rm, B₁, B₂, R4 and R5 are as defined for a compound of the formula I, or a reactive derivative thereof, with a compound of the formula V, 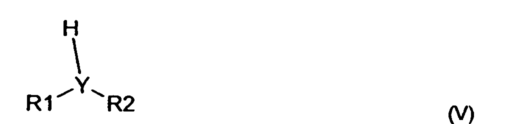 wherein R1, R2 and Y are as defined for a compound of the formula I, or
c) for the manufacture of a compound of the formula I wherein each X is hydrogen and the other symbols are as defined for a compound of the formula I, a compound of the formula VI, 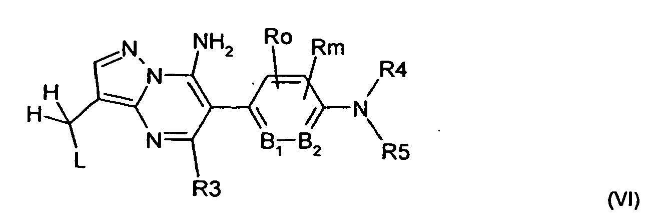
wherein L is a leaving group and R3, Ro, Rm, R4, R5, B₁ and B₂ are as defined for a compound of the formula I, with a compound of the formula V as defined under b);
and, if desired, transforming a compound of formula I into a different compound of formula I, transforming a salt of an obtainable compound of formula I into the free compound or a different salt, transforming an obtainable free compound of formula I into a salt thereof, and/or separating an obtainable mixture of isomers of a compound of formula I into individual isomers.

11. A compound of the formula XIII, 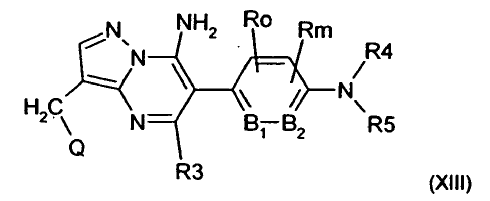 wherein Q is hydroxyl or esterified or etherified hydroxyl and R3, R4, R5, B₁, B₂, R0 and Rm are as defined in any one of claims 1 to 9, or a pharmaceutically acceptable salt thereof.

12. A compound of the formula XIII according to claim 11 wherein Q is hydroxyl, C₁-C₇ alkoxy or C₁-C₇ alkoxy-C₁-C₇ alkoxy and R3, R4, R5, B₁, B₂, R0 and Rm are as defined in any one of claims 1 to 9, or a pharmaceutically acceptable salt thereof.

13. A compound of the formula XIII according to any one of claims 11 or 12, selected from the group of compounds with the following names:
N-[4-(7-amino-3-hydroxymethyl-pyrazolo[1,5-a]pynmidin-6-yl)-3-chlorophenyl]-benzenesulfonamide;
N-[4-(7-amino-3-hydroxymethyl-pyrazolo[1,5-a]pyrimidin-6-yl)-3-fluorophenyl]-2-chloro-benzenesulfonamide;
N-[4-(7-amino-3-hydroxymethyl-pyrazolo[1,5-a]pyrimidin-6-yl)-3-fluorophenyl]-2,3-dichloro-benzenesulfonamide;
N-[4-(7-amino-3-methoxymethyl-pyrazolo[1, 5-a]pyrimidin-6-yl)-3-fluorophenyl]-2,3-dichloro-benzenesulfonamide;
N-[4-(7-amino-3-(2-methoxyethoxymethyl)-pyrazolo[1,5-a]pyrimidin-6-yl)-3-fluorophenyl]-2,3-dichloro-benzenesulfonamide; and
N-[4-(7-amino-3-hydroxymethyl-pyrazolo[1,5-a]pyrimidin-6-yl)-phenyl]-2,3-dichloro-benzenesulfonamide;
or a pharmaceutically acceptable salt thereof.

14. A compound of the formula XII. 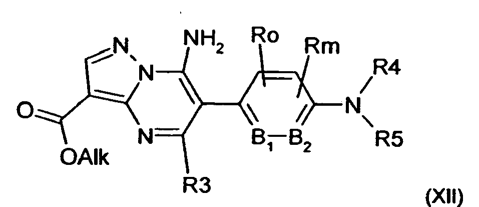 wherein Alk is unsubstituted or substituted C₁-C₇ alkyl, preferably C₁-C₇ alkyl, and
R3, R4, R5, B₁, B₂, Ro and Rm are as defined in any one of claims 1 to 9.

15. A compound of the formula XII according to claim 14 selected from the group of compounds with the names
7-amino-6-[2-chloro-benzene sulfonylamino)-phenyl]-pyrazolo[1,5-a]pyrimidine-3-carboxylic acid ethyl ester; and
7-amino-6-[2,3-dichloro-benzene sulfonylamino)-3-fluoro-phenyl]-pyrazolo[1,5-a]pyrimidine-3-carboxylic acid ethyl ester,
or a pharmaceutically acceptable salt thereof.

16. The use of a compound of the formula I, or a pharmaceutically acceptable salt thereof, according to any one of claims 1 to 9, a compound of the formula XIII, or a pharmaceutically acceptable salt therof, according to any one of claims claim 11 to 13 or a compound of the formula XII, or a Pharmaceutical acceptable salt thereof, according to any one of claims claim 14 or 15, for the manufacture of a pharmaceutical composition for the treatment of a disease that depends on activity of a protein kinase, especially Tie-2 kinase.

17. A pharmaceutical formulation, comprising a compound of the formula I, or a pharmaceutically acceptable salt thereof, according to any one of claims 1 to 9, a compound of the formula XIII, or a pharmaceutically acceptable salt therof, according to any one of claims claim 11 to 13 or a compound of the formula XII, or a pharmaceutically acceptable salt thereof, according to any one of claims claim 14 or 15, and at least one pharmaceutically acceptable carrier material.

18. A compound of the formula I, or a pharmaceutical acceptable salt thereof, according to any one of claims 1 to 9, a compound of the formula XIII, or a pharmaceutically acceptable salt therof, according to any one of claims claim 11 to 13 or a compound of the formula XII, or a pharmaceutically acceptable salt thereof, according to any one of claims claim 14 or 15, for use in the diagnostic or therapeutic treatment of an animal or human body, especially for treatment of a kinase dependent disease, preferably a disease that depends on Tie-2.

## Patentansprüche

1. Verbindungen der Formel I 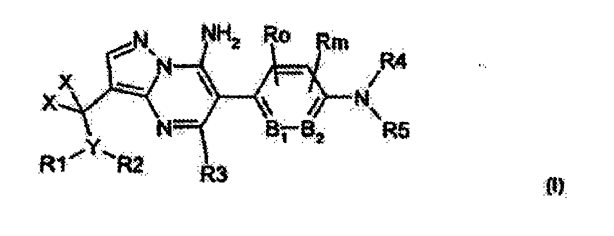 wobei
entweder
R1 und R2 jeweils unabhängig voneinander für unsubstituiertes oder substituiertes C₁-C₇-Alkyl , unsubstituiertes oder substituiertes C₃-C₁₀-Cycloalky, unsubstituiertes oder substituiertes C₆-C₁₄-Aryl oder unsubstituiertes oder substituiertes Heterocyclyl mit 3 bis 14 Ringatomen stehen und Y für N steht,
oder R1, Y und R2 zusammen unsubstituiertes oder substituiertes Heterocyclyl mit 3 bis 14 Ringatomen und wenigstens einem Stickstoffheteroatom bilden, das über einen Ringsstickstoff gebunden ist;
die beiden X jeweils für Wasserstoff stehen oder beide zusammen Oxo oder Thioxo bilden;
R3 für Wasserstoff oder C₁-C₇-Alkyl steht;
R4 für Wasserstoff oder ünsubstituiertes oder substituiertes C₁-C₇-Alkyl steht;
R5 für Acyl steht;
B₁ für N oder CRo steht,
B₂ für N oder CRm steht,
und Ro und Rm jeweils unabhängig voneinander für Wasserstoff, C₁-C₇-Alkyl, Halogen oder C₁-C₇-Alkoxy stehen:
und deren Salze.

2. Verbindungen der Formel I nach Anspruch 1, wobei
R5 für unsubstituiertes oder substituiertes C₆-C₁₄-Arylaminocarbonyl, das besonders bevorzugt ist, unsubstituiertes oder substituiertes Heterocyclylaminocarbonyl, das besonders bevorzugt ist und wobei Heterocyclyl 3 bis 14 Ringatome aufweist, unsubstituiertes oder substituiertes C₆-C₁₄-Arylaminosulfonyl, unsubstituiertes oder substituiertes Heterocyclylaminosulfonyl, wobei Hetorocyclyl 3 bis 14 Ringatome aufweist, unsubstituiertes oder substituiertes Niederalkansulfonyl, welches besonders bevorzugt ist, unsubstituiertes oder substituiertes C₆-C₁₄-Arylsulfonyl, welches besonders bevorzugt ist, unsubstituiertes oder substituiertes Heterocyclylsulfonyl, wobei Heterocyclyl 3 bis 14 Ringatome aufweist oder unsubstituiertes oder substituierter C₆-C₁₄-Arylcarbonyl steht,
und die anderen Symbole R1, R2, Y, X, R3, R4, B₁, B₂, Ro und Rm die in Anspruch 1 angegebenen Bedeutungen haben
und deren (vorzugsweise pharmazeutisch unbedenkLiche) Salze.

3. Verbindungen der Formel I nach Anspruch 1, wobei
R5 für Phenylaminocarbonyl, wobei Phenyl unsubstituiert oder durch eine oder mehrere Einheiten unabhängig voneinander ausgewählt aus C₃-C₇-Alkyl, Halogen (ganz besonders bevorzugt), insbesondere Chlor; Halogen-C₁-C₇-alkyl, C₁-C₇-Alkoxy und Cyano, substituiert ist ;
Pyrazolylaminocarbonyl oder Isoxazolylaminocarbonyl, wobei pyrazolyl bzw. Isoxazolyl unsubstituiert oder durch eine: oder zwei Einheiten unabnängig voneinander ausgewählt aus der aus C₁-C₇-Alkyl und Phenyl, welches unsubstituiert oder durch Halogen C₁-C₇-Alkoxy Piperazin-C₁-C₇ alkyl, 4-C₁-C₇-Alkylpiperazin-C₁-C₇-alkyl und Morpholino-C₁-C₇-alkyl substituiert ist, bestehenden Gruppe substituiert ist;
Pyrazolylaminosulfonyl oder Isoxaszolylaminosulfonyl, wobei Pyrazolyl oder isoxazolyl jeweils unsubstituiert oder durch eine oder mehrere Einheiten unabhängig voneinander ausgewählt aus der aus C₁-C₇-Alkyl und Phenyl, welches unsübstitüiert oder durch Halogen, C₁-C₇-Alkoxy, Piperazin-C₁-C₇-alkyl 4-C₁-C₇-alkylpiperazin C₁-C₇-alkyl und Morpholino-C₁-C₇-alkyl substituiert ist, bestehenden Gruppe substituiert ist;
Phenyl-C₁-C₇-alkansulfonyl, wobei Phenyl unsubstituiert (bevorzugt) oder durch eine oder mehrere z.B. bis zu drei, Einheiten unabhängig voneinander ausgewählt aus der aus C₁-C₇-Alkyl, Halogen (besonders bevorzugt), Halogen-C₁-C₇-alkyl, C₁-C₇-Alkoxy und Cyano bestehenden Gruppe substituiert ist;
Phenylsulfonyl, wobei das Phenyl unsubstituiert oder durch eine oder mehrere Einheiten unabhängig voneinander ausgewählt aus der aus C₁-C₇-Alkyl, Halogen (bevorzugt), Halogen-C₁-C₇-alkyl, C₁-C₇-Alkoxy und Cyano bestehenden Gruppe substituiert ist, steht;
und die anderen Symbole R1, R2, X, Y, R3, R4, B₁, B₂, Ro und Rm die in Anspruch 1, angegebenen Bedeutungen haben,
und deren (vorzugsweise pharmazeutisch unbedenkliche) Salze,

4. Verbindungen der Formel I nach Anspruch 1, wobei
R5 für 3-Trifluormethylphenylaminocarbonyl, 4-Fluorphenylaminocarbonyl, 3- oder 2-Chlorphenylaminocarbonyl, 3-tert.-Butyl-1-(4-fluorphenyl)-pyrazol-5-ylaminocarbonyl 3-tert.-Butyl-1-(4-methoxyphenyl)pyrazol-5-ylaminecarbonyl, 3-tert.-Butyl-1-(4-(4-methylpiperazinmethyl)phenyl)-pyrazol-5-ylaminocarbonyl, 3-tert.-Butyl-1-(3-(4-methypiperazinmethyl)phenyl)pyrazol-5-ylamino-Carbonyl, 3-tert.-Butyl-1-(4-(morpholinomethyl)-phenyl)pyrazol-5-ylamincarbonyl, 5-tert.-Butylisoxazol-3-ylaminocarbonyl, 3-tert.-Butyl-1-(4-fluorphenyl)pyrazol-5-ylaminosulfonyl, Phenylmethylsulfonyl oder 2-Phenylethylsulfonyl, 2,3-Dimethylphenylsulfonyl, 2-, 3- oder 4-Methylphenylsulfonyl, 2-, 3- oder 4-Methoxyphenylsulfonyl, 2-Methyl-4,5-dimethoxyphenylsulfonyl, 2,5-Dimethoxyphenylsulfonyl, 2-, 3- oder 4-Trifluormethylphenylsulfonyl, 2-Chlor-5-trifluormethylphenylsulfonyl, 2-Chlor-4-trifluormethylphenylsulfonyl oder insbesondere 2-, 3- oder 4-chlorphenylsulfonyl 2,3-, 2,4-, 2,5 - 3,5- oder 2,6-Dichlorphenylsulfonyl, 2-Chlor-4-cyanophenylsulfonyl oder 4-Fluor-2-chlorphenylsulfonyl steht;
und die anderen Symbole R1, R2, X, Y, R3, R4, B₁, B₂, Ro und Rm die in Anspruch 1 angegebenen Bedeutungen haben,
und deren (vorzugsweise pharmazeutisch unbedenkliche) Salze.

5. Verbindungen der Formel I nach einem der Ansprüche 1 bis 4, wobei
R1R2Y zusammen genommen für C₁-C₇-Alkylamino, Di-(C₁-C₇-alkyl) amino unsubstituiertes Amino-C₁-C₇-alkylamino, N-mono-, N,N-Di- oder N,N,N'-Tri-(C₁-C₇-alkyl) amino-C₁-C₇ alkylamino, C₁-C₇-Alkoxy-C₁-C₇-alkylamino, pyrolidinyl-C₁-C₇-alkylamino, Oxopyrrolidinyl-C₁-C₇-alkylamino, Piperidinyl- C₁-C₇-alkylamino, (N-C₁-C₇-Alkylpiperidinyl)-C₁-C₇-alkylamino, Pyridyl-C₁-C₇-alkylamino, C₃-C₆-cycloalkylamino, Piperidinylamino, N-C₁-C₇-Alkylpiperidinylamino, Pyrrolidino, Amino-, N-C₁-C₇-Alkylamino- oder N,N-Di-C₁-C₇-alkylamino-pyrrolidino, Amino-, N-C₁-C₇-Alkylamino- oder N,N-Di-C₁-C₇-alkylaminopiperidino, Piperazin, N-C₁-C₇-Alkylpiperazin, N-C₁-C₇-Alkanoylpiperazin, N-C₁-C₇-Alkansulfonylpiperazin, Morpholino, Thiomorpholino oder S,S-Dioxothiomorpholino steht;
R₅ wie in einein der Ansprüche 1 bis 4 definiert ist;
und die anderen Symbole R3, R4, X, B₁, B₂, Ro und Rm die in Anspruch 1 angegebenen Bedeutungen haben,
und deren (vorzugsweise pharmazeutisch unbedenkliche) Salze.

6. Verbindungen der Formel I nach einem der Ansprüche 1 bis 5, wobei
R1, R2 und Y wie in Anspruch 1 oder 5 definiert sind,
R5 wie ein Anspruch 1 oder 4 definiert ist,
R3 für Wasserstoff oder Methyl steht,
R4 für Wasserstoff steht,
X jeweils für Wasserstoff steht,
B₁ für N oder CRo steht,
B₂ für CRm steht,
und Ro und Rm jeweils unabhängig voneinander für Wasserstoff, Methyl, Fluor, Chlor oder Methoxy, vorzugsweise Chlor, stehen,
und deren pharmazeutisch unbedenkliche Salze.

7. Verbindungen der Formel I nach einem der Ansprüche 1 bis 5, wobei
R1, R2 und Y wie in Anspruch 1 oder 5 definiert sind,
R5 wie in Anspruch 1 oder 4 definiert ist,
R3 für Wasserstoff oder Ethyl steht,
R4 für Wasserstoff steht,
die beiden X zusammen Oxo bilden,
B₁ für N oder CRO steht,
B₂ für CRm steht,
und Ro und Rm jeweils unabhängig voneinander für Wasserstoff, Methyl, Fluor, Chlor oder Methoxy, vorzugsweise Chlor, stehen,
und deren pharmazeutisch unbedenkliche Salze.

8. Verbindungen der Formel I nach Anspruch 1, ausgewählt aus der Gruppe von Verbindung mit den Namen
N-(4-7-Amino-3-(3-dimethylaminopyrrolidin-1-carbonyl)pyrazol [1,5-a]pyrimidin-6-yl]phenyl)-2,3-dichlorbenzolsulfonamid
1-(4-[7-Amino-3-(3-dimethylaminopyrrollidin-1-carbonyl)pyrazol[1,5-a]pyrimidin-6-yl]-3-methylphenyl)-3-(2-chlorphenyl)harnstoff
N-[4-(7-Amino-3-{[(3-dimethylaminopropyl)methylamino]methyl}pyrazol[1,5-a]pyrimidin-6-yl)-3-chlorphenyl]-2-chlorbenzolsulfonamid und
N-[4-(7-Amino-3-{[(3-dimethylaminopropyl)methylamino]methyl]pyrazol [1,5-a]pyrimidin-6-yl)-3-chlorphenyl]-2-chlorbenzosulfonamid und
N-[4-(7-Amino-3-dimethylaminoethylpyrazol [1,5-a]pyrimidin-6-yl)phenyl]-2,3-dichlorbenzolsulfonamid;
und deren pharmazeutisch unbedenkliche Salze.

9. Verbindungen der Formel I nach Anspruch 1, ausgewählt aus der in der folgenden Tabelle wiedergegebenen Gruppe von Verbindungen: 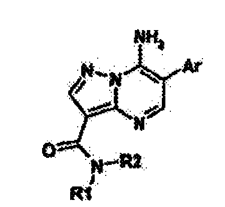
| Verbindung | 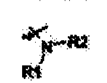 | 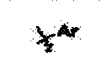 |
|---|---|---|
| **3** | 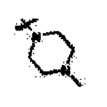 | 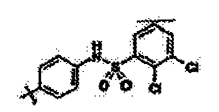 |
| **4** | 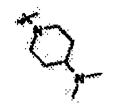 | 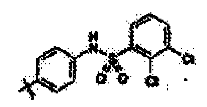 |
| **5** | 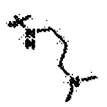 | 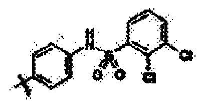 |
| **6** | 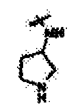 | 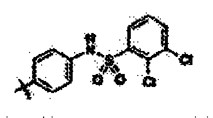 |
| **7** | 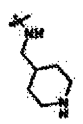 | 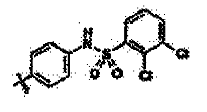 |
| **8** | 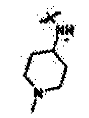 | 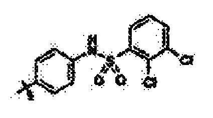 |
| **9** | 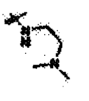 | 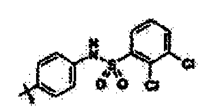 |
| **10** | 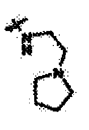 | 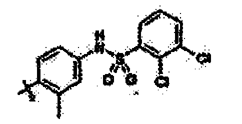 |
| **11** | 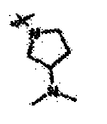 | 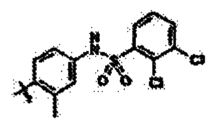 |
| **12** | 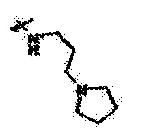 | 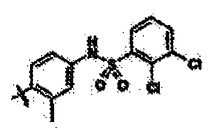 |
| **13** | 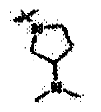 | 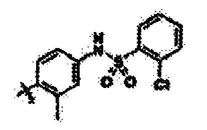 |
| **14** | 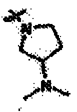 | 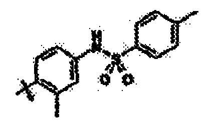 |
| **15** | 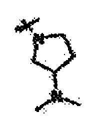 | 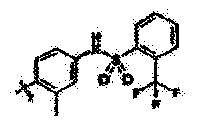 |
| **16** | 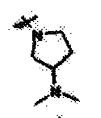 | 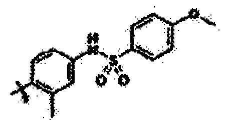 |
| **17** | 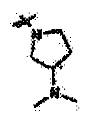 | 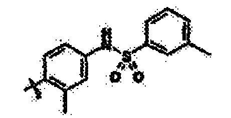 |
| **18** | 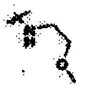 | 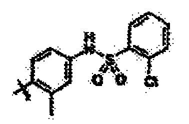 |
| **19** | 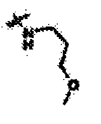 | 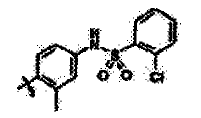 |
| **20** | 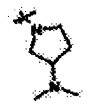 | 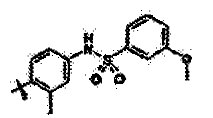 |
| **21** | 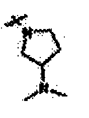 | 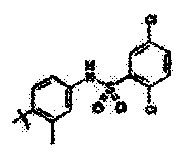 |
| **22** | 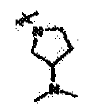 | 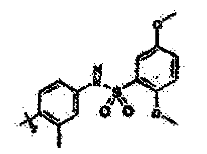 |
| **23** | 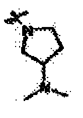 | 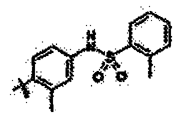 |
| **24** | 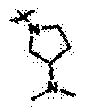 | 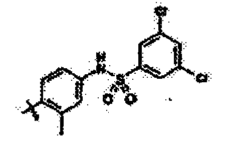 |
| **25** | 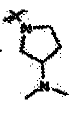 | 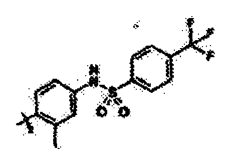 |
| **26** | 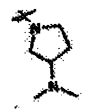 | 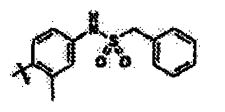 |
| **27** | 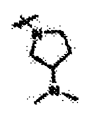 | 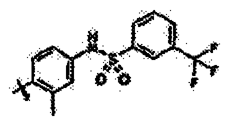 |
| **28** | 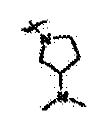 | 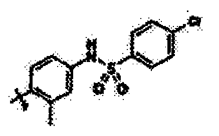 |
| **29** | 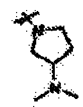 | 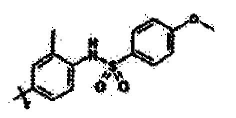 |
| **30** | 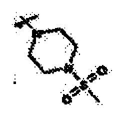 | 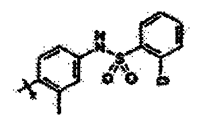 |
| **31** | 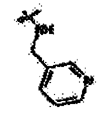 | 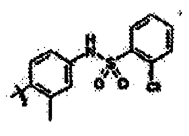 |
| **32** | 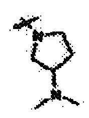 | 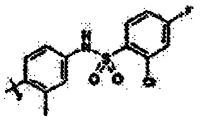 |
| **33** | 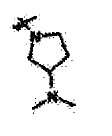 | 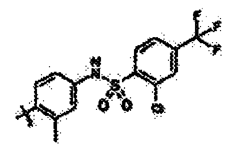 |
| **34** | 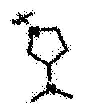 | 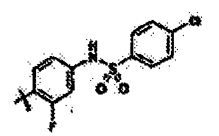 |
| **35** | 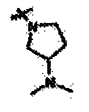 | 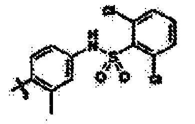 |
| **36** | 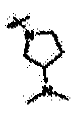 | 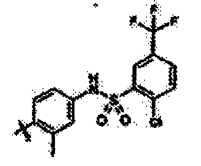 |
| **37** | 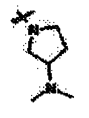 | 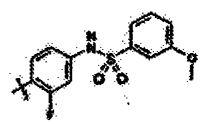 |
| **38** | 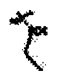 | 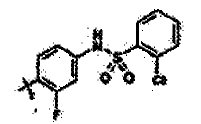 |
| **39** | 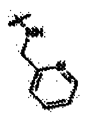 | 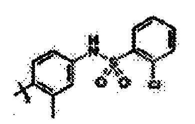 |
| **40** | 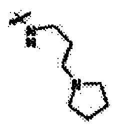 | 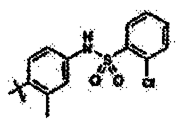 |
| **41** | 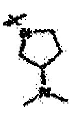 | 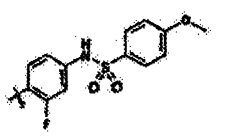 |
| **42** | 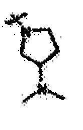 | 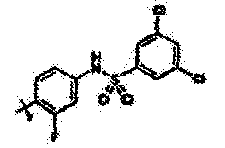 |
| **43** | 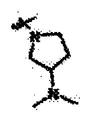 | 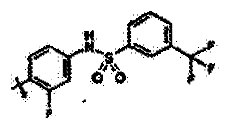 |
| **44** | 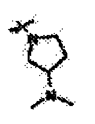 | 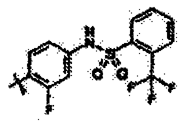 |
| **45** | 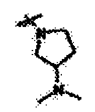 | 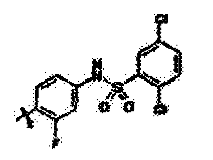 |
| **46** | 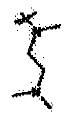 | 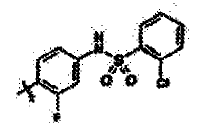 |
| **47** | 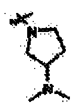 | 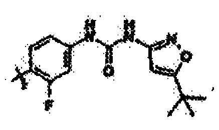 |
| **48** | 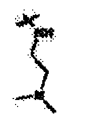 | 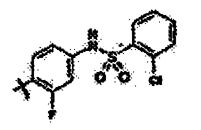 |
| **49** | 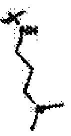 | 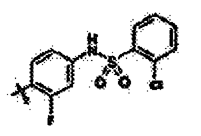 |
| **50** | 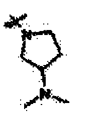 | 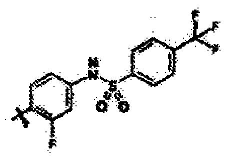 |
| **51** | 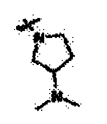 | 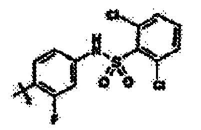 |
| **52** | 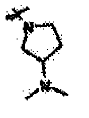 | 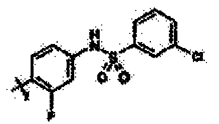 |
| **53** | 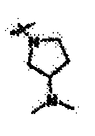 | 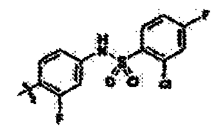 |
| **54** | 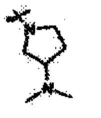 | 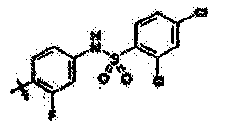 |
| **55** | 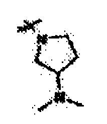 | 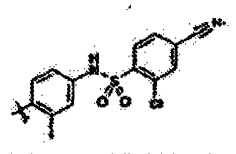 |
| **56** | 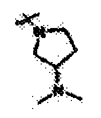 | 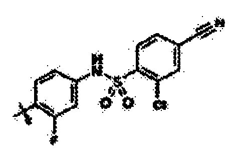 |
| **57** | 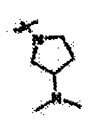 | 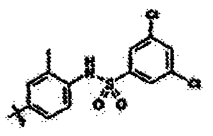 |
| **58** | 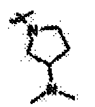 | 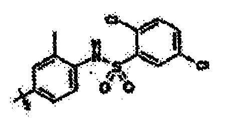 |
| **59** | 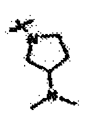 | 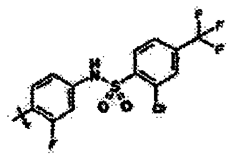 |
| **60** | 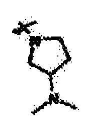 | 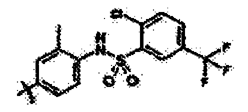 |
| **61** | 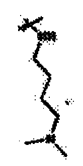 | 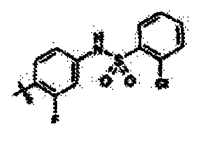 |
| **62** | 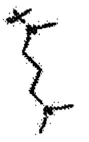 | 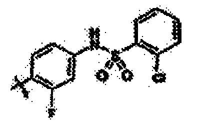 |
| **63** | 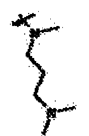 | 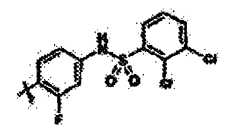 |
| **64** | 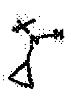 | 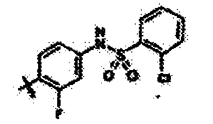 |
| **65** | 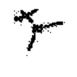 | 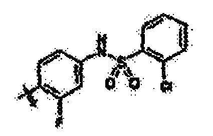 |
| **66** | 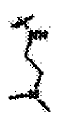 | 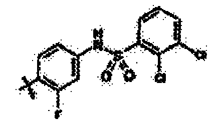 |
| **67** | 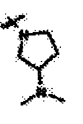 | 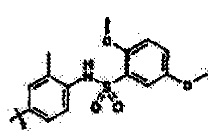 |
| **68** | 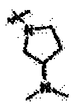 | 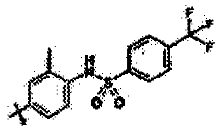 |
| **69** | 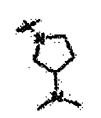 | 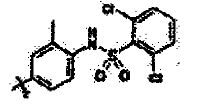 |
| **70** | 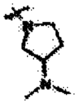 | 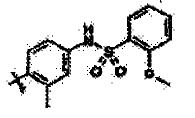 |
| **71** | 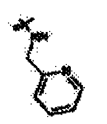 | 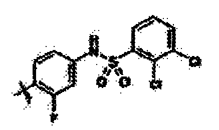 |
und deren pharmazeutisch unbedenkliche Salze.

10. Verfahren zur Herstellung einer Verbindung der Formel I nach einem der Ansprüche 1 bis 9, bei dem man
a) eine Verbindung der Formel II 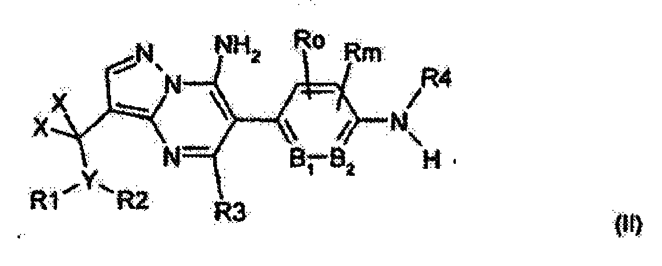 wobei R1, R2, X, R3, B₁, B₂, Ro, Rm und R4 wie für eine Verbindung der Formel I definiert sind, mit einer Säure der Formel III
R₅-OH (III)
wobei R5 wie für eine Verbindung der Formel I definiert ist, oder einem reaktiven Derivat davon, das in der Lage ist, eine wie für eine Verbindung der Formel I definierte Einheit R5 einzuführen, umsetzt oder
b) zur Herstellung einer Verbindung der Formel I, wobei die beiden Symbole X zusammen für Oxo (=O) stehen und die restlichen Symbole wie für eine Verbindung der Formel I definiert sind, eine Säure der Formel IV 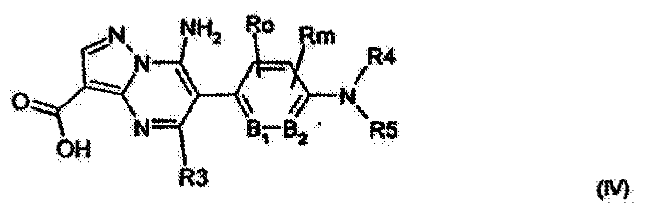 wobei R2, Ro, Rm, B₁, B₂, R4 und R5 wie für eine Verbindung der Formel I definiert sind, oder ein reaktives Derivat davon, mit einer Verbindung der Formel V 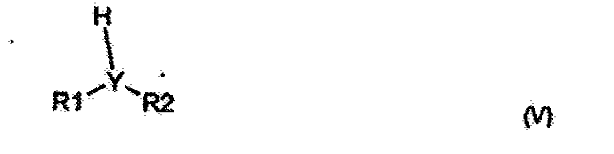 wobei R1, R2 und Y wie für eine Verbindung der Formel I definiert sind, umsetzt oder
c) zur Herstellung einer Verbindung der Formel I, wobei X jeweils für Wasserstoff steht und die anderen Symbole wie für eine Verbindung der Formel I definiert sind, eine Verbindung der Formel VI 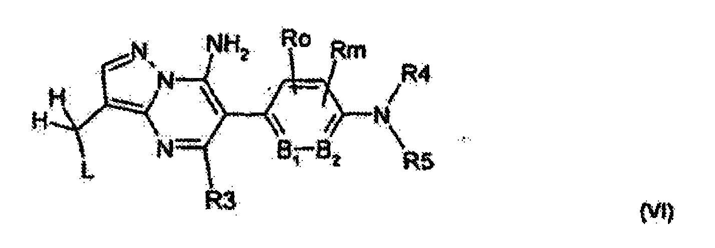 wobei für eine Abgangsgruppe steht und R3, Ro, Rm, R4, R5, B₁ und B₂ wie für eine Verbindung der Formel I definiert sind, mit einer wie unter b) definierten Verbindung der Formel V umsetzt;
und, falls gewünscht, eine Verbindung der Formel I in eine andere Verbindung der Formel I umwandelt, ein Salz einer erhältlichen Verbindung der Formel I in die freie Verbindung oder ein anderes Salz umwandelt, eine erhältliche freie Verbindung der Formel I in ein Salz davon umwandelt und/oder eine erhältliche Mischung von Isomeren einer Verbindung der Formel I in die einzelnen Isomere auftrennt.

11. Verbindungen der Formel XIII 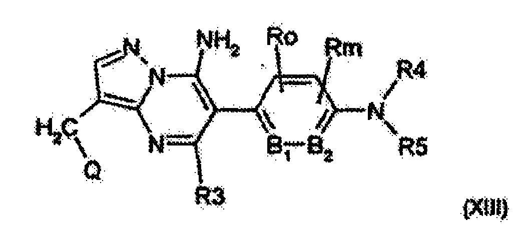 wobei Q für Hydroxy oder verestertes oder verethertes Hydroxy steht und R3, R4, R5, B₁, B₂,
Ro und Rm wie in einem der Ansprüche 1 bis 9 definiert sind, und deren pharmazeutisch unbedenkliche Salze.

12. Verbindungen der Formel XIII nach Anspruch 11, wobei Q für Hydroxy, C₁-C₇-Alkoxy oder C₁-C₇-Alkoxy-C₁-C₇-alkoxy steht und R3, R4, R5, B₁, B₂, Ro und Rm wie in einem der Ansprüche 1 bis 9 definiert sind, und deren pharmazeutisch unbedenkliche Salze.

13. Verbindungen der XIII nach Anspruch 11 oder 12, ausgewählt aus der Gruppe von Verbindungen mit den folgenden Namen:
N-[4-(7-Amino-3-hydroxymethylpyrazol [1,5-a]pyrimidin-6-yl)-3-chlorphenyl]benzosulfonamid
N-[4-(7-Amino-3-hydroxymethylpyrazol[1,5-a] pyrimidin-6-yl)-3-fluorphenyl]-2-chlorbenzosulfonamid
N-[4-(7-Amino-3-hydroxymethylpyrazol[1,5-a]pyrimidin-6-yl)-3-fluorphenyl]-2,3-dichlorbenzolsulfonamid
N-[4-(7-Amino-3-methoxymethylpyrazol[1,5-a]pyrimidin-6-yl)-3-fluorphenyl]-2,3-dchlorbenzolsulfonamid
N-[4-(7-Amino-3-(2-methoxyethoxymethyl)pyrazol[1,5-a]pyrimidin-6-yl)-3-fluorphenyl]-2,3-diclorbenzolsulfonamid und
N-[4-(7-Amino-3-hydroxymethylpyrazol[1,5-a]pyrimidin-6-yl)phenyl]-2,3-dichlorbenzolsulfonamid
und deren pharmazeutisch unbedenkliche Salze.

14. Verbindungen der Formel XII 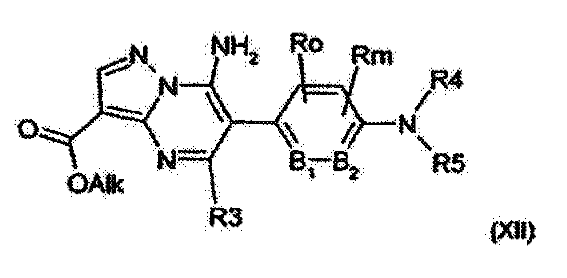 wobei Alk für unsubstituiertes oder substituiertes C₁-C₇-Alkyl, vorzugsweise C₁-C₇-Alkyl, steht, und R3, R4, R5, B₁, B₂, Ro und Rm wie in einem der Ansprüche 1 bis 9 definiert sind.

15. Verbindungen der Formel XII nach Anspruch 14, ausgewählt aus der Gruppe von Verbindungen mit den Namen
7-Amino-6-[2-chlorbenzosulfonylamino)phenyl]-pyrazol[1,5-a]pyrimidin-3-carbonsäureethylester und
7-Amino-6-[2,3-dichlorbenzolsulfonylamino)-3-fluorphenyl]pyrazol[1,5-a]pyrimidin-3-carbonsäureethylester;
und deren pharmazeutische unbedenkliche Salze.

16. Verwendung einer Verbindung der Formel I oder eines pharmazeutisch unbedenklichen Salzes davon nach einem der Ansprüche 1 bis 9, einer Verbindung der Formel XIII oder eines pharmazeutisch unbedenklichen Salzes davon nach einem der Ansprüche 11 bis 13 oder einer Verbindung der Formel XII oder eines pharmazeutisch unbedenklichen Salzes davon nach Anspruch 14 oder 15 zur Herstellung einer pharmazeutischen Zusammensetzung zur Behandlung einer Krankheit, die von der Aktivität einer Proteinkinase, insbesondere Tie-2-Kinase, abhängt.

17. Pharmazeutische Formulierung, enthaltend eine Verbindung der Formel I oder ein pharmazeutisch unbedenkliches Salz davon nach einem der Ansprüche 1 bis 9, eine Verbindung der Formel XIII oder ein pharmazeutisch unbedenkliches Salz davon nach einem der Ansprüche 11 bis 13 oder eine Verbindung der Formel XII oder ein pharmazeutisch Unbedenkliches Salz davon nach Anspruch 14 oder 15 und wenigstens ein pharmazeutisch unbedenkliches Tragermaterial.

18. Verbindung der Formel I oder ein pharmazeutisch unbedenkliches Salz davon nach einem der Ansprüche 1 bis 9, Verbindung der Formel XIII oder ein pharmazeutisch unbedenkliches Salz davon nach einem der Ansprüche 11 bis 13 oder Verbindung der Formel XII oder ein pharmazeutisch unbedenkliches Salz davon nach Anspruch 14 oder 15 zur Verwendung bei der diagnostischen oder therapeutischen Behandlung eines tierischen oder menschlichen Körpers, insbesondere zur Behandlung einer kinaseabhängigen Krankheit, vorzugsweise einer Krankheit, die von Tie-2 abhängt.

## Revendications

1. Composé de formule I, 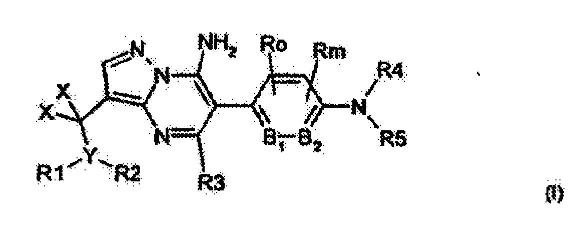 dans laquelle
soit
chacun de R1 et R2 est, indépendamment de l'autre, alkyle en C₁-C₂ non substitué ou substitué, C₃-C₁₀-cycloalkyle non substitué ou substitué, C₆-C₁₄-alkyle non substitué ou substitué ou hétérocyclyle non substitué ou substitué ayant 3 à 14 atomes du cycle et Y est N,
soit R1, Y et R2 ensemble forment un hétérocyclyle non substitué ou substitué ayant 3 à 14 atomes du cycle et au moins un hétéroatome d'azote qui est lié par l'intermédiaire d'un azote du cycle ;
chacun des deux X représente hydrogène ou les deux ensemble forment oxo ou thioxo ;
R3 est hydrogène ou alkyle en C₁-C₇;
R₄ est hydrogène ou alkyle en C₁-C₇ non substitué ou substitué ;
R5 est acyle;
B₁ est N ou CRo,
B₂ est N ou CRm,
et chacun de Ro et Rm, indépendamment de l'autre, est hydrogène, alkyle en C₁-C₇, halogène ou alcoxy en C₁-C₇;
ou un sel de celui-ci.

2. Composé de formule I selon la revendication 1, **caractérisé en ce que**
R5 est C₆-C₁₄-arylaminocarbonyle non substitué ou substitué qui est notamment préféré, hétérocyclylaminocarbonyle non substitué ou substitué qui est notamment préfère et où hétérocyclyle a 3 à 14 atomes du cycle, C₆-C₁₄-arylaminosulfonyle non substitué
ou substitué, hétérocyclylaminosulfonyle non substitué
ou substitué, où hétérocyclyle a 3 à 14 atomes du cycle, (alcane inférieur) sulfonyle non substitué ou substitué qui est notamment préféré, C₆-C₁₄-arylsulfonyle non substitué ou substitué qui est notamment préféré, hétérocyclylsulfonyle non substitué
ou subtitué, ou hétérocyclyle a 3 à 14 atomes du cycle, ou C₆-C₁₄-arylcarbonyle non substitué ou substitué,
et les autres symboles R1, R2, Y, X, R3, R4, B₁, B₂, Ro et Rm ont les significations données dans la revendication 1,
ou un sel (de préférence pharmaceutiquement acceptable) de celui-ci.

3. Composé de formule I selon la revendication 1, **caractérisé en ce que**
R5 est phénylaminocarbonyle où phényle est non substitué ou substitué par un ou plusieurs motifs choisis indépendamment parmi alkyle en C₃-C₇, halogéno (très préféré), notamment chloro; halogénoalkyle en C₃-C₇, alcoxy en C₁-C₇ et cyano;
pyrazolyl-aminocarbonyle ou isoxazolylaminocarbonyle où pyrazolyle ou isoxazolyle est non substitué ou substitué par un ou deux motifs choisis indépendament dans le groupe constitué d'alkyle en C₁-C₇ et phényle qui est non substitué ou substitué par halogéno, alcoxy en C₁-C₇, pipérazino-alkyle en C₁-C₇, 4-(alkyl en C₁-C₇) pipérazino-alkyle en C₁-C₇ et morpholino-alkyl en C₁-C₇; pyrazolyl-aminosulfonyle ou isoxasolylaminosulfonyle, où chaque pyrazolyle ou isoxazolyle est non substitué ou substitué par un ou deux motifs choisis indépendamment dans le groupe constitué d'alkyle en C₁-C₇ et de phényle qui est non substitué ou substitué par halogéno, alcoxy en C₁-C₇, piperazino-alkyle en C₁-C₇, 4-(alkyl en C₁-C₂) pipérazino-alkyle en C₁-C₇ et morpholino-alkyle en C₁-C₇; phényl-(alcane en C₁-C₇)sulfonyle, où phényle est non substitué (préféré) ou substitué par un ou plusieurs, par exemple jusqu'à trois, motifs choisis indépendamment dans le groupe constitué d'alkyle en C₁-C₇, halogèno (notamment préféré), halogéno-alkyle en C₁-C₇, alcoxy en C₁-C₇ et cyano ;
phénylsulfonyle où le phényle est non substitué ou substitué par un ou plusieurs motifs choisis indépendamment dans le groupe constitué d'alkyle en C₁-C₇, halogéno (préféré), halogéno-alkyle en C₁-C₇, alcoxy en C₁-C₇ et cyano ;
et les autres symboles R1, R2, X, Y, R3, R4, B₁ B₂, Ro et Rm ont les significations données dans la revendication 1,
ou un sel (de préférence pharmaceutiquement acceptable) de celui-ci.

4. Composé de formule I selon la revendication 1, **caractérisé en ce que**
R5 est 3-trifluorométhyl-phénylaminocarbonyle, fluorophénylaminocarbonyle, 3- ou 2-chlorophenylaminocarbonyle, 3-tert-butyl-1-(4-fluorophényl)-pyrazol-5-ylaminocarbonyle, 3-tert-butyl-1-(4-méthoxyphényl)-pyrazol-5-ylaminocarbonyle, 3-tert-butyl-1-(4-(4-méthyl-pipérazinométhyl)-phényl)-pyrazol-5-ylaminocarbonyle, 3-tert-butyl-1-(3-(4-methylpipérazinométhyl)-phényl)-pyrazol-5-ylaminocarbonyle, 3-tert-butyl-1-(4-(morpholinométhyl)-phényl)-pyrazol-5-ylaminocarbonyle, 5-tert-butyl-isoxazol-3-ylaminocarbonyle, 3-tert-butyl-1-(4-fluorophéhyl)-pyrazol-5-ylaminosulfonyle, phénylmethylsulfonyle ou 2-phényléthylsulfonyle, 2,3-dimethylphénylsulfonyle,2-, 3- ou 4-méthylphénylsulfonyle, 2-, 3- ou 4-méthoxyphénylsulfonyle, 2 -méthyl-4,5-diméthoxyphénylsulfonyle, 2,5-diméthoxyphénylsulfonyle, 2-, 3- ou 4-trifluorométhylphénylsulfonyle,2-chloro-5-trifluorométhylphénylsulfonyle, 2-chloro-4-trifluororméthyl-phénylsulfonyle, ou notamment 2,- 3- ou 4-chlorophénylsulfonyle, 2,3-, 2,4-, 2,5-, 3,5- ou 2,6-dichiprophenylsulfonyle, 2-chloro-4-cyanophénylsulfonyle ou 4-fludro-2-chlorophénylsulfonyle ; et les autres symboles R1, R2, X, Y, R3,R4, B₁, B₂, Ro et Rm ont les significations données dans la revendication 1,
ou un sel (de préférence pharmaceutiquement acceptable} de celui-ci,

5. Composé de formule I selon l'une quelconque des revendications 1 a 4, **caractérisé en ce que**
R1R2Y -pris ensemble - est alkylamino en C₁-C₇,di-(alkyl en G₁-C₇)-amino, amino-(alkyl en C₁-C₇)amino non substitué, N-mono- , N,N-di- ou N,N,N'-tri-(alkyl inférieur)-amino-(alkyl en C₁-C₇)-amino, (alcoxy en C₁-C₇)-(alkyl en C₁-C₇)amino,pyrrolidinyl-(alkyl en C₁-C₇)amino, oxopyrrolidinyl-(alkyl en C₁-C₇)amino, pipéridinyl-(alkyl en C₁-C₇)amino, (N-(alkyl en C₁-C₇)pipéridinyl)- (alkyl en C₁-C₇)amino,pyridyl-(alkyl en C₁-C₇) -amino, C₃-C₆-cycloalkylamino, pipéridinylamino, N-(alkyl en C₁-C₇)pipéridinylamino, pyrrolidino, amino-, N-(alkyl en C₁-C₇)amino- ou N,N-di-(alkyl en C₁-C₇)amino-pyrrolidino, amino-, N-(alkyl en C₁-C₇)amino- ou N,N-di-(alkyl en C₁-C₇amino-pipéridino, pipérazino, N-(alkyl en C₁-C₇)pipérazino, N-(alcanoyl en C₁-C₇)pipérazino, N-(alcane en C₁-C₇)sulfonyl-pipérazino, morpholin,thiomorpholino ou S, *S*-dioxathiomorpholino ;
R5 est tel que défini dans l'une quelconque des revendications 1 à 4;
et les autres symboles R3, R4, X, B₁, B₂,Ro et Rm ont les significations données dans la revendication 1,
ou un sel (de préférence pharmaceutiquement acceptable) de celui-ci.

6. Composé de formule I selon l'une quelconque des revendications 1 à 5, **caractérisé en ce que**
R₁, R2 et Y sont tels que définis dans l'une quelconque des revendications 1 ou 5,
R5 est tel que défini dans l'une quelconque des revendications 1 à 4,
R3 est hydrogène ou méthyle,
R4 est hydrogène,
chaque X représente hydrogène,
B₁ est N ou CRo,
B₂ est CRm,
et chacun parmi Ro et Rm, indépendamment de l'autre, est hydrogène, méthyle, fluoro, chloro ou méthoxy, de préférence chloro,
ou un sel pharmaceutiquement acceptable de celui-ci.

7. Composé de formule I selon l'une quelconque des revendications 1 à 5, **caractérisé en ce que**
R1,R2 et Y sont tels que définis dans l'une quelconque des revendications 1 ou 5,
R5 est tel que défini dans l'une quelconque des revendications 1 à 4;
R3 est hydrogène ou méthyle,
R4 est hydrogène,
les deux X ensemble forment oxo,
B₁ est N ou CRo,
B₂ est CRm,
et chacun parmi Ro et Rm, indépendamment de l'autre, est hydrogène, méthyle, fluoro, chloro ou méthoxy, de préférence chloro,
ou un sel pharmaceutiquement acceptable de celui-ci.

8. Composé de formule I selon la revendication 1, choisi dans le groupe de composés portant les noms
N-{4-{7-amino-3-(3-diméthylamino-pyrrolidine-1-carbonyl)-pyrazolo[1,5-a]pyrimidin-6-yl]-phényl}-2,3-dichloro-benzènesulfonamide
1-{4-[7-amino-3-(3-diméthylamino-pyrrolidine-1-carbonyl)-pyrazolo[1,5-a]pyrimidin-6-yl]-3-méthylphényl}-3-(2-chloro-phényl)-urée
N-[4-(7-amino-3-[[(3-dimethylamino-propyl)-méthylamino]-méthyl}-pyrazolo[1,5-a]pyrimidin-6-yl)-3-chlorophényl]-2-chloro-benzènesulfonamide, et
N-[4-(7-amino-3-{[(3-dimethylamino-propyl)-méthylamino]-méthyl}-pyrazolo[1,5-a]pyrimidin-6-yl)-3-chlorophényl]-2-chloro-benzènesulfonamide
N-[4-(7-amino-3-diméthylaminométhyl-pyrazolo[ 1,5-a]pyrimidin-6-yl}-phényl]-2,3-dichlorobenzènesulfonamide;
ou un sel pharmaceutiquement acceptable de ceux-ci.

9. Composé de formule I selon la revendication 1, choisi dans le groupe de composés représentés dans le tableau suivant : 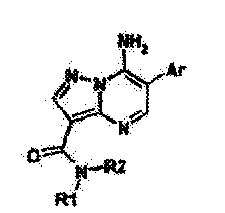
| composé | 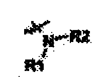 | 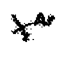 |
|---|---|---|
| 3 | 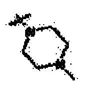 | 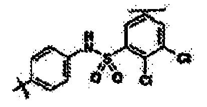 |
| 4 | 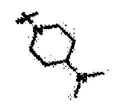 | 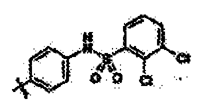 |
| 5 | 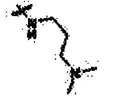 | 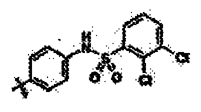 |
| 6 | 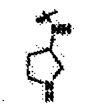 | 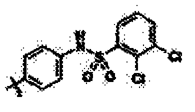 |
| 7 | 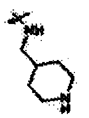 | 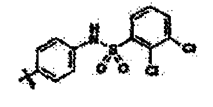 |
| 8 | 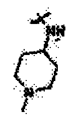 | 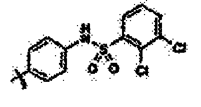 |
| 9 | 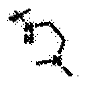 | 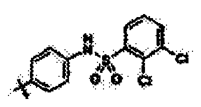 |
| 10 | 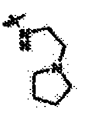 | 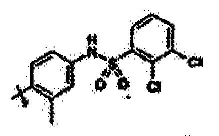 |
| 11 | 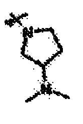 | 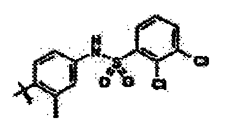 |
| 12 | 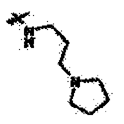 | 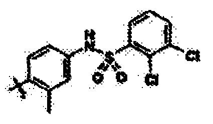 |
| 13 | 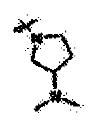 | 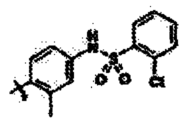 |
| 14 | 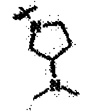 | 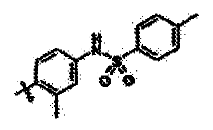 |
| 15 | 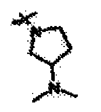 | 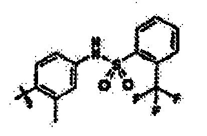 |
| 16 | 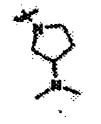 | 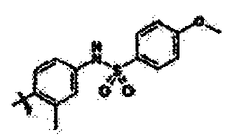 |
| 17 | 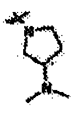 | 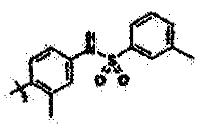 |
| 18 | 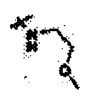 | 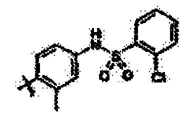 |
| 19 | 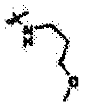 | 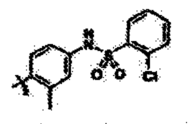 |
| 20 | 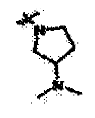 | 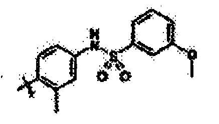 |
| 21 | 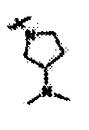 | 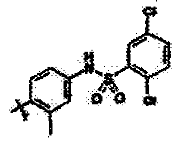 |
| 22 | 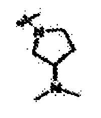 | 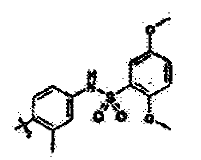 |
| 23 | 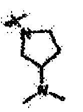 | 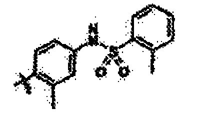 |
| 24 | 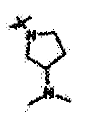 | 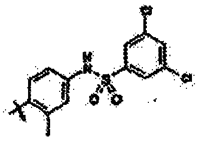 |
| 25 | 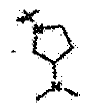 | 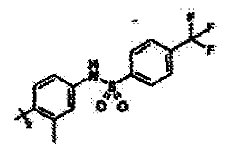 |
| 26 | 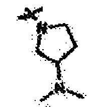 | 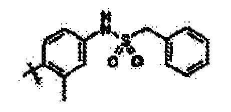 |
| 27 | 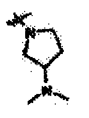 | 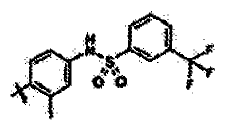 |
| 28 | 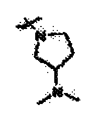 | 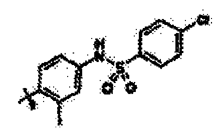 |
| 29 | 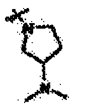 | 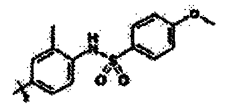 |
| 30 | 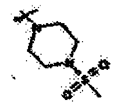 | 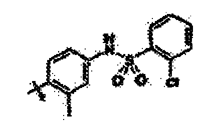 |
| 31 | 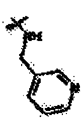 | 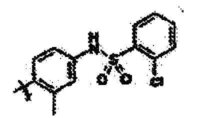 |
| 32 | 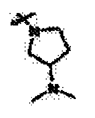 | 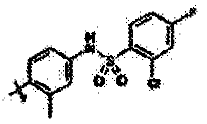 |
| 33 | 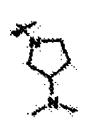 | 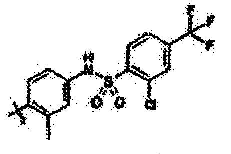 |
| 34 | 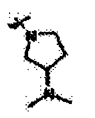 | 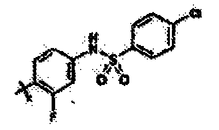 |
| 35 | 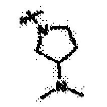 | 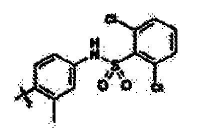 |
| 36 | 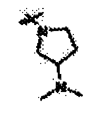 | 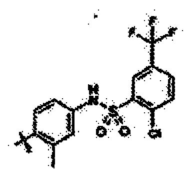 |
| 37 | 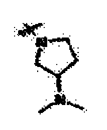 | 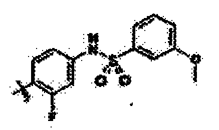 |
| 38 | 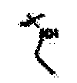 | 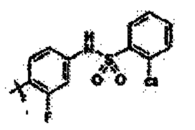 |
| 39 | 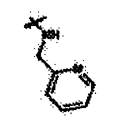 | 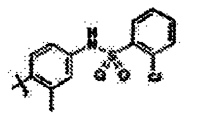 |
| 40 | 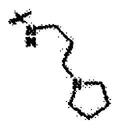 | 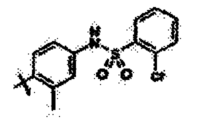 |
| 41 | 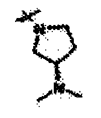 | 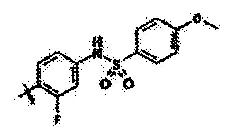 |
| 42 | 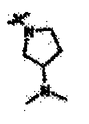 | 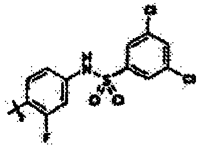 |
| 43 | 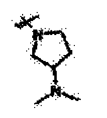 | 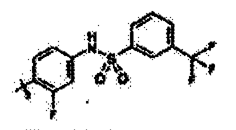 |
| 44 | 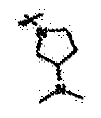 | 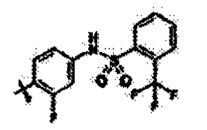 |
| 45 | 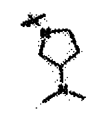 | 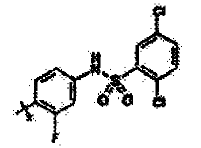 |
| 46 | 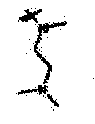 | 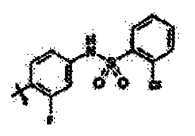 |
| 47 | 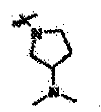 | 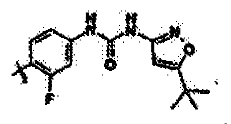 |
| 48 | 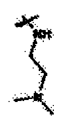 | 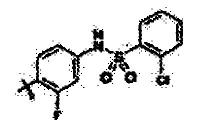 |
| 49 | 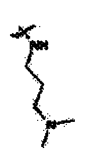 | 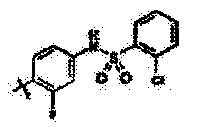 |
| 50 | 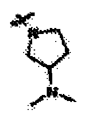 | 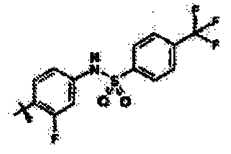 |
| 51 | 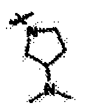 | 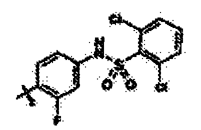 |
| 52 | 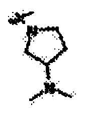 | 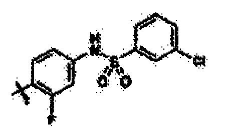 |
| 53 | 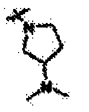 | 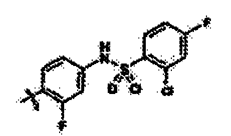 |
| 54 | 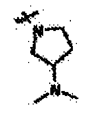 | 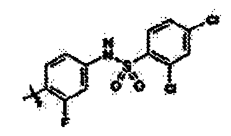 |
| 55 | 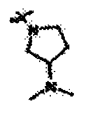 | 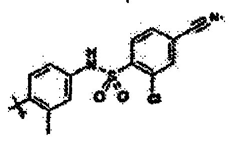 |
| 56 | 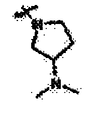 | 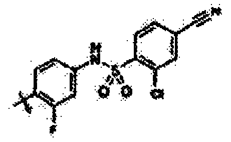 |
| 57 | 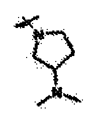 | 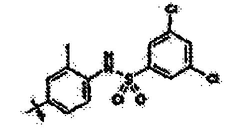 |
| 58 | 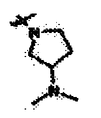 | 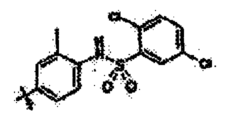 |
| 59 | 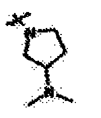 | 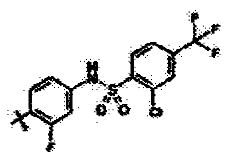 |
| 60 | 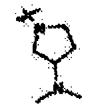 | 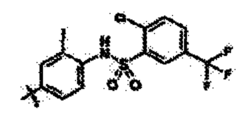 |
| 61 | 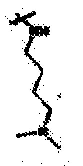 | 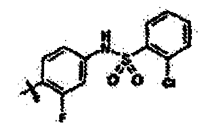 |
| 62 | 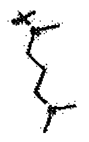 | 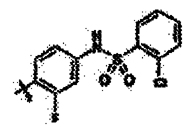 |
| 63 | 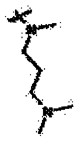 | 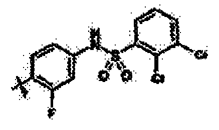 |
| 64 | 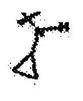 | 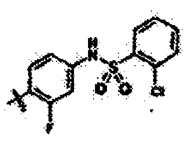 |
| 65 | 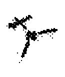 | 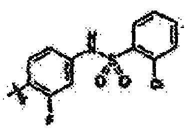 |
| 66 | 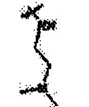 | 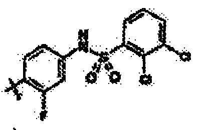 |
| 67 | 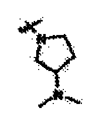 | 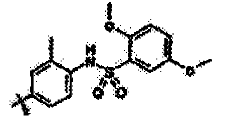 |
| 68 | 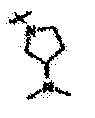 | 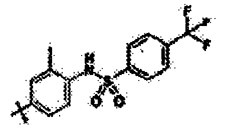 |
| 69 | 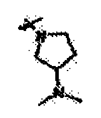 | 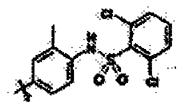 |
| 70 | 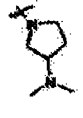 | 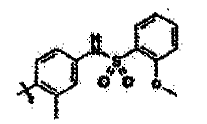 |
| 71 | 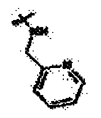 | 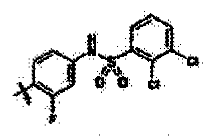 |
ou un sel pharmaceutiquement acceptable de ceux-ci.

10. Procédé de fabrication d'un composé de formule I selon l'une quelconque des revendications 1 à 9, qui consiste à faire réagir
a) un composé de formule II, 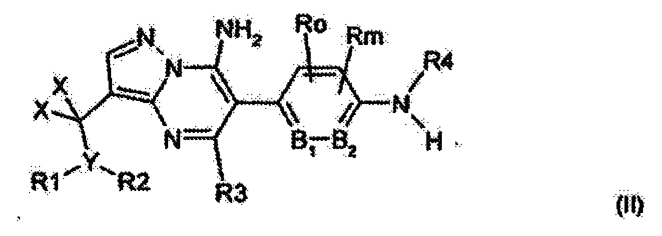 dans laquelle R1, R2, X, R3, B₁,B₂, Ro, Rm et R4 sont tels que définis pour un composé de formule I, avec un acide de formule III,
R5-OH (III)
dans laquelle R5 est tel que défini pour un composé de formule I, ou un dérivé réactif de celui-ci capable d'introduire un motif R5 comme défini pour un composé de formule I, ou
b) pour la fabrication d'un composé de formule I dans laquelle les deux symboles X ensemble sont oxo (=O) et les symboles restants sont tels que définis pour un composé de formule I, un acide de formule IV, 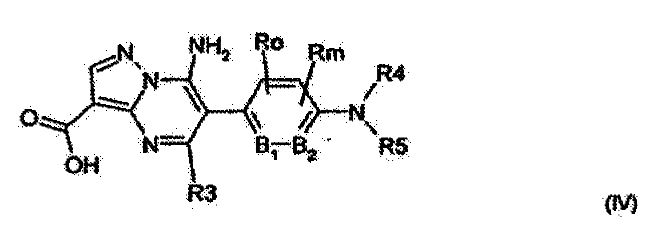 dans laquelle R2, Ro, Rm, B₁, B₂, R4 et R5 sont tels que définis pour un composé de formule I, ou un dérivé réactif de celui-ci, avec un composé de formule V, 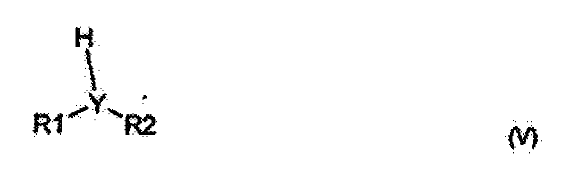 dans laquelle R1, R2 et Y sont tels que définis pour un composé de formule I, ou
c) pour la fabrication d'un composé de formule I dans laquelle chaque X est hydrogène et les autres symboles sont tels que définis pour un composé de formule I, un composé de formule VI, 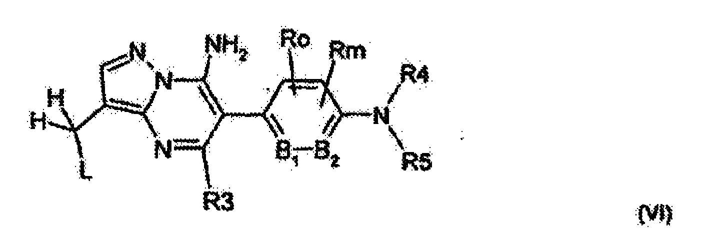 dans laquelle L est un groupement partant et R3, Ro, Rm, R4, R5, B₁ et B₂ sont tels que définis pour un composé de formule I, avec un composé de formule v tel que défini en b) ;
et, si on le souhaite, à transformer un composé de formule I en un composé différent de formule I, à transformer un sel d'un composé de formule I susceptible d'être obtenu en le composé libre ou un sel différent, à transformer un composé libre de formule I susceptible d'être obtenu en un sel de celui-ci, et/ou à séparer un mélange d'isomères susceptible d'être obtenu d'un composé de formule I en isomères individuels.

11. Composé de formule XIII, 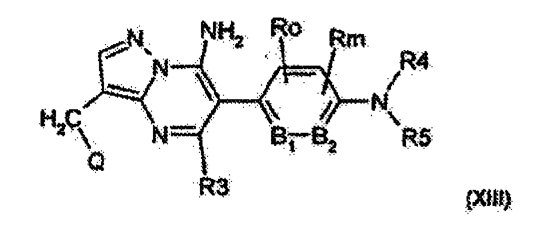 dans laquelle Q est hydroxy ou hydroxy estérifié ou éthérifié et R3, R4, R5, B₁, B₂, Ro et Rm sont tels que définis dans l'une quelconque des revendications 1 à 9,
ou un sel pharmaceutiquement acceptable de celui-ci.

12. Composé de formule XIII selon la revendication 11,
**caractérisé en ce que** Q est hydroxy, alcoxy en C₁-C₇ ou alcoxy en C₁-C₇-alcoxy en C₁-C₇ et R3, R4, R5, B₁, B₂, Ro et Rm sont tels que définis dans l'une quelconque des revendications 1 à 9, ou un sel pharmaceutiquement acceptable de celui-ci.

13. Composé de formule XIII selon l'une ou l'autre des revendications 11 et 12, choisi dans Le groupe de composés ayant les noms suivants :
N-[4-(7-amino-3-hydroxyméthyl-pyraxolo[1,5-a]pyrimidin-6-yl)-3-chlorophenyl]-benzènesulfonamide;
N-[4-(7-amino-3-hydroxyméthyl-pyrazolo[1,5-a]pyrimidin-6-yl)-3-fluorophényl]-2-chloro-benzènesulfonamide :
N-[4-(7-amino-3-hydroxymethyl-pyrazolo[1,5-a]pyrimidin-6-yl)-3-fluorophényl]-2,3-dichloro-benzènesulfonamide :
N-[4-(7-amino-3-méthoxyméthyl-pyrazolo[1,5-a]pyrimidin-6-yl)-3-fluorophenyl]-2,3-dichloro-benzènesulfonamide ;
N-[4-(7-amino-3-(2-méthoxyéthoxyméthyl)-pyrazolo[1,5-a]pyrimidin-6-yl)-3-fluorophenyl]-2,3-dichlorobenzènesulfonamide; et
N-[4-(7-amino-3-hydroxyméthyl-pyrazolo[1,5-a]pyrimidin-6-yl)-phényl]-2,3-dichloro-benzènesulfonamide;
ou un sel pharmaceutiquement acceptable de ceux-ci.

14. Composé de formule XII, 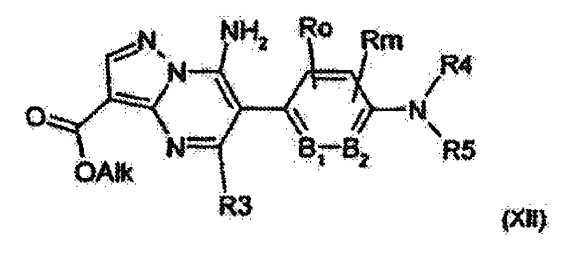 dans laquelle Alk est alkyle en C₁-C₇ non substitué ou substitué, de préférence alkyle en C₁-C₇ , et R3, R4 R5, B₁, B₂, Ro et Rm sont tels que définis dans l'une quelconque des revendications 1 à 9,

15. Composé de formule XII selon la revendication 14, choisi dans le groupe de composés portant les noms
ester éthylique d'acide 7-amino-6-[2-chlorobenzénesulfonylamino)- phényl]-pyrazolo[1,5-a]pyrimidine-3-carboxylique; et
ester éthylique d'acide 7-amino-6-[2,3-dichlorobenzénesulfonylamino)-3-fluoro-phényl]-pyrazolo[1,5-a]pyrimidine-3-carboxylique,
ou un sel pharmaceutiqeument acceptable de ceux-ci.

16. Utilisation d'un compose de formule I, ou d'un sel pharmaceutiquement acceptable de celui-ci, selon l'une quelconque des revendicatidns 1 à 9, d'un composé de formule XIII, ou d'un sel pharmaceutiquement acceptable de celui-ci, selon l'une quelconque des revendications 11 à 13 ou d'un compose de formule XII, ou d'un sel pharmaceutiquement acceptable de celui-ci, selon l'une ou l'autre des revendications 14 et 15, pour la fabrication d'une composition pharmaceutique destinée au traitement d'une maladie qui dépend de l'activité d'une protéine kinase, notamment de la kinase Tie-2.

17. Formulation pharmaceutique, comprenant un composé de formule I ou un sel pharmaceutiquement acceptable de celui-ci, selon l'une quelconque des revendications 1 à 9, un composé de formule XIII, ou un sel pharmaceutiquement acceptable de: celui-ci, selon l'une quelconque des revendications 11 à 13. ou un composé de formule XII, ou un sel pharmaceutiquement acceptable de celui-ci, selon l'une ou l'autre des revendications 14 et 15, et au moins un matériau support pharmaceutiquement acceptable,

18. Composé de formule I, ou un sel pharmaceutiquement acceptable de celui-ci, selon l'une quelconque des revendications 1à 9, un composé de formule XIII, ou un sel pharmaceutiquement acceptable de celui-ci, selon l'une quelconque des revendications 11 à 13 ou un composé de formule XII, ou un sel pharmaceutiquement acceptable de celui ci, selon l'une ou l'autre des revendications 14 et 15, destinés à être utilisés dans le diagnostique ou le traitement thérapeutique d'un organisme animal eu humain, notamment pour le traitement d'une maladie kinase-dépendante de préférence d'une maladie qui dépend de Tie-2,
